(19) Europäisches Patentamt
European Patent Office
Office européen des brevets

(11) Veröffentlichungsnummer : **0 538 300 B1**

(12) # EUROPÄISCHE PATENTSCHRIFT

(45) Veröffentlichungstag der Patentschrift : **13.04.94 Patentblatt 94/15**

(51) Int. Cl.$^5$ : **C12N 15/21,** C12P 21/02, C12P 21/08, A61K 37/66

(21) Anmeldenummer : **91912306.7**

(22) Anmeldetag : **06.07.91**

(86) Internationale Anmeldenummer : **PCT/EP91/01266**

(87) Internationale Veröffentlichungsnummer : **WO 92/01055 23.01.92 Gazette 92/03**

(54) **O-GLYCOSYLIERTES IFN-ALPHA.**

(30) Priorität : **10.07.90 DE 4021917**
**12.11.90 DE 4035877**

(43) Veröffentlichungstag der Anmeldung : **28.04.93 Patentblatt 93/17**

(45) Bekanntmachung des Hinweises auf die Patenterteilung : **13.04.94 Patentblatt 94/15**

(84) Benannte Vertragsstaaten : **AT BE CH DE DK ES FR GB GR IT LI LU NL SE**

(56) Entgegenhaltungen :
**EP-A- 158 420**
**WO-A-83/00693**
**DE-A- 3 306 060**
**US-A- 4 289 690**
**J. of Interferon Research, vol. 9 SUP, no. 2, 1989, p. 184; K. Zoon et al.**
**Archives of Biochemistry and Biophysics, vol. 232, no. 1, July 1984, New York, pp. 422-426; J.E. Labdon et al.**
**Nature, vol. 287, 2 Oct. 1980, London, GB, pp. 408-411; G. Allen et al.**

(56) Entgegenhaltungen :
**The J. of General Virology, vol. 68, no. 6, June 1987, Colchester, GB, pp. 1669-1676; G.R. Adolf**
**The Biomedical Journal, vol. 276, no. 2, 29 May 1991, Colchester, GB, pp. 511-518; G.R. Adolf et al.**

(73) Patentinhaber : **BOEHRINGER INGELHEIM INTERNATIONAL GmbH**
**D-55216 Ingelheim (DE)**

(72) Erfinder : **ADOLF, Günther**
**Stiftgasse 15-17/10**
**A-1070 Wien (AT)**
Erfinder : **HIMMLER, Adolf**
**Fürst Liechtensteinstr. 2/3**
**A-1236 Wien (AT)**
Erfinder : **AHORN, Horst, Johann**
**Eisenstädterstr. 3/1**
**A-2484 Weigelsdorf (AT)**
Erfinder : **KALSNER, Inge**
**Geusaugasse 51/20**
**A-1030 Wien (AT)**
Erfinder : **MAURER-FOGY, Ingrid**
**Lindauergasse 35**
**A-1238 Wien (AT)**

**EP 0 538 300 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der vorliegenden Erfindung sind O-glycosylierte alpha Interferone, vorzugsweise ein Interferon alpha, im wesentlichen mit den biologischen und/oder immunologischen Eigenschaften eines IFN-α2, Verfahren zu dessen Herstellung sowie die Verwendung der O-glycosylierten Interferone als Arzneimittel.

Seit der Entdeckung der Interferone vor mehr als dreißig Jahren werden ihre biologischen Eigenschaften als Mediatoren der interzellulären Kommunikation intensiv untersucht. Ursprünglich wurde die Bezeichnung der verschiedenen Arten von der jeweiligen Zelle, in der sie entstanden sind, abgeleitet (z.B. Leukocyten-IFN, Fibroblasten-IFN). Mit zunehmender Kenntnis ihrer Struktur wurde eine neue Nomenklatur eingeführt. Man unterscheidet zur Zeit vier Arten von Interferonen (IFN-α, IFN-β, IFN-γ und IFN-ω), wobei IFN-α, IFN-β und IFN-ω zu den sogenannten "Klasse 1 Interferonen" zusammengefaßt werden, da sie ähnliche Strukturen und Eigenschaften aufweisen.

IFN-γ wird von Lymphozyten, die durch Antigene oder mitogene Substanzen stimuliert werden, gebildet. Die Aminosäuresequenz, die keine Homologie zu den Klasse 1 Interferonen aufweist, enthält zwei potentielle N-Glycosylierungsstellen.

IFN-α, IFN-β und IFN-ω werden von verschiedenen Zellen als Reaktion auf Virusinfektion oder nach Induktion mit doppelsträngiger RNA synthetisiert.

Bei IFN-α handelt es sich eigentlich um eine ganze Gruppe von Proteinen. Bisher wurden mindestens 14 funktionelle Gene entdeckt, die für verschiedene IFN-α-Typen kodieren. Diese Proteine sind nahe verwandt und weisen zumeist etwa 80-90% Homologie in ihrer Aminosäuresequenz auf. Mit Ausnahme von IFN-α14 ist in keiner der übrigen IFN-α- Aminosäuresequenzen eine N-Glycosylierungsstelle (ASN-X-SER/THR) vorhanden. N-Glycosylierung ist somit in allen Fällen (außer IFN-α14) auszuschließen, jedoch wurde O-Glycosylierung von IFN-α diskutiert (Labdon et al., Arch. Biochem. Biophys. 232, 422-426 (1984))

Viele in der Natur vorkommende Proteine werden posttranslational modifiziert, wobei Glycosylierung eine der häufigsten Modifikationen ist. Glycoproteine kommen membrangebunden oder löslich sowohl in der intra- als auch extrazellulären Matrix vor. Über die Funktion der Glycosylierung gibt es unterschiedliche Auffassungen. Gesichert ist, daß Glycane die Proteine vor proteolytischem Abbau schützen können oder daß sie in vielen Fällen für Zell-Zell-Wechselwirkungen verantwortlich sind. Weiterhin beeinflussen sie die Proteinfaltung und tragen zur Stabilität der Konformation des Moleküls bei. Auch die Löslichkeit der Proteine unterliegt dem Einfluß der Kohlenhydratketten.

Man unterscheidet zwischen N- und O-glycosylierten Proteinen. N-Glycane werden ausschließlich auf das ASN des Triplets -ASN-X-SER/THR- übertragen, wobei X jede beliebige Aminosäure mit Ausnahme von PRO oder GLU sein kann. Diese Anforderung an die Struktur des Proteins kann aber nur eine von mehreren sein, da nicht alle potentiellen Glycosylierungsstellen mit einem Kohlenhydrat besetzt sind. Für O-Glycane gibt es keine genau definierten Strukturmerkmale. Es gibt allerdings Hinweise darauf, daß O-Glycane bevorzugt in PRO-, SER- und THR-reichen Regionen synthetisiert werden. Das läßt vermuten, daß eher die sterische Zugänglichkeit der Glycosylierungsstelle als eine bestimmte Aminosäuresequenz für die O-Glycosylierung bedeutend ist. Einflüsse der Glycosylierung auf die Pharmakokinetik sowie auf die immunologischen Eigenschaften des Proteins können nicht ausgeschlossen werden. So wurde kürzlich darüber berichtet (Gibbon et al., Lancet, 335, 434-437 (1990), daß 4 von 16 Patienten, die mit rekombinantem humanem GM-CSF (granulocytemacrophage-colony stimulating factor), der in Hefe produziert worden war, behandelt worden waren, Antikörper gegen dieses Protein entwickelten. Man stellte fest, daß diese Antikörper mit Epitopen reagierten, die in endogenem GM-CSF durch O-Glycosylierung geschützt vorliegen, im rekombinanten Faktor jedoch frei zugänglich sind.

Im IFN-α2 konnten bislang weder Kohlenhydratanteile nachgewiesen, noch konnte O-glycosyliertes IFN-α isoliert werden. Verschiedene Präparationen von natürlichem IFN-α und von rekombinantem IFN-α2 sind als Medikamente gegen virale und Krebserkrankungen im Einsatz. Da dieses IFN-α2 in E. coli produziert wird und daher nicht glycosyliert sein kann, scheint der Kohlenhydratanteil für die in vivo biologische Aktivität nicht bedeutend zu sein. In letzter Zeit mehren sich jedoch Berichte, daß Patienten, die längere Zeit mit rekombinantem, in E. coli produziertem IFN-α2 behandelt wurden, Antikörper dagegen entwickelten (z.B. Figlin & Itri, Semin. Haematol. 25. 9-15 (1988)).

Aufgabe der vorliegenden Erfindung war, ein neues IFN-α2 bereitzustellen.

Gelöst wurde diese Aufgabe durch die Insertion der für IFN-α2 kodierenden DNA Sequenz in einen speziellen Expressionsvektor, mit dem Zellen multizellulärer Organismen transfiziert wurden. Nach Kultivierung dieser so modifizierten Zellen erhielt man überraschenderweise IFN-α2-artige Proteine, die sich im Molekulargewicht eindeutig von dem bekannten rekombinanten IFNα2 unterschieden.

Zur Herstellung der erfindungsgemäßen, neuen Interferone eignen sich Kulturen von Zellen multizellulärer Organismen, insbesondere Kulturen von Wirbeltierzellen oder von Insektenzellen. Als Beispiele von Wirbeltierzellinien sind VERO-Zellen, HeLa-Zellen, CHO-Zellen, WI38-Zellen, BHK-Zellen, COS-7-Zellen, MDCK-Zellen oder Maus-Myelomzellen zu nennen. Expressionsvektoren für diese Zellen enthalten wenn nötig eine Replikationsstelle, einen Promotor, falls erforderlich eine RNA-Splicing-Stelle, eine Polyadenylierungsstelle und transkriptionelle Terminations-Sequenzen. Die Kontrollfunktionen solcher Expressionsvektoren stammen üblicherweise aus viralem Material. Gebräuchliche Promotoren stammen aus Polyoma, Adenovirus 2, Simian Virus 40 (SV40), bevorzugt aus Cytomegalovirus (CMV).Die erforderliche Replikationsstelle kann entweder durch eine entsprechende Vektorkonstruktion vorgesehen werden, so beispielsweise die Replikationsstelle aus SV40, Polyoma, Adeno, VSV, oder PBV oder kann durch die chromosomalen Replikationsmechanismen der Wirtszelle vorgesehen werden. Bei Integration des Vektors in das Wirtszellenchromosom reicht die letztgenannte Maßnahme aus.

Erfindungsgemäß werden vorzugsweise Expressionsvektoren verwendet, die aus Teilen von Plasmiden neu konstruiert wurden. Diese erfindungsgemäßen Expressionsvektoren weisen eine Multiklonierstelle für die gerichtete Insertion heterologer DNA-Sequenzen auf und lassen sich vorzugsweise in E. coli mittels Ampicillinresistenz mit hoher Kopienzahl vermehren. Um die Expression heterologer Gene in Säugetierzellen zu ermöglichen, enthalten die erfindungsgemäßen Expressionsplasmide vorzugsweise den Cytomegalovirus (CMV) Promotor/Enhancer (M. Boshart et al., Cell 41, (1985), 521-530). Um die autonome Replikation der erfindungsgemäßen Expressionsplasmide zu hohen Kopienzahlen und damit hohe Raten in transienter Expression in geeigneten Zellinien wie beispielsweise in COS-7 oder in der mit Adenovirus transformierten Zellinie 293 (ATCC CRL 1573), zu ermöglichen, wurde der SV40 Replikationsursprung verwendet. Zur Herstellung permanent transformierter Zellinien und zur nachfolgenden Amplifikation der Expressionskassette mittels Methotrexat dient ein modifiziertes Hamster Minigen (Promotor mit kodierendem Bereich und dem ersten Intron) für Dihydrofolatreduktase (DHFR) als Selektionsmarker. Um die Herstellung einzelsträngiger Plasmid-DNA nach Superinfektion der transformierten Bakterien mit einem Helferphagen, beispielsweise mit R408 oder M13KO7, zur erleichterten Sequenzierung und Mutagenese der Plasmid-DNA zu ermöglichen, enthielten vorzugsweise Ausgestaltungen der erfindungsgemäßen Plasmide die intergenische Region von M13. Wird in einer weiteren vorzugsweisen Ausgestaltung der T7 Promotor der Multiklonierstelle vorangestellt, wird dadurch die Herstellung von RNA Transkripten in vitro ermöglicht.

Erfindungsgemäß bevorzugt sind die Expressionsplasmide pAD-CMV13, pAD-CMV15 insbesondere pAD-CMV19. Ihre Herstellung ist in Beispiel 1 ausführlich beschrieben.

Zur Erzielung einer verbesserten Expression und zur Erleichterung einer gerichteten Klonierung der für IFN-α2 kodierenden cDNA wurde die für IFN-α2 kodierende cDNA mittels PCR in der 5'-nicht kodierenden Region erfindungsgemäß dahingehend modifiziert, daß die Sequenz dieser Region gegen die Sequenz der 5'-nicht kodierenden Region der humanen β-Globin mRNA (Lawn et al., Cell 21, (1980), 647-651) ausgetauscht wird. Gleichzeitig wurden an beiden Enden der cDNA Restriktionsenzymschnittstellen eingeführt, die eine gerichtete Klonierung erleichtern. Überraschenderweise bewirkt eine derartige Veränderung eine deutliche Erhöhung der Expression.

Die so modifizierte cDNA für IFN-α2 wurde in ein mit den entsprechenden Restriktionsenzymen geschnittenes erfindungsgemäßes Expressionsplasmid, vorzugsweise in das Plasmid pAD-CMV19 inseriert. Mit den so erhaltenen Expressionsplasmiden für IFN-α2 wurden geeignete Säugetierzellen transfektiert, die daraufhin in einem geeigneten Kulturmedium kultiviert wurden. Der Kulturüberstand der Säugetierzellen wurde in an sich bekannter Weise unter schonenden Bedingungen gereinigt. Vorzugsweise verwendet man affinitätschromatographische Reinigungsverfahren mit Hilfe monoklonaler Antikörper gegen IFN-α2. Bevorzugte monoklonale Antikörper sind EBI-1 oder EBI-10 beziehungsweise deren Äquivalente. Die Herstellung dieser hochspezifischen Antikörper ist beschrieben (Adolf G.R. J. Gen. Virol. 68, 1669-1676 (1987); Adolf et al. J. Cell. Physiol. suppl. 2, 61-68 (1982)). Die zu verwendenden Methoden sind ebenfalls beschrieben (Secher und Burke, Nature 285, 446-450 (1980); Adolf et al., J. Biol. Chem. 265, 9290-9295 (1990); Adolf et al., Biochem. J. 276, 511-518 (1991)). Besonders vorteilhaft ist das Reinigungsverfahren gemäß EPA 0 203 382 zu verwenden, wobei auf das Aufbrechen der Zellen verzichtet werden kann. Zur Charakterisierung von rekombinantem, in Säugetierzellen hergestelltem IFN-α2 wurde die Reverse Phase HPLC verwendet. Der N-Terminus und C-Terminus wurden analysiert. Zum Vergleich wurde jeweils rekombinantes, in E. coli hergestelltes IFN-α2c verwendet. Anhand SDS-Gelelektrophoretischer Untersuchungen war festzustellen, daß das in Säugetierzellen hergestellte rekombinante IFN-α2 ein höheres Molekulargewicht aufwies, als das in E. coli hergestellte IFN-α2. Nach Behandlung beider rekombinanter Interferone mit NaOH reduzierte sich das Molekulargewicht des in Säugetierzellen hergestellten, rekombinanten IFN-α2's auf das Molekulargewicht des in E. coli hergestellten IFN-α2's. In Säugetierzellen exprimiertes, rekombinantes IFN-α2 muß daher glycosyliert sein. Bei der Identifizierung der Glycopeptide mittels Peptide Mapping und Sequenzanalyse konnte festgestellt werden, daß das an Position 106 befindliche Threonin (THR-106) die Glycosylierung trägt. Bei einem Vergleich der Resultate mit denen, die beim natürlichen IFN-α2 aus virusstimulierten Leukocyten erhalten wurden (s. unten) zeigte es sich, daß sowohl die Glycosylierungsstelle als auch der Oligosaccharidanteil weitgehend identisch sind.

Gelöst wurde die erfindungsgemäße Aufgabe aber auch durch ein Reinigungsverfahren, das keine Verfahrensschritte enthält, die evtl. vorhandene Substitutionen des IFN-α2 verändern oder eliminieren. Das erfindungsgemäße Reinigungsverfahren bediente sich hochspezifischer monoklonaler Antikörper, wobei während des gesamten Reinigungsverfahrens alkalische Bedingungen mit einem pH-Wert größer als 8,0 sorgsam vermieden wurden.

Natürliches humanes IFN-α2 wurde mit Hilfe eines hochspezifischen monoklonalen Antikörpers aus Leukozyteninterferon isoliert. Zwei aufeinanderfolgende Reinigungsschritte über eine Immunoaffinitätssäule führten zu einer Reinheit des Proteins von >95%. Die Sequenzanalyse ergab die erwartete N-terminale Sequenz, wobei CYS als erste Aminosäure nur indirekt nachgewiesen wurde.

Von IFN-α2 sind bisher drei Varianten, die sich in den Aminosäuren an den Positionen 23 und 34 unterscheiden, bekannt: IFN-α2a mit $^{23}$LYS und $^{34}$HIS (früher als Le IFA bezeichnet; Goeddel et al., Nature, 290, 20-26 (1981)), IFN-α2b mit $^{23}$ARG und $^{34}$HIS (Streuli et al., Science, 209, 1343-1347 (1980)) und IFN-α2c mit $^{23}$ARG und $^{34}$ARG (früher als IFN-α2"Arg" bezeichnet; Dworkin-Rastl et al., J. Interferon Res.-2, 575-585 (1982); Bodo & Maurer-Fogy, The Biology of the Interferon System 1985 (Stewart II, W.E. & Schellehus H. Hrsg.) 59-64 (1986). Bei dem isolierten Interferon konnte an Position 23 nur ARG nachgewiesen werden, was das Vorhandensein von IFN-α2a ausschließt. Die Aminosäure an Position 34 war eindeutig Histidin, so daß es sich bei dem isolierten Interferon um IFN-α2b handelte. Ebenso sind jedoch auch die Varianten IFN-α2a bzw. IFN-α2c erhältlich je nachdem, welches Zellmaterial als Ausgangsmaterial verwendet wird. Es ist bekannt, daß in Namalwa-Zellen neben IFN-α2b auch IFN-α2c zu finden ist. Bei dem als Vergleichssubstanz verwendeten rekombinanten Interferon aus E. coli handelte es sich um IFN-α2c.

RP-HPLC-Analysen des gereinigten natürlichen IFN-α2 zeigten, daß die Präparation zwei Peaks enthielt, die beide früher von der Säule eluierten als das rekombinante E. coli-IFN-α2c. Auch mittels SDS-PAGE konnte eine starke Heterogenität in der scheinbaren molekularen Masse von natürlichem IFN-α2 nachgewiesen werden. Alle in natürlichem IFN-α2 nachgewiesenen Proteine hatten eine wesentlich höhere scheinbare molekulare Masse als rekombinantes IFN-α2c aus E. coli. Sämtliche bisher beschriebene IFN-α-Spezies - mit Ausnahme von IFN-α14 - weisen keine N-Glycosylierungsstelle (-ASN-X-THR/SER-) auf. Somit kann auch für IFN-α2 N-Glycosylierung ausgeschlossen werden. Für O-Glycane kennt man solche Strukturmerkmale nicht. Nicht auszuschließen ist daher, daß das vorliegende IFN-α2 O-glycosyliert ist.

Da O-Glycane schon unter schwach alkalischen Bedingungen vom Protein abgespalten werden können, wurden beide Peakfraktionen schwach alkalischen Bedingungen unterworfen. Diese Reaktion führte in beiden Fällen zu einer Reduktion der scheinbaren molekularen Masse auf die des rekombinanten IFN-α2c aus E. coli; ein deutlicher Hinweis auf O-Glycosylierung.

Versuche mit Neuraminidase und O-Glycanase ergaben für den einen Peak (Peak 2) (s. Fig. 14) ebenfalls eine Reduktion der scheinbaren molekularen Masse auf jene von E. coli-IFN-α2c und bestätigten damit die O-Glycosylierung. Die Ergebnisse dieses sequentiellen Abbaues des Glycans mit Neuraminidase und O-Glycanase zeigten, daß die Heterogenität des Peak 2 auf dem unterschiedlichen Gehalt von N-Acetylneuraminsäure (NeuAc = Sialinsäure) beruhte. Die drei Banden (Fig. 20, Spur 4) repräsentierten die di- bzw. monosialylierte (Mr 21.000 bzw. 20.000) und die nichtsialylierte (Mr 19.000) Form des natürlichen IFN-α2. Die leichteste Form des IFN-α2 konnte durch Reaktion mit O-Glycanase allein abgebaut werden. Da O-Glycanase nur das unsubstituierte Disaccharid Gal(β1-3)GalNAc spaltet, ist die Reaktion als Beweis dafür anzusehen, daß neben den beiden sialylierten Formen auch eine Asialo-Variante des IFN-α2 existiert.

Die scheinbare molekulare Masse von Peak 1 hingegen konnte mittels Enzymreaktionen nicht reduziert werden. Inkubation mit Neuraminidase führte nicht wie erwartet zu einer Reduktion der scheinbaren molekularen Masse. Das Disaccharid-Core mußte demnach anders als mit NeuAc substituiert sein und konnte daher nicht durch O-Glycanase abgespalten werden.

Durch Vergleich der Peptide Maps nach Trypsinspaltung von natürlichem und rekombinantem, in E. coli exprimiertem IFN-α2 konnten die Glycopeptide aus den Peaks 1 und 2 identifiziert werden. Die Sequenzierung dieser Glycopeptide ergab $^{106}$THR als Glycosylierungsstelle.

Hinweise auf die Struktur der Oligosaccharide des natürlichen IFN-α2 gaben neben den Enzymreaktionen auch massenspektrometrische Untersuchungen der Glycopeptide. Die Interpretation der Massenspektren zusammen mit den Ergebnissen der SDS-PAGE ergaben, daß natürliches IFN-α2 zumindest vier verschiedene Glycanstrukturen enthält: im Peak 2 das neutrale Disaccharid Gal(β1-3)GalNAc, dessen Struktur aufgrund der hohen Spezifität der O-Glycanase mit großer Sicherheit anzunehmen ist, außerdem die mono- und die disialylierte Variante; im Peak 1 ein neutrales Oligosaccharid, bestehend aus zwei Hexose- und zwei N-Acetylhexosamin-Einheiten. Als Struktur dieses Tetrasaccharides kann in Analogie zu bereits beschriebenen häufiger vorkommenden O-Glycanen vorgeschlagen werden: Gal-(Gal-GlcNAc-)GalNAc.

Durch die vorliegende Erfindung konnte überraschenderweise erstmals O-glycosyliertes IFN-α2 in hochreiner Form bereitgestellt werden. Dieses Interferon ist an der Aminosäure Threonin an Position 106 ($^{106}$THR) O-glycosyliert. Die Oligosaccharide, die an dieser Position enthalten sein können, sind das neutrale Disaccharid Gal(β1-3)GalNAc, dessen mono- und disialylierte Varianten sowie ein neutrales Tetrasaccharid Gal-(Gal-GlcNAc-)GalNAc.

Dieses O-glycosylierte IFN-α2 kann in an sich bekannter Weise, in Analogie zum rekombinanten in E. coli exprimierten IFN-α2, formuliert und in allen, für IFN-α bekannten Indikationen zur Behandlung eingesetzt werden.

Die erfindungsgemäßen Proteine können für die Behandlung der viralen Infektionen und von malignen Erkrankungen in der Form von pharmazeutischen Präparaten verwendet werden, die eine wirksame Menge des IFN's gegebenenfalls zusammen mit einer signifikanten Menge eines anorganischen oder organischen, festen oder flüssigen, pharmazeutisch verwendbaren Trägerstoffes enthalten.

Bevorzugt sind pharmazeutische Präparate zur parenteralen, beispielsweise intramuskulären, subkutanen oder intravenösen Verabreichung am Menschen. Solche Präparate sind isotonische wässrige Lösungen oder Suspensionen, die die erfindungsgemäßen Proteine enthalten, gegebenenfalls zusammen mit einem Trägermaterial und, wenn erwünscht, Hilfsmittel, beispielsweise Stabilisatoren, Emulgiermittel, lösungsvermittelnde Stoffe, Salze für die Regulierung des pH und des osmotischen Druckes, Konserviermittel und/oder Netzmittel. Die pharmazeutischen Präparationen können nach an sich bekannten Methoden hergestellt werden, beispielsweise in einem Verfahren, worin die erfindungsgemäßen Proteine und die pharmazeutisch verwendbaren Träger und Hilfsstoffe gemischt, gewünschtenfalls lyophilisiert und vor Verwendung gelöst werden.

Die Dosierung der pharmazeutischen Präparate hängt von der zu behandelnden Krankheit, dem Körpergewicht, Alter und individuellen Zustand des Patienten gemäss Einschätzung des behandelnden Arztes und der Applikationsweise ab.

Durch die vorliegende Erfindung wird daher erstmals ein O-glycosyliertes Interferon-α2 enthaltendes Mittel bereitgestellt, das aufgrund der antiviralen und antineoplastischen Eigenschaften des IFN-α2 u.a. zur Behandlung von viralen und tumoralen Erkrankungen geeignet ist.

Die nachfolgenden Beispiele sollen die Erfindung erläutern ohne sie einzuschränken.

**Legenden zu den Figuren**

Fig. 1: Konstruktion des Plasmides pCMV+SV40
Fig. 2: Konstruktion des Plasmides pAD-CMV10A
Fig. 3: Konstruktion des Plasmides pAD-CMV15
Fig. 4: Konstruktion der Plasmide pAD-CMV13 und pAD-CMV19
Fig. 5: Konstruktion des Expressionsplasmides pAD 19B-IFN
Fig. 6: HindIII/XBaI-Insert des Expressionsplasmides pAD19B-IFN
Fig. 7: DNA-Sequenz des Plasmides pAD-CMV19
Fig. 8: Konstruktion des Plasmides pCMV-SV40
Fig. 9: Konstruktion des Plasmides pSV2gptDHFRMut2
Fig. 10: Konstruktion der Plasmide pAD-CMV1 und pAD-CMV2
Fig. 11: DNA-Sequenz des Plasmides pAD-CMV1
Fig. 12: Monoklonale Antikörper Affinitätschromatographie des humanen Leukozyten Interferons
Fig. 13: ELISA für human IFN-α: (O) Referenzpräparation des rekombinanten human IFN-α2c; (O) Leukozyteninterferon (Ausgangsmaterial) (□) Durchfluß (□) eine Fraktion des Eluates A; (Δ) eine Fraktion des Eluates B
Fig. 14: RP-HPLC des natürlichen IFN-α2 (b) und E. coli IFN-α2c (a)
Fig. 15: Aminosäuresequenz des IFN-α2c
Fig. 16: SDS-PAGE von natürlichem IFN-α2 vor und nach Reaktion mit Neuraminidase und O-Glycanase. (1) Peak 1, unbehandelt; (2) Peak 1, nach Reaktion mit Neuraminidase; (3) Peak 1, nach Reaktion mit Neuraminidase und O-Glycanase; (4) Peak 2, unbehandelt; (5) Peak 2, nach Reaktion mit Neuraminidase; (6) Peak 2, nach Reaktion mit Neuraminidase und O-Glycanase; (7) E. coli-IFN-α2c
Fig. 17: SDS-PAGE von natürlichem IFN-α2 (Peak 2 aus Fig. 14b) vor (1) und nach (2) Reaktion mit O-Glycanase.
Fig. 18: SDS-PAGE von natürlichem IFN-α2 (Peak 1 und 2) und E. coli-IFN-α2c nach Inkubation mit 0.1 M NaOH. (1) E. coli-IFN-α2; (3) Peak 1; (5) Peak 2; unbehandelte Vergleichsproben von Peak 1 (2) und von Peak 2 (4) wurden ebenfalls aufgetragen.
Fig. 19: Vergleichendes Peptide Map von E. coli-IFN-α2c und natürlichem IFN-α2. (1) Peak 1 aus Fig. 14b; (2) Peak 2 aus Fig. 14b; *, diese Peaks stammen von unglycosylierten Peptiden, deren Retentionszeit immer gleich war.

Fig. 20: SDS-PAGE von natürlichem IFNα2 und E. coli-IFNα2c. (1) Molekulargewichtsmarker; (2) E. coli-IFNα2c; (3) natürliches IFN-α2, Peak 1 aus Fig. 14b; (4) natürliches IFN-α2, Peak 2 aus Fig.14b; Färbung: Coomassie-Blue.

Fig. 21: Reverse Phase HPLC (RP-HPLC) des CHO-IFN-α2c (a) und des E.coli-IFN-α2c (b)

Fig. 22: Vergleichende Peptide Maps von Peak 1 (a) und Peak 2 (b) aus CHO-IFN-α2c und von E.coli-IFN-α2c (c)

Fig. 23: SDS-Gelelektrophorese (SDS-PAGE) von CHO-IFN-α2c und E.coli-IFN-α2c. Spuren 1 und 8: Molekulargewichtsmarker (Skala in kD); Spuren 2-4: nichtreduzierende Bedingungen, Spuren 5-7: reduzierende Bedingungen;
Spuren 2 und 5: Peak 1 aus CHO-IFN-α2c;
Spuren 3 und 6: Peak 2 aus CHO-IFN-α2c;
Spuren 4 und 7: E.coli-IFN-α2c;
Oberes Gel: Alle IFN-Spuren mit je 4 μg;
Unteres Gel: Alle IFN-Spuren mit je 1 μg;
Färbung: Coomassie Blue

Fig. 24: SDS-Gelelektrophorese (SDS-PAGE) von CHO-IFN-α2c und E.coli-IFN-α2c vor und nach Inkubation mit 0,1 M NaOH.
Spuren 1 und 8: Molekulargewichtsmarker (Skala in kD); Spuren 2, 4, 6: unbehandelte Proben, Spuren 3, 5, 7: mit 0,1 M NaOH inkubierte Proben;
Spuren 2, 3: E.coli-IFN-α2c,
Spur 4, 5: Peak 1 aus CHO-IFN-α2c,
Spur 6, 7: Peak 2 aus CHO-IFN-α2c;
Auf alle IFN-Spuren wurden je etwa 1,5 μg unter reduzierenden Bedingungen aufgetragen.
Färbung: Coomassie Blue

Beispiel 1

Konstruktion der Expressionsplasmide pAD-CMV13, pAD-CMV15 und pAD-CMV19

Aus Teilen von Expressionsplasmiden (pCDM8, Seed & Aruffo, Proc. Natl.Acad.Sci.USA 84 (1987) 8573-8577; B. Seed, Nature 329 (1987) 840-842); Invitrogen, Inc., San Diego, CA; pSV2gptDHFR20, EP-A1 0321842) und dem Plasmid pBluescript KS- (Short et al., Nucleic Acids Res., 11 (1988) 5521-5540; Stratagene, La Jolla, CA)) wurden neue Plasmide konstruiert, die eine Multiklonierstelle für die gerichtete Insertion heterologer DNA-Sequenzen aufweisen und sich in E.coli mittels Ampicillinresistenz mit hoher Kopienzahl vermehren lassen. Die intergenische Region von M13 ermöglicht die Herstellung einzelsträngiger Plasmid-DNA nach Superinfektion der transformierten Bakterien mit einem Helferphagen (z.B. R408 oder M13K07), zur erleichterten Sequenzierung und Mutagenese der Plasmid-DNA. Der T7 Promotor, der der Multiklonierstelle vorangeht, ermöglicht in vitro die Herstellung von RNA Transkripten. In Säugetierzellen erfolgt die Expression heterologer Gene getrieben vom Cytomegalovirus (CMV) Promotor / Enhancer (M. Boshart et al., Cell 41 (1985) 521-530). Der SV40 Replikationsursprung ermöglicht in geeigneten Zellinien (z.B. SV40 transformierte Zellen wie COS-7, Adenovirus transformierte Zellinie 293 (ATCC CRL1573)) die autonome Replikation des Expressionsplasmides zu hohen Kopienzahlen und damit hohe Raten in transienter Expression. Für die Herstellung permanent transformierter Zellinien und die nachfolgende Amplifikation der Expressionskassette mittels Methotrexat dient ein modifiziertes Hamster Minigen (Promotor mit kodierendem Bereich und dem ersten Intron) für Dihydrofolatreduktase (DHFR) als Selektionsmarker.

Herstellung der Vektor- und Promotoranteile durch Polymerase Kettenreaktion (polymerase chain reaction, PCR)

Das Plasmid pBluescript KS- wurde mit HindIII linearisiert und 100 ng DNA in einem 100 μl PCR (Saiki et al., Science 239 (1988) 487-491) Ansatz eingesetzt (Reaktionsmedium: 50 mM KCl, 10 mM Tris-Cl pH 8,3, 1,5 mM $MgCl_2$, 0,01% (w/v) Gelatine, 0,2 mM jeder der vier Desoxynukleosidtriphosphate (dATP, dGTP, dCTP, dTTP), 2,5 units Taq Polymerase pro 100 μl). Als Primer wurden je 50 pmol der synthetischen Oligonukleotide EBI-1786 (5'-GGAATTCAGCCTGAA- TGGCGAATGGG-3') und EBI-2134 (5'-CACTGAACTCGAGCAGC-TGCGTTGCTGGCGTTTTTCC-3') eingesetzt. Nach 5 Minuten Denaturieren bei 94°C erfolgte die PCR über 10 Zyklen (Zyklusbedingungen: 40 sec bei 94°C, 45 sec bei 55°C, 5 Min bei 72°C, Perkin Elmer Cetus Thermal Cycler). Die Oligonukleotide flankieren die intergenische Region von M13 bzw. den Replikationsursprung (ori) mit dem dazwischenliegenden Gen für die β-Lactamase. Gleichzeitig wird am Ende des ori eine XhoI- und eine PvuII- und am anderen Ende eine EcoRI-Schnittstelle erzeugt. Das Reaktionsgemisch wurde durch Extraktion mit Phenol-Chloroform von Protein befreit und die DNA mit Äthanol präzipitiert. Die erhaltene DNA wurde mit XhoI und EcoRI geschnitten und nach Elektrophorese in einem Agarosegel ein Fragment mit 2,3 kb isoliert. 50 ng mit SacII linearisiertes Plasmid pCDM8 wurde mit den Oligonukleotiden EBI-2133 (5'-GGTCACTGTCGACAT- TGATTATTGACTAG-3') und EBI-1734 (5'-GGAATTCCCT- AGGAATACAGCGG-3') unter identischen Bedingungen wie zuvor beschrieben durch PCR amplifiziert. Die Oligonukleotide binden am Beginn der CMV-Promotor / Enhancer Sequenz und erzeugen eine SalI Schnittstelle (EBI-2133), bzw. binden am Ende der SV40 poly-Adenylierungstelle und erzeugen eine EcoRI Schnittstelle (EBI-1734). Das PCR Produkt wurde mit SalI und EcoRI geschnitten und ein DNA Fragment von 1,8 kb aus einem Agarosegel isoliert.

Die beiden nachgeschnittenen PCR Produkte wurden mit T4 DNA-Ligase ligiert und E.coli HB101 transformiert. Ein Plasmid der gewünschten Struktur (siehe Fig.1) wurde pCMV+M13 benannt.

Der SV40 Replikationsursprung (SV40 ori) wurde aus dem Plasmid pSV2gptDHFR20 (EP-A1 0321842) isoliert. Dazu wurde dieses Plasmid mit HindIII und PvuII doppelt geschnitten und die DNA-Enden durch nachfolgende Behandlung mit dem großen Fragment der E.coli DNA Polymerase (Klenow Enzym) in Gegenwart der vier Desoxynukleotidtriphosphate stumpf gemacht. Ein entstandenes 0,36 kb DNA Fragment wurde aus einem Agarosegel isoliert und in mit EcoRI linearisiertem Plasmidvektor pCMV+M13 ligiert. Ein nach Transformation von E.coli HB101 erhaltenes Plasmid, das den SV40 ori in gleicher Orientierung wie β-Lactamase Gen und CMV-Promotor enthielt, wurde pCMV+SV40 benannt (Fig.1).

Plasmid pCMV+SV40 wurde mit EcoRI und BamHI doppelt geschnitten und die DNA-Enden anschließend mit Klenow-Enzym stumpf gemacht. Die DNA wurde durch Extraktion mit Phenol-Chloroform und Äthanolfällung gereinigt. Ein Teil der DNA wurde mit T4 DNA Ligase zirkularisiert und ein nach Transformation von E.coli erhaltenes Plasmid pAD-CMV10 benannt (Fig.2). Der Rest der pCMV+SV40 DNA wurde durch Inkubation mit alkalischer Phosphatase dephosphoryliert und der 4,4 kb lange Vektor aus einem Agarosegel isoliert.

Plasmid pSV2gptDHFR-Mut2 (siehe Beispiel 4, Fig. 9), das ein modifiziertes Hamster Dihydrofolatreduktase (DHFR) Minigen enthält, aus dem durch gerichtete Mutagenese die Restriktionsenzymschnittstellen für EcoRI, PstI, BglII, BamHI und KpnI entfernt wurden, wurde mit EcoRI und PstI doppelt geschnitten und die DNA-Enden durch 20 Minuten Inkubation bei 11°C mit 5 units T4 DNA-Polymerase (Reaktionsmedium: 50 mM Tris-Cl pH 8,0, 5 mM $MgCl_2$, 5 mM Dithiothreit, 0,1 mM jedes der vier Desoxynukleotidtriphosphate, 50 μg/ml Rinderserumalbumin) stumpf gemacht. Das 2,4 kb lange DNA-Fragment mit dem mutierten DHFR-Gen wurde aus einem Agarosegel isoliert und mit dem wie oben beschriebenen präparierten pCMV+SV40 ligiert. Ein nach Transformation von E.coli erhaltenes Plasmid, in dem das DHFR-Gen in derselben Orientierung wie der CMV-Promotor enthalten war, wurde pAD-CMV10A benannt (Fig.2).

Ausgehend vom Expressionsplasmid pAD-CMV1 (siehe Beispiel 4, Fig. 10), das zwischen Multiklonierstelle und poly-Adenylierungssignal eine Intronsequenz enthält, wurden mehrere Varianten hergestellt, die sich durch die Anzahl und Lage der Introns relativ zur Multiklonierstelle unterscheiden. In pAD-CMV13 (Fig. 4) wurde das SV40 t Antigen Intron zwischen Multiklonierstelle und poly-Adenylierungstelle deletiert; pAD-CMV15 (Fig. 3) enthält ein synthetisches Intron zwischen CMV Promotor und Multiklonierstelle und das SV40 t Antigen Intron zwischen Multiklonierstelle und poly-Adenylierungssignal; pAD-CMV19 (Fig. 4) enthält nur ein Intron zwischen CMV Promotor und Multiklonierstelle.

Ausgehend von 100 ng des mit HindIII linearisierten Plasmid pAD-CMV1 wurde mit je 50 pMol der Oligonukleotide EBI-2625 (5'-CACTGATCTAGAGATATCTTGTTTATTGCAGCTTATAATGG-3') und EBI-1857 (5'-GGCAAGGGCAGCAGCCGG-3') in 100 μl PCR Ansatz (siehe oben) in 10 PCR Zyklen (40 sec 94°C, 45 sec 55°C, 90 sec 72°C) ein 1,26 kb langes DNA Fragment amplifiziert. EBI-2625 bindet kurz vor dem SV40 poly-Adenylierungssignal (Position 1280 in pAD-CMV1) und enthält zusätzliche Restriktionsschnittstellen für XbaI und EcoRV. EBI-1857 bindet am komplementären DNA Strang im ersten Intron des nachfolgenden DHFR Minigens (Position 2525 in pAD-CMV1). Das PCR Produkt wurde durch Extraktion mit Phenol und Chloroform von Protein befreit und die DNA mit Äthanol gefällt. Die DNA wurde mit XbaI und BglII doppelt geschnitten, ein 0,32 kb langes DNA Fragment aus einem Agarosegel isoliert und in mit den gleichen Enzymen doppelt geschnittenen Plasmidvektor (5,8 kb) pAD-CMV1 ligiert. Ein nach Transformation von E.coli HB101 erhaltenens Plasmid der gewünschten Beschaffenheit (siehe Fig.4) wurde pAD-CMV13 benannt.

Die dem CMV Promotor folgende Spleiß-Donor Sequenz (M. Boshart et al., Cell 41 (1985) 521-530) wurde durch SOE-PCR (splicing by overlap extension; S.N. Ho et al., Gene 77 (1989) 51-59) mit der Spleiß-Acceptorstelle des ersten Introns des humanen β-Globin Gens (Lawn et al., Cell 21 (1980) 647-651) gefolgt von der Multiklonierstelle von Plasmid pAD-CMV1 verbunden. Dazu wurden 100 ng Plasmid pGJ7 (G. Jahn et al., J. Virology 49 (1984) 363-370) enthaltend die Promotor und Enhancer Sequenz von humanem Cytomegalovirus StammAD169 (Boshart et al., Cell 41 (1985) 521-530) mit je 50 pMol der Oligonukleotide EBI-2133 (siehe oben) und EBI-2586 (5'-GCAGAGAGAGTCAGTGCCTATCAGAAACCCAAGAG- TCTTCTCTATAGGCGGTACTTACCTGACTCTTG-3') in 100 µl PCR Reaktionsgemisch über 30 Zyklen amplifiziert (Zyklusbedingungen: 40 sec 94°C, 45 sec 45°C, 90 sec 72°C). Die letzten 24 Basen von EBI-2586 passen perfekt an die CMV-Sequenz (in antisense Orientierung) und die vorangehenden Basen entsprechen der β-Globin Intron Sequenz, wobei 18 Basen perfekt zur revers komplementären Sequenz von Oligonukleotid EBI-2585 passen und die überlappende DNA-Sequenz für die SOE-PCR bilden. Die PCR Produkte wurden in einem Agarosegel aufgetrennt und ein 0,8 kb DNA Fragment isoliert (Fig.3). 100 ng Plasmid pAD-CMV1 wurden in gleicher Weise mit den Oligonukleotiden EBI-2585 (5'-GCACTGACTCTCTCTGCCTATTGGTCTATTTTCCCACCCTTAGGCTGCT- GGTGCTTAACTGGCTTATCG-3') und EBI-2112 (5'-GTCCAATTATGTCACACC-3') durch PCR amplifiziert und ein 0,2 kb DNA Fragment aus einem Agarosegel isoliert. EBI-2585 enhält die letzten 45 Basen des β-Globin Introns und die fünf darauf folgenden Basen, sowie 17 Basen am 3'-Ende, die perfekt an Position 611-627 der pAD-CMV1 Sequenz hybridisieren können. EBI-2112 bindet am komplementären DNA Strang an Position 743-760 an die pAD-CMV1 Sequenz. 1/10 des isolierten 0,8 kb DNA Fragments und 1/30 des 0,2 kb DNA Fragments wurden in einem neuen 100 µl PCR Ansatz (SOE-PCR) gemischt und mit je 50 pMol der Oligonukleotide EBI-2133 und EBI-2112 in 30 PCR Zyklen (40 sec 94°C, 45 sec 45°C, 2 Min 72°C) amplifizert. Die Reaktion wurde durch Extraktion mit Phenol und Chloroform gestoppt und die DNA mit Äthanol gefällt. Die 5'-Enden des PCR Produktes wurden mit T4 Polynukleotidkinase phosphoryliert (Reaktionspuffer: 70 mM Tris-Cl pH 7,6, 10 mM $MgCl_2$, 5 mM Dithiothreit, 1 mM ATP) und anschließend mit XbaI geschnitten. Die DNA wurde in einem Agarosegel aufgetrennt und ein Fragment von 0,98 kb Länge isoliert. Plasmid pAD-CMV10 wurde mit PvuII und XbaI doppelt geschnitten und der Vektoranteil ohne CMV Promotor aus einem Agarosegel isoliert. Dieser Plasmidvektor wurde mit dem 0,97 kb DNA Fragment, enthaltend den CMV Promotor und Enhancer mit Intron und Multiklonierstelle, ligiert und E.coli HB101 transformiert. Von den erhaltenen Transformanten wurde Plasmid DNA hergestellt und das neue DNA Insert mit den Oligonukleotiden EBI-2112, EBI-2586 und EBI-1733 (5'-GGTCGACATTGATTATTGACTAG-3') nach der Didesoxy-Kettenabbruch Methode (F. Sanger et al., Proc. Natl. Acad. Sci.USA 74 (1977) 5463-5467) mit modifizierter T7 DNA Polymerase (S. Tabor and C.C. Richardson, Proc. Natl. Acad. Sci.USA 84 (4767-4771); Sequenase, United States Biochemical Corp.) sequenziert. Ein Plasmid mit der erwarteten Sequenz wurde pAD-CMV15 benannt (Fig.3).

pAD-CMV10A wurde mit SpeI und BglII doppelt geschnitten und der Vektoranteil ohne CMV Promotor aus einem Agarosegel isoliert. pAD-CMV15 wurde mit SpeI und HindIII doppelt geschnitten und ein 0,8 kb DNA Fragment enthaltend den CMV Promotor und das synthetische Intron, isoliert. pAD-CMV13 wurde mit HindIII und BglII doppelt geschnitten und ein 0,36 kb DNA Fragment isoliert, das die Multiklonierstelle, das SV40 early poly-Adenylierungsignal und einen Teil der Hamster-DHFR Promotorregion enthielt. Diese drei DNA Fragmente wurden mit T4 DNA Ligase ligiert und E.coli HB101 transformiert. Von den erhaltenen Transformanten wurde Plasmid DNA hergestellt und durch Schneiden mit verschiedenen Restriktionsenzymen charakterisiert. Ein Plasmid der gewünschten Struktur wurde pAD-CMV19 benannt (Fig.4, Fig.5).

Beispiel 2

Herstellung einer modifizierten cDNA für huIFN-α2c

Die für humanes IFN-α2c kodierende cDNA des Klons 1F7 (E. Dworkin-Rastl et al., J.Interferon Res. 2 (1982) 575-585; E. Dworkin-Rastl et al., Gene 21 (1983) 237-248) wurde mittels PCR in der 5'-nicht kodierenden Region modifiziert, indem diese gegen die Sequenz der 5'-nicht kodierenden Region der humanen β-Globin mRNA (Lawn et al., Cell 21 (1980) 647-651) ausgetauscht wurde. Eine derartige Veränderung der 5'-nicht kodierenden Region bewirkt eine deutliche Erhöhung der Expression, möglicherweise durch eine effizientere Initiation der Translation. Gleichzeitig wurden an beiden Enden der cDNA Restriktionsenzymschnittstellen eingeführt, die eine nachfolgende gerichtete Klonierung der cDNA in Expressionsplasmide erleichterten.

100 ng mit EcoRI linearisiertes Plasmid 1F7 wurden mit je 50 pMol der Oligonukleotide EBI-2747 (5'-CTTCAGAAGCTTACATTTGCTTCTGACACAACTGTGTTCACTAGCAACCT- CAAACAGACACC<u>ATG</u>GCC-TTGACCTTTGCTTTAC-3') und EBI-2744 (5'-GACTTCAGTCTAGAGAACCAGTTT<u>TCA</u>TTCCTTACTTC-3') in 100 µl PCR Ansatz in 20 Zyklen (40 sec 94°C, 45 sec 55°C, 90 sec 72°C) amplifiziert. EBI-2747 enthält nach einer HindIII Schnittstelle die 5'-nicht kodierende Region der humanen β-Globin mRNA gefolgt von den ersten 22 Basen der für das Signalpeptid von huIFN-α2c kodierenden Sequenz (Startkodon ist unterstrichen). EBI-2744 bindet am komplementären Strang am Ende der für huIFN-α2c kodierenden Sequenz (Stopkodon ist unterstrichen) und enthält eine Schnittstelle für XbaI. Die Reaktion wurde durch Extraktion mit Phenol und Chloroform gestoppt und die DNA mit Äthanol gefällt. Das PCR Produkt wurde mit HindIII und XbaI an den Enden nachgeschnitten und das 0,64 kb lange DNA Fragment aus einem Agarosegel isoliert (Fig.6, Fig.7). Plasmid pAD-CMV19 wurde ebenfalls mit HindIII und XbaI doppelt geschnitten und anschließend mit dem cDNA Fragment ligiert. Nach Transformation von E.coli HB101 erhaltene Kolonien wurden zur Präparation von Plasmid DNA gezüchtet. Eines der erhaltenen Plasmide wurde über den Verlauf des insertierten HindIII-XbaI Bereiches vollständig sequenziert. Mit Ausnahme eines einzigen Basenaustausches (CTG zu TTG) im 8. Kodon des Signalpeptides, der jedoch zu keiner Änderung der kodierten Aminosäure (Leu) führte, wurde die erwartete Sequenz erhalten. Das Expressionsplasmid für sekretiertes und O-glycosyliertes huIFN-α2c wurde pAD19B-IFN benannt (Fig.6).

Beschreibung der Sequenzelemente von Plasmid pAD-CMV19 (Fig.5)

| Basen | |
|---|---|
| 1 - 21 | Bindungstelle von Oligonukleotid EBI-2133 |
| 1 - 590 | Cytomegalovirus Enhancer und Promotor |
| 722 - 740 | Intronsequenz von Cytomegalovirus (Splice Donor) |
| 741 - 805 | Intronsequenz von humanem ß-Globin (Splice Acceptor) |
| 836 - 853 | T7 Promotor |
| 862 - 922 | Multiklonierstelle |
| 923 -1055 | Polyadenylierungsstellen von SV40 |
| 1056 -1953 | Promotor und 5'-nicht kodierende Region von Hamster DHFR Gen |
| 1954 -2039 | DHFR Exon 1 |
| 2040 -2333 | DHFR Intron 1 |
| 2151 -2168 | Bindungstelle von EBI-1857 |
| 2344 -2821 | DHFR Exons 2-6 kodierender Bereich |
| 2822 -3474 | DHFR 3'-nicht kodierende Region |
| 3475 -3812 | SV40 Replikationsursprung (SV40 ori) |
| 3813 -6055 | pBluescript Anteil |
| 3813 -4291 | M13 intergenische Region (M13 ori) |
| 4423 -5283 | ß-Lactamase, kodierende Region |
| 6038 -6062 | Bindungstelle von EBI-2134 |

Beispiel 3

Transiente Expression von huIFN-α2c in höheren eukaryotischen Zellen

Etwa $10^6$ Zellen (293, humane embryonale Nierenzellen transformiert mit einem Teil des Adenovirus AD5 Genoms; F.L. Graham et al., J.Gen.Virol., 36 (1977) 59-77; ATCC CRL1573) pro 80 mm Petrischale wurden 24 Stunden vor der Transfektion mit Medium (Dulbecco's MEM/Nutrient Mix F12 (1:1) mit 15 mM Hepes; Gibco) mit 10% hitzeinaktiviertem fötalem Kälberserum angesetzt und bei 37°C in 5% $CO_2$ Atmosphäre inkubiert. Die Zellen wurden 3 Stunden vor der Transfektion mit 10 ml frischem Medium versehen und bei 37°C inkubiert. 10 µg Plasmid DNA (gereinigt durch zweimalige CsCl Dichtegradientenzentrifugation) pAD19B-IFN gelöst in 0,5 ml 250 mM $CaCl_2$ wurden tropfenweise zu 0,5 ml 2x HBS (16,36 g/l NaCl, 11,9 g/l Hepes, 0,40 g/l $Na_2HPO_4$, pH 7,12) zugefügt. Das entstandene Präzipitat wurde zu einer Petrischale zugegeben und die Zellen weitere 4 Stunden bei 37°C inkubiert. Die Zellen wurden mit PBS gewaschen, 30 Sekunden mit 15% Glyzerin in lx HBS geschockt, nochmals mit PBS gewaschen und mit 10 ml frischem Medium mit 10% Kälberserum bei 37°C inkubiert. Nach 72 Stunden wurde der Zellüberstand geerntet und zum Nachweis des sekretierten IFN verwendet.

Beispiel 4:

Konstruktion der Expressionsplasmide pAD-CMV1 und pAD-CMV2

Aus Teilen der Expressionsplasmide pCDM8 (Seed & Aruffo, Proc. Natl.Acad.Sci.USA 84 (1987) 8573-8577; Seed, Nature 329 (1987) 840-842; Invitrogen Inc., San Diego, CA), pSV2gptDHFR20 (EP-A1 0321 842) und dem Plasmid Bluescript SK+ (Short et al., Nucleic Acids Res., 11 5521-5540; Strategene, La Jolla, CA) wurde ein neues Plasmid konstruiert, das eine Multiklonierstelle für die gerichtete Insertion heterologer DNA-Sequenzen aufweist und sich in E.coli mittels Ampicillinresistenz mit hoher Kopienzahl vermehren läßt. Die intergenische Region von M13 ermöglicht die Herstellung einzelsträngiger Plasmid-DNA mittels Superinfektion der transformierten Bakterien mit einem Helferphagen (z.B. R408 oder M13K07) zur erleichterten Sequenzierung und Mutagenese der Plasmid-DNA. Der T7 Promotor, der der Multiklonierstelle vorangeht, ermöglicht die Herstellung von RNA Transkripten in vitro. In Säugetierzellen erfolgt die Expression heterologer Gene getrieben vom Cytomegalovirus (CMV) Promotor/Enhancer (Boshart et al., Cell 41 (1985) 521-530). Der SV40 Replikationsurprung ermöglicht in geeigneten Zellinien (z.B. SV 40 transformierte Zellen wie COS-7, Adenovirus transformierte Zellinie 293 (ATCC CRL1573)) die autonome Replikation des Expressionsplasmides zu hohen Kopienzahlen und damit hohe Raten in transienter Expression. Für die Herstellung permanent transformierter Zellinien und die nachfolgende Amplifikation der Expressionskassette mittels Methotrexat dient ein modifiziertes Hamster-Minigen (Promotor mit kodierendem Bereich und dem ersten Intron) für Dihydrofolatreduktase (DHFR) als Selektionsmarker.

a) Herstellung der Vektor- und Promotoranteile durch PCR

Das Plasmid Bluescript SK+ wurde mit HindIII linearisiert und 5 ng DNA in einem 100 µl PCR Ansatz eingesetzt (Reaktionspuffer: 50 mM KCl, 10 mM Tris-Cl pH=8,3, 1,5 mM $MgCl_2$, 0,01% (w/v) Gelatine, 0,2 mM der vier Desoxynukleotidtriphosphate (dATP, dGTP, dCTP, dTTP), 2,5 Einheiten Taq Polymerase pro 100 µl). Als Primer wurden je 50 pmol der synthetischen Oligonukleotide EBI-1786 (5'-GGAATTCAGCCTGAA-TGGCGAATGGG-3'; bindet knapp außerhalb von M13 ori-Region in Bluescript Pos. 475, unabhängig von M13 ori-Orientierung) und EBI-1729 (5'-CCTCGAGCGTTGC- TGGCGTTTTTCC-3'; bindet an Bluescript an Pos. 1195 vor ori, entspricht dem Anfang der Bluescript-Sequenz in pCDM8, 6 Basen 5'ergeben XhoI) eingesetzt. Nach 5 Minuten Denaturieren bei 94°C erfolgte die PCR über 20 Zyklen (40 sec bei 94°C, 45 sec bei 55°C, 5 Min bei 72°C, Perkin Elmer Cetus Thermal Cycler). Die Oligonukleotide flankieren die intergenische Region von M13 bzw. den Replikationsursprung (ori) mit dem dazwischenliegenden Gen für die β-Lactamase. Gleichzeitig wird am Ende des Replikationsursprungs eine XhoI- und am anderen Ende eine EcoRI-Schnittstelle erzeugt. Das Reaktionsgemisch wurde durch Extraktion mit Phenol-Chloroform von Protein befreit und die DNA mit Ethanol präzipitiert. Die erhaltene DNA wurde mit XhoI und EcoRI geschnitten und nach Elektrophorese in einem Agarosegel ein Fragment mit 2,3 kb isoliert.

5 ng mit SacII linearisiertes Plasmid pCDM8 wurden mit den Oligonukleotiden EBI-1733 (5'-GGTCGACATTGA- TTATTGACTAG-3'; bindet an CMV-Promotorregion (Pos. 1542) von pCDM8, entspricht Pos.l in pAD-CMV, SalI-Stelle für Klonierung) und EBI-1734(5'-GGAATTCCCTAGGAATACAGCGG-3'; bindet an Polyoma origin von 3'SV40 polyA-Region in pCDM8 (Pos. 3590)) unter identischen Bedingungen wie für Bluescript SK+ beschrieben, durch PCR amplifiziert. Die Oligonukleotide binden am Beginn der CMV-Promotor/Enhancer-Sequenz und erzeugen eine SalI Schnittstelle (EBI-1733) bzw. binden am Ende der SV40 poly-Adenylierungstelle und erzeugen eine EcoRI Schnittstelle (EBI-1734). Das PCR-Produkt wurde mit SalI und EcoRI geschnitten und ein DNA Fragment von 1,8 kb aus einem Agarosegel isoliert.

Die beiden PCR Produkte wurden mit T4 DNA-Ligase ligiert, mit dem erhaltenen Ligationsprodukt E.coli HB101 transformiert und nach Standardmethoden Plasmid-DNA amplifiziert und präpariert. Das Plasmid der gewünschten Beschaffenheit (siehe Fig.8) wurde pCMV-M13 benannt.

Der SV40 Replikationsursprung (SV40 ori) wurde aus dem Plasmid pSV2gptDHFR20 (EP-A1 0321842) isoliert. Dazu wurde dieses Plasmid mit HindIII und PvuII doppelt geschnitten und die DNA-Enden durch nachfolgende Behandlung mit dem großen Fragment der E.coli DNA Polymerase (Klenow Enzym) in Gegenwart der vier Desoxynukleotidtriphosphate stumpf gemacht. Ein dabei erhaltenes 0,36 kb DNA Fragment wurde aus einem Agarosegel isoliert und in mit EcoRI linearisiertem pCMV-M13 ligiert. Ein nach Transformation von E.coli HB101 erhaltenes Plasmid mit dem SV40 ori in gleicher Orientierung wie das β-Lactamase Gen und dem CMV-Promotor wurde pCMV-SV40 benannt. Die Konstruktion dieses Plasmids ist in Fig.8 dargestellt.

b) Mutagenese des DHFR-Gens

Zur Herstellung eines Expressionsplasmids mit einer vielseitigen Multiklonierstelle wurden aus dem DHFR Minigen durch gerichtete Mutagenese zwei und durch Deletion drei Restriktionsenzymschnittstellen entfernt. Dazu wurde aus dem Plasmid pSV2gptDHFR20 ein 1,7 kb BglII Fragment, das die gesamte kodierende Region des Hamster DHFR-Gens enthält, in die BglII Stelle des Plasmids pUC219 (IBI) kloniert und das Plasmid pUCDHFR erhalten. Mit pUCDHFR transformierte E.coli JM109 (Stratagene) Zellen wurden mit etwa 40-fachem Überschuß des Helferphagen R408 (Stratagene) infiziert und 16 Stunden bei 37°C in LB-Medium geschüttelt. Aus dem Bakterienüberstand wurde einzelsträngige Plasmid-DNA isoliert.

Die gerichtete Mutagenese erfolgte in zwei aufeinanderfolgenden Schritten, wobei das in vitro Mutagenese System RPN1523 (Amersham) verwendet wurde. Die am Beginn von Exon 2 befindliche EcoRI Stelle wurde durch Austausch einer Base von GAATTC zu GAGTTC zerstört. Dieser Basenaustausch führt zu keiner Änderung der kodierten Aminosäuresequenz und entspricht außerdem der Nukleotidsequenz im natürlichen murinen DHFR-Gen (McGrogan et al., J. Biol. Chem. 260 (1985) 2307-2314; Mitchell et al., Mol. Cell. Biol. 6 (1986) 425-440). Für die Mutagenese wurde ein Oligonukleotid (Antisense-Orientierung) der Sequenz 5'-GTACTTGA- ACTCGTTCCTG-3' (EBI-1751) verwendet. Ein Plasmid mit der gewünschten Mutation wurde, wie oben beschrieben, als Einzelstrang-DNA präpariert und die im ersten Intron befindliche PstI Stelle durch Mutagenese mit dem Oligonukleotid EBI-1857 (Antisense Orientierung, 5'-GGCAAGGGCAGCAGCCGG-3') von CTGCAG in CTGCTG entfernt. Die Mutationen wurden durch Sequenzierung bestätigt und das erhaltende Plasmid pUCDHFR-Mut2 benannt. Aus dem Plasmid pUCDHFR-Mut2 wurde das 1,7 kb BglII Fragment isoliert und in mit BglII und BamHI doppelt geschnittenes Plasmid pSV2gptDHFR20 ligiert. Nach Transformation von E.coli, Amplifikation und DNA-Isolierung wurde ein Plasmid der gewünschten Beschaffenheit erhalten, das als pSV2gptDHFR-Mut2 bezeichnet wurde. Durch Schneiden mit BamHI wurde in der 3'-nicht-kodierenden Region des DHFR Gens ein auf die BglII Stelle folgendes 0,12 kb DNA-Fragment entfernt, das außerdem noch eine KpnI Schnittstelle enthält. Durch Verknüpfen der mit BglII und BamHI entstandenen überhängenden DNA-Enden wurden auch die Erkennungssequenzen für diese beiden Enzyme zerstört.

Das Plasmid pCMV-SV40 wurde mit EcoRI und BamHI doppelt geschnitten, die DNA-Enden nachfolgend mit Klenow-Enzym stumpf gemacht. Die DNA wurde durch Extraktion mit Phenol-Chloroform und Ethanolfällung gereinigt, anschließend durch Inkubation mit alkalischer Phosphatase dephosphoryliert und die 4,4 kb lange Vektor DNA aus einem Agarosegel isoliert.

Das Plasmid pSV2gptDHFR-Mut2 (Fig.9) wurde mit EcoRI und PstI doppelt geschnitten und die DNA-Enden durch 20 Minuten Inkubation bei 11°C mit 5 Einheiten T4 DNA-Polymerase (50 mM Tris-HCl pH=8,0, 5 mM $MgCl_2$, 5 mM Dithiothreit, 0,1 mM jedes der vier Desoxynukleotidtriphosphate, 50 µg/ml Rinderserumalbumin) stumpf gemacht. Das 2,4 kb lange DNA-Fragment mit dem mutierten DHFR-Gen wurde aus einem Agarosegel isoliert und mit dem wie oben beschrieben präparierten pCMV-SV40 ligiert. Ein nach Transformation von E.coli erhaltenes Plasmid, das das DHFR-Gen in derselben Orientierung wie den CMV-Promotor enthielt, wurde pCMV-SV40DHFR benannt. Im letzten Schritt wurde das 0,4 kb "Stuffer"-Fragment nach dem CMV-Promotor,das noch aus dem Ausgangsplasmid pCDM8 stammte, gegen eine Multiklonierstelle ausgetauscht. Dazu wurde das Plasmid pCMV-SV40DHFR mit HindIII und XbaI doppelt geschnitten und der Vektoranteil aus einem Agarosegel isoliert. Die Multiklonierstelle, gebildet aus den beiden Oligonukleotiden EBI-1823 (5'-AGCTTCTGCAGGTCGA- CATCGATGGATCCGGTACCTCGAGCGGCCGCGAATTCT-3') und EBI-1829 (5'-CTAGAGAATTCGCGGCCGCTCGAGGTACCGGATCCATCGATG- TCGACCTGCAGA-3'), enthält inklusive der für die Klonierung in HindIII - XbaI kompatiblen Enden Restriktionsschnittstellen für die Enzyme PstI, SalI, ClaI, BamHI, KpnI, XhoI, NotI und EcoRI.

Je 1 µg der beiden Oligonukleotide wurden in 20 µl Reaktionspuffer (70 mM Tris-Cl pH=7,6, 10 mM $MgCl_2$, 5 mM Dithiothreit, 0,1 mM ATP) mit 5 Einheiten T4 Polynukleotidkinase eine Stunde bei 37°C inkubiert, um die 5'-Enden zu phosphorylieren. Die Reaktion wurde durch 10 minütiges Erhitzen auf 70°C gestoppt und die komplementären Oligonukleotide miteinander hybridisiert, indem die Probe weitere 10 Minuten bei 56°C inkubiert und anschließend langsam auf Raumtemperatur abgekühlt wurde. 4 µl der hybridisierten Oligonukleotide (100 ng) wurden mit etwa 100 ng Plasmidvektor ligiert und E.coli HB101 transformiert. Ein Plasmid, das sich mit den Enzymen der Multiklonierstelle (ausgenommen NotI) linearisieren ließ, wurde pAD-CMV1 benannt. Von vielen getesteten Klonen konnte keiner identifiziert werden, dessen Plasmid sich mit NotI schneiden ließ. Die Sequenzierung zeigte immer die Deletion von einigen Basen innerhalb der NotI Erkennungssequenz. In gleicher Weise wurde mit dem Oligonukleotidpaar EBI-1820 (5'-AGCTCTAGAGAATT-CGCGGCCGCTCGAGGTACCGGATCCATCGATGTCGACCTGCAGAAGCTTG-3') und EBI-1821 (5'-CTAGCAAGCTTCTGCAGGTCGACATCGATGGATCC GGTACCTCGAGCGGCCGCGAATTCTCTAG-3') das Expressionsplasmid pAD-CMV2 hergestellt, das die Restriktionsschnittstellen innerhalb der Multiklonierstelle in umgekehrter Reihenfolge enthält. Dabei wurde das Plasmid pAD-CMV2 erhalten, das sich mit sämtlichen Restriktionsenzymen, einschließlich NotI, linearisieren ließ.

Die Nukleotidsequenz des 6414 bp großen Plasmids pAD-CMV1 (Fig.10) ist in Fig.11 vollständig dargestellt.

Die Abschnitte auf dem Plasmid (angegeben in der Numerierung der Basen) entsprechen folgenden Sequenzen:

| | |
|---|---|
| 1-21 | EBI-1733, Beginn CMV Enhancer - Promotor (aus CDM8) |
| 632-649 | T7 Promotor |
| 658-713 | Multiklonierstelle (HindIII bis XbaI aus EBI-1823, EBI-1829) |
| 714-1412 | SV40 Intron und poly-Adenylierungsstelle (aus CDM8) |
| 1413-2310 | 5'nicht kodierende Region und Promotor des Hamster DHFR Gens (aus pSV2gptDHFR20) |
| 2311-2396 | Hamster DHFR: Exon 1 |
| 2516 | A zu T Mutation zerstört PstI Stelle in DHFR Intron 1 |
| 2701-3178 | DHFR Exons 2-6 (kodierende Region) |
| 2707 | A zu G Mutation zerstört EcoRI Stelle |
| 3272-3273 | Deletion zwischen BglII und BamHI in DHFR 3'nicht kodierender Region |
| 3831 | Ende DHFR Gen (aus pSV2gptDHFR20) |
| 3832-4169 | SV40 ori (aus pSV2gptDHFR20) |
| 4170-4648 | M13 ori (aus pBluescript SK+) |
| 4780-5640 | β-Lactamase (kodierende Region) |
| 6395-6414 | EBI-1729, Ende der pBluescript Vektorsequenz |

Die Herstellung der Plasmide pAD-CMV1 und pAD-CMV2 ist in Fig.10 dargestellt.

Beispiel 5

Entwicklung von rekombinanten "Chinese hamster ovary (CHO)"-Zellinien, die glycosyliertes Human-Interferon-α2 produzieren

a) Transfektion von CHO-Zellen und Selektion stabil transfizierter Zellinien

Die parentalen Zellinien, CHO-DXB11 und CHO-DG44 (Proc. Natl. Acad. Sci. USA 77, 4216-4220, 1980; Som. Cell. Molec. Genet. 12, 555-666, 1986) wurden in Roswell Park Memorial Institute (RPMI) Medium 1640 supplementiert mit 10% fötalem Rinderserum, Hypoxanthin (100 μM), Thymidin (16 μM), Natrium-Penicillin G (100 Einheiten/ml), und Streptomycin (50 Einheiten/ml) gezüchtet. Zwei Tage vor der Transfektion wurden die Zellen in 25 $cm^2$ - Flaschen angesetzt; zum Zeitpunkt der Transfektion waren die Zellen nahezu konfluent.

Das Transfektionsexperiment wurde wie folgt durchgeführt. 20 μl einer Lösung von Plasmid pAD19B-IFN (1μg/ml) wurden mit 125 μl 2 M $CaCl_2$ und 855 μl sterilem deionisiertem Wasser verdünnt. Diese Lösung wurde tropfenweise zu 1 ml 2 x HSB zugesetzt (1 x HSB enthält pro Liter Lösung: 8,18 g NaCl, 5,94 g HEPES, 0,2 g $Na_2HPO_4$; pH 7,1). Das Kulturmedium der CHO-Zellen wurde entfernt, und 0,25 ml der Suspension wurden zu jeder Flasche zugesetzt; die Kulturen wurden 4 Stunden bei 37°C inkubiert. Die Suspension wurde dann entfernt, die Zellen wurden mit Hilfe von Trypsin/EDTA-Lösung von der Oberfläche gelöst und in Selektionsmedium suspendiert (das Selektionsmedium bestand aus Minimum Essential Medium, alpha-Modifikation ohne Ribonucleotide and Deoxyribonucleotide, ergänzt mit 10% dialysiertem fötalem Rinderserum, Natrium-Penicillin G 100 Einheiten/ml, Streptomycin 50 Einheiten/ml, and Amphotericin B 2,5 μg/ml; 40 ml pro Flasche). Die Zellsuspension wurde dann in die Vertiefungen von zwei Zellkultur-Mikrotiterplatten transferiert (96 Vertiefungen pro Platte, 0,2 ml pro Vertiefung) und zwei Wochen bei 37°C inkubiert. Das angegebene Selektionsmedium wurde, allerdings ohne Amphotericin B, auch für alle weiteren Experimente verwendet.

Die Zellkulturen wurden visuell auf Zellwachstum überprüft. Kulturmedium aus Vertiefungen, die Zellwachstum zeigten, wurden auf IFN-α2-Gehalt mit Hilfe eines Enzym-Immunoassays getestet, der zwei monoklonale Antikörper gegen IFN-α2 verwendet (Biochem. J. 276, 511-518, 1991). Dieser Test wurde mit neuen Kulturüberständen eine Woche später wiederholt. Zellen aus positiven Kulturen wurden mit Hilfe von Trypsin-/EDTA-Lösung von der Oberfläche gelöst und jeweils in Kulturplatten mit 24 Vertiefungen transferiert. Kulturen, die gutes Zellwachstum zeigten, wurden dann wiederholt auf IFN-Produktion getestet. Die IFN-α2 - Konzentrationen in den Überständen lagen typischerweise im Bereich zwischen 2.000 und >10.000 Einheiten/ml (1 ng IFN-α2 - Protein entspricht 230 Einheiten).

b) Amplifikation des IFN-α2 Gens durch Methotrexat-Selektion

Transfizierte Klone von beiden Parental-Zellinien, CHO-DXB11 und CHO-DG44, die in mehreren Tests hohe IFN-Konzentrationen gezeigt hatten, wurden für die Amplifikation ausgewählt; zusätzlich wurden jeweils 3 - 5 weitere Klone vereinigt. Diese Kulturen wurden nun in 25 $cm^2$ - Flaschen in Selektionsmedium (ohne Amphotericin B) gehalten, dem Methotrexat in Konzentrationen von 20 nM oder 50 nM zugesetzt wurde. Die Kulturen wurden wöchentlich einmal mit frischem Medium versehen. Überlebende Klone wurden nach etwa 2 - 3 Wochen beobachtet. Sobald die Zellen etwa 50% der Kulturfläche bewachsen hatten, wurden die Überstände wieder auf ihren IFN-α2 Gehalt getestet. Die Zellen wurden dann abgelöst, verdünnt und in neue Flaschen transferiert. Die Methotrexat-Konzentration wurde nun um den Faktor von etwa 2 - 5 erhöht, z.B. von 20 nM auf 50 und 100 nM, oder von 50 nM auf 100 und 200 nM. Nach mehreren derartigen Selektionszyklen in Gegenwart steigender Mengen an Methotrexat, und Selektion resistenter Kulturen nach ihrer IFN-Produktion, konnten schließlich Zellinien erhalten werden, die resistent gegen Methotrexat-Konzentrationen bis zu 5.000 nM waren und relativ große Mengen an IFN-α2 sezernierten. Die folgende Tabelle I illustriert den Anstieg der Produktivität am Beispiel der Zellinie CHO-DXB11-IFN-α2c-3/2D4.

Die nachfolgende Tabelle II zeigt die Ergebnisse mit der Zellinie CHO-DG44-IFN-α2c-pool"S":

**Tabelle I**

| Methotrexat-Konzentration (nM) | IFN-α2c in Kulturüberständen (Einheiten/ml) |
|---|---|
| 0 | 6.000 - 14.000 |
| 20 | 12.000 - 89.000 |
| 50 | 29.000 -125.000 |
| 100 | 96.000 -120.000 |
| 200 | 110.000 -190.000 |
| 500 | 140.000 -300.000 |
| 1000 | 350.000 -900.000 |
| 2000 | 200.000 -350.000 |
| 5000 | 210.000 -960.000 |

**Tabelle II**

| Methotrexat-Konzentration (nM) | IFN-α2c in Kulturüberständen (Einheiten/ml) |
|---|---|
| 20 | 29.000 - 83.000 |
| 50 | 58.000 -112.000 |
| 100 | 69.000 - 98.000 |
| 200 | 71.000 -220.000 |
| 500 | 190.000 -220.000 |
| 1000 | 170.000 -570.000 |
| 2000 | 200.000 -350.000 |
| 5000 | 190.000 -320.000 |

Aus Kulturüberständen rekombinanter CHO-Zellen konnte IFN-α2 mit Hilfe von Affinitätschromatographie an monoklonalen Antikörpern (z. B. den Antikörpern EBI-1 oder EBI-10) nach bereits bekannten Methoden gereinigt werden (z.B. Nature 285, 446-450, 1980; J. Biol. Chem. 265, 9290-9295, 1990; Biochem. J. 276, 511-518, 1991). Zur Reinigung der erfindungsgemäßen Proteine eignet sich in besonderer Weise das in der EPA 0 203 382 beschriebene Verfahren.

Beispiel 6

Charakterisierung von rekombinantem, glycosyliertem humanem IFN-α2c aus "Chinese hamster ovary (CHO)"-Zellen.

a) Reverse Phase HPLC (RP-HPLC)

Affinitätsgereinigtes rekombinantes glycosyliertes IFN-α2c aus CHO-Zellen wurde mittels RP-HPLC mit rekombinantem IFN-α2c aus E.coli, das nicht glycosyliert ist, verglichen. Die genaue Analysenmethode ist in Adolf et al., J. Biol. Chem. 265, 9290-9295 (1990) beschrieben. Glycosyliertes CHO-IFN-α2c (oberer Teil von Fig.21) besteht aus zwei Hauptpeaks (Peaks 1 und 2) und zwei kleineren IFN-Peaks (Peaks 3 und 4). Unglycosyliertes E.coli-IFN-α2c dagegen (unterer Teil von Fig.21) zeigt einen Hauptpeak (korrekte Disulfidbrücken) und einen kleineren Nebenpeak, der von einer Form mit "scrambled" Disulfidbrücken stammt. Aus dem Vergleich der Retentionszeiten sieht man, daß die zwei Hauptpeaks des CHO-IFN-α2c etwas früher eluieren als der Hauptpeak des E.coli-IFN-α2c. Der Grund für diese verringerte Hydrophobizität ist die Anwesenheit von Oligosacchariden im CHO-IFN-α2c. Die zwei kleineren IFN-Peaks im CHO-IFN-α2c haben etwa die gleiche Retentionszeit wie der Hauptpeak des E.coli-IFN-α2c und stammen damit höchstwahrscheinlich von einem kleineren unglycosylierten Anteil des CHO-IFN-α2c.

b) N-terminale Sequenzierung

Die zwei Hauptpeaks des CHO-IFN-α2c wurden von der RP-HPLC isoliert und gemeinsam sequenziert. Die Sequenzierbedingungen sind in Adolf et al., J. Biol. Chem. 265, 9290-9295 (1990) beschrieben. Die ersten 15 Aminosäuren konnten in Übereinstimmung mit der cDNA-Sequenz identifiziert werden. Es gab keine Hinweise auf eine Heterogenität am N-Terminus.

c) C-terminale Analyse

Der Hauptpeak des E.coli-IFN-α2c und die zwei Hauptpeaks des CHO-IFN-α2c wurden von der RP-HPLC isoliert und mit Trypsin gespalten. Die tryptischen Peptide wurden wieder mittels RP-HPLC getrennt. Die experimentellen Bedingungen sind in Adolf et al., Biochem. J. 276, 511-518 (1991) beschrieben. Fig.22 zeigt einen Vergleich der erhaltenen Peptide Maps. Zwischen den Peptide Maps von Peak 1 und Peak 2 des CHO-IFN-α2c (oberer und mittlerer Teil) gab es nur einen einzigen Unterschied. Das tryptische Peptid 18 aus Peak 1 ist im Peak 2 (dort als Peptid 15) nahezu nicht vorhanden. Stattdessen wurde ein neues Peptid (Nummer 19) im Map von Peak 2 gefunden, das sowohl im Peak 1 als auch im E.Coli-IFN-α2c (unterer Teil von Fig.22) überhaupt nicht vorkommt.

Die Peptide 12 (aus E.coli-IFN-α2c), 18 (aus Peak 1 des CHO-IFN-α2c), 15 und 19 (aus Peak 2 des CHO-IFN-α2c) wurden mit Plasmadesorptions-Massenspektrometrie (PD-MS) analysiert. Die experimentellen Details dafür sind in Adolf et al., Biochem. J. 276, 511-518 (1991) beschrieben. Für die ersten drei der genannten Proben wurde ein Molekulargewicht gefunden, das den Aminosäuren 150 bis 162 der IFN-α2c-Sequenz entspricht. Peptid 19 aus Peak 2 des CHO-IFN-α2c ergab dagegen ein geringeres Molekulargewicht, entsprechend den Aminosäuren 150 bis 161. Ein Teil des Peptids 19 wurde auch sequenziert, wobei sich eindeutig zeigte, daß dieses Peptid mit der Aminosäure 150 beginnt und mit LEU-161 endet. Aus diesen Ergebnissen kann geschlossen werden, daß der Peak 1 des CHO-IFN-α2c ein vollständiges IFN-Molekül enthält, während beim Peak 2 die 4 C-terminalen Aminosäuren (162-165) fehlen. Die Aminosäuren 163-165 können in einem Peptide Map nach Trypsinspaltung nicht positiv identifiziert werden, da das daraus resultierende Dipeptid (163/164) und die freie Aminosäure (165) im Totvolumen der RP-Säule eluieren. Der kleine Anteil von Peptid 15 (unverkürztes tryptisches Peptid mit den Aminosäuren 150-162), der auch im Peak 2 des CHO-IFN-α2c gefunden wurde, ist wohl auf einen kontaminierenden Anteil von Peak 1 zurückzuführen, da die Peaks 1 und 2 mittels RP-HPLC nicht vollständig getrennt werden können.

In weiteren Experimenten wurde festgestellt, daß die C-terminale Verkürzung des O-glycosylierten IFN-α2 aus CHO-Zellen verhindert werden kann, wenn für das Ablösen der Zellen von der Oberfläche der Kulturgefäße an Stelle der üblichen Trypsin/EDTA-Lösung eine trypsinfreie Lösung verwendet wird (z.B. EDTA Dinatriumsalz, 200 mg/L mit D(+)Glucose Monohydrat, 200 mg/L in phosphatgepufferter Natriumchloridlösung pH 7.4). IFN-α2, das aus derart kultivierten Zellkulturen nach den oben beschriebenen Verfahren gereinigt wurde, zeigte in der Reverse Phase - HPLC (analog zu Abbildung 21a) nur den Peak 1, der dem vollständigen Protein entspricht, aber nicht den Peak 2, der dem verkürzten Protein entspricht. Weiterhin konnte mit Hilfe der tryptischen Peptide Maps (analog zu Abbildung 22) gezeigt werden, daß das Peptid-Muster dieses ohne Verwendung von Trypsin hergestellten Proteins identisch mit dem Muster der aus dem Peak 1 generierten Peptide (Abbildung 22a) ist.

SDS-Gelelektrophorese

Die Peaks 1 and 2 des CHO-IFN-α2c wurden von der RP-HPLC isoliert. Sie wurden einzeln und im Vergleich zu E.coli-IFN-α2c sowohl unter reduzierenden (nach Kochen mit Dithiothreitol) als auch unter nichtreduzierenden Bedingungen mittels SDS-Gelelektrophorese analysiert. Die experimentellen Details sind in Adolf et al., J. Biol. Chem., 265, 9290-9295 (1990) beschrieben. Die Ergebnisse sind in Fig.23 gezeigt (Spuren 2-4 unter nichtreduzierenden Bedingungen, Spuren 5-7 unter reduzierenden Bedingungen; oberer Teil mit 4 μg IFN in jeder Spur, unterer Teil mit je 1 μg IFN). Speziell aus den Spuren 5-7 des unteren Teils ist ersichtlich, daß sowohl Peak 1 als auch Peak 2 des CHO-IFN-α2c ein höheres Molekulargewicht haben als das unglycosylierte E.coli-IFN-α2c. Wegen der nicht vollständigen Trennung der Peaks 1 und 2 bei der RP-HPLC ist eine gegenseitige Kontamination der Peaks 1 und 2 vorhanden. Aus demselben Grund ist auch der Peak 2 mit einer geringen Menge von unglycosyliertem CHO-IFN-α2c, das aus Peak 3 (siehe Fig.21) stammt, kontaminiert. Unter Berücksichtigung dieser Kontaminationen scheinen die Hauptbanden der Peaks 1 und 2 des CHO-IFN-α2c homogen zu sein. Da sich die Peaks 1 und 2 bezüglich des C-Terminus unterscheiden (siehe oben), kann aus den Ergebnissen der SDS-Gelelektrophorese geschlossen werden, daß die Oligosaccharid-Anteile der Peaks 1 und 2 des CHO-IFN-α2c identisch sind (siehe auch später in Kapitel f).

e) Deglykosylierung von CHO-IFN-α2c

Die Peaks 1 and 2 des CHO-IFN-α2c wurden von der RP-HPLC isoliert und in einem SpeedVac Konzentrator getrocknet. Diese Proben sowie E.coli-IFN-α2c wurden in 10 µl 0,1 M NaOH 20 Stunden bei Raumtemperatur inkubiert. Die durch diese β-Elimination deglycosylierten Proben wurden im Vergleich zu unbehandelten Proben mittels SDS-Gelelektrophorese analysiert. Die Ergebnisse in Fig.24 zeigen, daß das Molekulargewicht der Peaks 1 und 2 des CHO-IFN-α2c nach Behandlung mit NaOH deutlich reduziert und identisch mit dem des NaOH-behandelten E.coli-IFN-α2c ist. Das diffuse Aussehen der Banden aller mit NaOH behandelten Proben ist auf Veränderungen in der Peptidkette unter den angewandten Reaktionsbedingungen zurückzuführen.

f) Identifizierung der Glykopeptide mittels Peptide Mapping

Der Vergleich der Peptide Maps nach Trypsinspaltung von E.coli-IFN-α2c (Fig.22, unterer Teil) und von Peak 1 (vollständiger C-Terminus) des CHO-IFN-α2c (Fig.22, oberer Teil) zeigt, daß jeweils zwei Peptide unterschiedliche Retentionszeiten aufweisen. Die Peptide 18 und 21 von E.coli-IFN-α2c, die die Aminosäuren 84-112 bzw. 71-112 enthalten, kommen im Peptide Map von Peak 1 des CHO-IFN-α2c nicht vor. Stattdessen gibt es dort zwei neue Peptide (Nummer 26 und 31), die das gleiche Verhältnis der Absorptionen bei 280 und 214 nm zeigen wie die Peptide 18 und 21 von E.coli-IFN-α2c. Man kann daraus folgern, daß die Peptide 26 und 31 die glycosylierten Versionen der Aminosäuresequenzen 84-112 bzw. 71-112 darstellen.

Daher eluieren sie auch deutlich früher von der RP-Säule als die analogen Peptide von E.coli-IFN-α2c. Für die beiden möglichen Längen der tryptischen Peptide (Aminosäuren 84-112 bzw. 71-112) gibt es jeweils einen Hauptpeak (Peptid 26 bzw. 31), woraus geschlossen werden kann, daß der Oligosaccharid-Anteil weitgehend homogen ist.

Aus dem Vergleich der Peptide Maps der Peaks 1 und 2 des CHO-IFN-α2c (oberer und mittlerer Teil von Fig.22) ist ersichtlich, daß die jeweiligen Glycopeptide (26 und 31 aus Peak 1 bzw. 24 und 30 aus Peak 2) identisch sind. Daraus folgt ebenfalls, daß die vier fehlenden Aminosäuren am C-Terminus von Peak 2 den einzigen Unterschied zwischen den Peaks 1 und 2 darstellen.

Alle die Aminosäuresequenzen 84-112 bzw. 71-112 betreffenden Hauptpeptide der drei IFN-Proben wurden von RP-HPLC isoliert und mit Staphylococcus Aureus V8 Protease an der C-terminalen Seite von Glutaminsäure weitergespalten. Die exakten Bedingungen sind in Adolf et al., Biochem. J. 276, 511-518 (1991) beschrieben. Die resultierenden Peptide Maps wurden verglichen, alle unterschiedlichen Peaks wurden isoliert und mittels N-terminaler Sequenzierung und/oder Massenspektrometrie weiter analysiert.

Eines der in den Peaks 1 und 2 des CHO-IFN-α2c, aber nicht in E.coli-IFN-α2c vorkommenden Staph.A.-Peptide enthielt die Aminosäuren 97-112 der IFN-α2c-Sequenz. Über die N-terminale Sequenzierung konnte in diesem Peptid THR-106 nicht identifiziert werden. Daraus kann geschlossen werden, daß THR-106 in diesem Peptid glycosyliert vorliegt. Bei der hier verwendeten Edman-Sequenzierung werden glycosylierte Aminosäuren derivatisiert und abgespalten wie unglycosylierte Aminosäuren, sie können jedoch wegen ihrer erhöhten Hydrophilizität mit Butylchlorid nicht aus dem Reaktionsgefäß extrahiert werden. Daher kann man in diesem Abbauschritt keinerlei Aminosäure identifizieren, die Sequenz geht jedoch danach völlig ungestört weiter. Ein weiterer Hinweis darauf, daß das Oligosaccharid an THR-106 gebunden ist, ergab sich aus dem Resultat, daß die GLU-107/THR-108-Bindung durch die Staph.A.-Protease nur teilweise gespalten wurde. Offensichtlich ist die Zugänglichkeit dieser Peptidbindung durch die Anwesenheit des Oligosaccharids eingeschränkt. In dem analogen Peptid aus E.coli-IFN-α2c wird diese Peptidbindung nahezu vollständig gespalten.

Ein weiteres Staph.A.-Peptid, das nur in CHO-IFN-α2c vorkommt, wurde mittels Plasmadesorptions-Massenspektrometrie analysiert. Das erhaltene Molekulargewicht entsprach den Aminosäuren 97-112 inklusive einem Oligosaccharid, bestehend aus je einem Molekül N-Acetylgalactosamin und Galactose sowie zwei Molekülen N-Acetylneuraminsäure.

Aus diesem Resultaten ist ersichtlich, daß sowohl die Glycosylierungsstelle als auch der Oligosaccharidanteil des CHO-IFN-α2c weitgehend identisch sind mit den in natürlichem IFN-α2 aus virusstimulierten Leukocyten gefundenen Verhältnissen.

Isolierung des O-glycosylierten Interferons aus virusstimulierten Zellen:

## Methoden

Interferon Bioassay:

Die antivirale Aktivität der IFN Präparationen wurde in einem Assay, der den cytopathischen Effekt (CPE) von Enzephalomycarditis Virus (EMCV) mißt, in Mikrotiterplatten durchgeführt. Als Testzellen werden die A549 humanen Lungencarcinomzellen verwendet. Details dieses Assays sind beschrieben worden (z.B. Adolf, G.R., J.Gen.Virol. 68, 1669-1676 (1987)). Bei jedem Bioassay wurden alle Titrationen zweimal durchgeführt. Eine Laborstandard-Präparation an rekombinantem in E. coli produziertem humanem IFN-α2c wurde in jedem Assay mitgeführt: die Aktivität dieser Präparation wurde kürzlich durch Vergleich mit der internationalen Referenzpräparation für human IFN-α2, Gxa 01-901-535 ermittelt. Alle beobachteten IFN-Aktivitäten wurden korrigiert im Hinblick auf die definiert Wirksamkeit dieser Referenzpräparation.

Interferon ELISA:

Ein ELISA wurde etabliert, der zwei neutralisierende murine IgG MAbs für IFN-α und eine IFN-α2c Laborreferenzpräparation (s. oben) als Standard verwendet. Die Herstellung der Antikörper und ihre Eigenschaften sind beschrieben (Adolf et al. J. Cell Physiol. suppl. 2, 61-68(1982); Adolf G.R. J. Gen. Virol. 68, 1669-1676 (1987)). Der Antikörper EBI-1 wurde zur Beschichtung der Assay Platten verwendet; der Antikörper EBI-10, kovalent gekoppelt an Meerrettich Peroxidase, wurde mit der zu untersuchenden Probe zugegeben. O-Phenylendiamin und Natriumperborat wurden als Substrate für das Enzym verwendet; die Reaktion wurde durch Zugabe von Schwefelsäure unterbrochen und die Absorption des resultierenden Produktes gemessen (492 nm, Referenz 690 nm).

Reinigung des natürlichen human IFN-α2:

Eine Affinitätssäule wurde durch Kopplung von 12 mg des monoklonalen Antikörpers, beispielsweise des MAb EBI-10 (gereinigt aus dem Maus-Ascites durch Ammoniumsulfat Präzipitation und Protein G Affinitätschromatographie nach Standardmethoden) an 1g CNBr-aktivierter Sepharose 4B nach den Empfehlungen des Herstellers (Pharmacia) hergestellt. Das endgültige Bettvolumen der Säule betrug annähernd 3 ml. Teilweise gereinigtes human Leukozyteninterferon (Cantell et al. Methods Enzymol. 78, 29-38 (1981); Cantell et al. ibid. 499-505) bei dem der IFN-ω Anteil entfernt worden war (Adolf et al. J. Biol. Chem. 265, 9290-9295 (1990)) und das etwa 2-3 x $10^6$ IU/ml mit einer totalen Protein Konzentration von 2 mg/ml enthielt, wurde mit einer Durchflußrate von 1 ml/Min auf die Säule aufgetragen (200 und 350 ml). Die Säule wurde dann mit 0,1 M Natriumphosphat Puffer pH 7,5 (Puffer A) gewaschen und mit einem Lineargradienten aus Puffer A und Puffer B (0,1 M Natriumcitrat pH 2,1) in einem FPLC-System (Pharmacia) bei einer Durchflußrate von 1 ml/Min eluiert. Die erhaltenen Fraktionen wurden auf IFN-Aktivität mit Hilfe des ELISA geprüft. Entsprechende Fraktionen beider Ansätze wurden gesammelt, mit 1 M NaOH neutralisiert und erneut auf dieselbe Säule aufgetragen, die mit Puffer A reäquilibriert worden war. Dasselbe Elutionsprogramm wurde verwendet. (Durchflußrate 0,25 ml/Min) Entsprechende Fraktionen wurden wieder gesammelt, neutralisiert und in Aliquots eingefroren.

SDS Gelelektrophorese, HPLC-Techniken und Aminosäuresequenzierungen:

SDS Polyacrylamid Gelelektrophorese und Reverse Phase HPLC wurden verwendet um das gereinigte IFN-α2 zu analysieren;sämtliche Methoden sind ausführlich beschrieben worden (Adolf et al., J. Biol. Chem. 265, 9290-9295 (1990)). Die Bestimmung der N-terminalen Sequenz wurde in einem automatischen Sequenator (Applied Biosystems, Modell 477A) durchgeführt; Aminosäurederivate wurden on-line durch RP-HPLC analysiert (Adolf et al., J. Biol. Chem. 265, 9290-9295 (1990)).

"Mapping" der proteolytischen Peptide:

Affinitätsgereinigtes IFN-α2 wurde weiterhin durch Reverse Phase HPLC gereinigt, denaturiert und entsalzt wie bei Adolf et al., J. Biol. Chem. 265, 9290-9295 (1990) beschrieben. Die Peakfraktionen wurden gesammelt und in einem SpeedVac Konzentrierer getrocknet. 29 µg (Peak 1) und 66 µg (Peak 2) Protein wurden in 0,1 ml 1%iger Ammoniumbicarbonatlösung aufgelöst; 0,5 bzw. 1 µg Trypsin (Boehringer Mannheim) in 3 bzw.

6 µl 0,01 %iger Trifluoressigsäure wurden zugegeben und die Reaktionsmischung wurde bei 37°C inkubiert. Nach 6 h Inkubationszeit wurde dieselbe Menge Trypsin erneut zugegeben und für weitere 18 h inkubiert. Die Reaktionsmischung wurde vor der Analyse durch Zugabe von 10 µl 0,5 M Dithiothreitol und 100 µl 6 M Harnstoff 2 h bei Raumtemperatur reduziert. Reverse Phase HPLC wurde auf einer Delta Pak C18 Säule (Waters; 3,9 x 150 mm; Teilchengröße 5 µm; Porendurchmesser 100°A) bei 30°C unter Verwendung folgender Lösungsmittel durchgeführt: Lösungsmittel A: 0,1 % Trifluoressigsäure in Wasser; Lösungsmittel B: 0,1 % Trifluoressigsäure in Acetonitril. Das folgende Gradientenprogramm wurde verwendet (Durchflußrate 1 ml/Min): 0-55 Min: 0-55 % B (linearer Gradient); 55-70 Min: 50 % B. Detektiert wurden die Peptide durch ihre Absorption bei 214 und 280 nm. Die resultierenden Muster wurden mit denen des rekombinanten aus E. coli stammenden IFN-α2c verglichen. Die Peptide des natürlichen IFN-α2, die sich in ihrem Elutionsverhalten anders verhielten als ihre rekombinanten Gegenstücke wurden gesammelt und N-terminal sequenziert oder wurden mit Staphylococcus aureus V8 Protease weiter abgebaut (Endopeptidase Glu-C, Boehringer Mannheim). 0,88 µg (Peak I/I), 2,6 µg (Peak 2/Ia) und 1,5 µg (Peak 2/Ib) der Peptide wurden jeweils in 0,1 ml 25 mM Phosphatpuffer pH 7,8 gelöst. In Wasser gelöste Protease wurde zugegeben (17,5 ng, 52,5 ng bzw. 29 ng) und die Reaktionsmischung wurde bei 37°C inkubiert. Nach 6 h wurden dieselben Mengen Protease erneut zugegeben und 18 h inkubiert. Die Proben wurden daraufhin einer Reverse Phase HPLC Analyse unterzogen (s. oben). Entsprechende Fraktionen wurden gesammelt und N-terminal sequenziert.

Deglykosylierung des IFN-α2:

Gereinigtes, denaturiertes und entsalztes IFN-α2 wurde mit Vibrio cholerae Neuraminidase (Boehringer Mannheim) (50 mU/ml, 18 h bei 37°C in 20 µl 50 mM Natriumacetat pH 5,5, 4 mM $CaCl_2$) und/oder Endo-α-N-acetyl-galactosaminidase -ist gleich O-Glycanase- (Boehringer Mannheim) (100 mM/ml, 18 h bei 37°C im selben Puffer) behandelt. Chemische Eliminierung wurde durch Inkubation in 0,1 M NaOH 20 h bei Raumtemperatur erreicht.

Plasmadesorptions-Massenspektrometrie:

Massenspektren der tryptischen Peptide wurden auf einem "BIO-ION 20 time-of-flight" Massenspektrometer (BIO-ION Nordic AB, Uppsala, Schweden) gemessen. Die Proben wurden in wäßriger Trifluoressigsäure (0,1 %) gelöst und auf Nitrozellulose-beschichtete Targets aufgebracht (BIO-ION). Die spektralen Akkumulationszeiten bewegten sich zwischen 0,5 und 12 h, abhängig von der Ausbeute. Die Spektren wurden gemessen bei einer Beschleunigungsspannung von 17 kV.

Beispiel 7:

Reinigung des natürlichen human IFN-α2

Humanes Leukozyten-Interferon, erhalten aus Sendai Virus induzierten humanen peripheren Leukozyten und teilweise gereinigt nach dem Reinigungsverfahren von Cantell et al. (Methods Enzymol 78, 29-38 und 78, 499-512 (1981)), wurde als Ausgangsmaterial für die Isolierung und Reinigung des IFN-α2 verwendet. Durch selektive Affinitätschromatographie mit Anti IFN-ω monoklonalen Antikörper, beispielsweise OMG-4, OMG-5 oder OMG-7 war der Anteil an IFN-ω entfernt worden (Adolf et al. Virology 175, 410-417 (1990); EPA 262 571). Die spezifische antivirale Aktivität betrug 1-2x$10^6$ IU/mg; IFN-α, mit einer spezifischen Aktivität von 2x$10^8$ IU/mg war demnach nur mit etwa 1% des gesamten Proteinanteils vertreten. Zur Reinigung des IFN-α2 von kontaminierenden Fremdproteinen und gleichzeitig von anderen IFN-α Spezies wurden hoch selektive Anti IFN-α2 monoklonale Antikörper verwendet. Diese Antikörper besitzen in standardisierten Neutralisations-Bioassays hohe Spezifität für das IFN-α2 (Adolf G.R. J. Gen. Virol. 68, 1669-1676 (1987)).

Eine Immunoaffinitätssäule wurde hergestellt, indem ein solcher monoklonaler Antikörper, beispielsweise der EBI-10 hergestellt z.B. gemäß J. Gen. Virol. 68, 1669-1676 (1987) oder DE 33 06 060.6 an CNBr-aktivierte Sepharose 4B gekoppelt wurde. Der Antikörper war aus dem Maus-Ascites durch Ammoniumsulfat Präzipitation und Protein G Affinitätschromatographie nach Standardverfahrensweisen gereinigt worden. Verwendet wurden beispielsweise 12 mg des monoklonalen Antikörpers EBI-10, die an lg CNBr-aktivierter Sepharose 4B gekoppelt wurden, wobei die vom Hersteller empfohlenen Bedingungen eingehalten wurden (Pharmacia). Das endgültige Bettvolumen der Säule betrug etwa 3 ml.

Die Leukozyten-Interferon Präparation wurde auf die Säule aufgetragen; ungefähr 20% der antiviralen Aktivität wurden gebunden. Die Säule wurde mit einem linearen Puffergradienten aus 0,1 M Natriumphosphat, pH 7,5 und 0,1 M Natriumcitrat pH 2,1 eluiert. Zwei Proteinpeaks konnten im Eluat festgestellt werden (Fig.

12): Fraktion A und Fraktion B. Beide Fraktionen wurden auf ihren Gehalt an IFN-α analysiert, wobei ein "zwei-Seiten ELISA" verwendet wurde, bei dem sowohl EBI-10 als auch EBI-1 verwendet wurde. Beide Antikörper zeigen hohe Spezifität für IFN-α2 (Adolf et al. J. Cell Physiol.suppl. 2, 61-68 (1982)). Rekombinantes IFN-α2c wurde als Standard verwendet. Die Fraktion, die bei niedrigem pH eluiert worden war (Fig. 12, Peak "A"), ebenso wie die Probe ergaben Titrationskurven, die parallel zu der Titrationskurve des rekombinanten IFN-α2c verliefen. Der Durchlauf und Fraktionen des 1. Peaks ("B") ergaben Kurven mit verschiedenen Steigungen; sie konnten daher nicht durch den ELISA quantifiziert werden (Fig. 13), sondern wurden im biologischen Assay überprüft (Tabelle III).

Der niedrige, zur Elution des Peak A" erforderliche pH, ebenso wie die Ergebnisse des ELISA deuteten darauf hin, daß IFN-α2 ein Hauptbestandteil des Peak "A" war. Um sicherzustellen, daß sämtliches immunreaktives IFN-α durch den Antikörper gebunden worden war, wurde der Durchlauf erneut über die Säule gegeben und wie oben beschrieben ein zweites Mal eluiert. Das eluierte Material ergab weniger als 10% der IFN-Aktivität, die beim ersten Durchlauf gebunden worden war.

Sowohl die Fraktionen "A" als auch "B" wurden getrennt gesammelt, neutralisiert und erneut einer chromatographischen Reinigung auf derselben Affinitätssäule unterzogen. In beiden Fällen wurde mehr als 95% der IFN-Aktivität gebunden; Elution erfolgte an derselben Gradientenposition wie im ersten Zyklus. Ausgangsprodukt, Durchlauf und die gesammelten Fraktionen beider Chromatographien wurden durch Coomassie Blau Färbungsassays auf ihren Proteingehalt und durch einen antiviralen Bioassay auf ihren IFN-Aktivitätsgehalt hin untersucht. Die Ergebnisse sind in Tabelle III zusammengefaßt:

## Tabelle III

Reinigung des natürlichen IFN-α2

| | Volumen | Protein | antivirale Aktivität | | Ausbeute |
|---|---|---|---|---|---|
| | ml | mg/ml | $\times 10^{-6}$ IU/ml[1] | IU total | % |
| P-IF[2] | 550 | 1,7 | 2,8 | 1540 | 100 |
| 1. Zyklus Durchfluß | 550 | 1,7 | 2,2 | 1216 | 79 |
| 1. Zyklus Eluat A | 18 | 0,08 | 9,6 | 172 | 11 |
| 1. Zyklus Eluat B | 17 | 0,05 | 4,3 | 73 | 4,8 |
| 2. Zyklus Eluat A | 8 | 0,1 | 12 | 96 | 6,2 |
| 2. Zyklus Eluat B | 4 | 0,1 | 13 | 54 | 3,5 |

1 Mittelwert von 5 verschiedenen Bioassays

2 teilweise gereinigtes humanes Leukocyten IFN nach Entfernung des IFN-ω1

Beispiel 8:

Identifizierung des Affinitäts-gereinigten Proteins als IFN-α2

Das Affinitäts-gereinigte IFN-α wurde zunächst durch Reverse Phase HPLC analysiert und gereinigt. Peak "A" zeigte zwei unvollständig aufgelöste Peaks "1" und "2" mit einem Massenverhältnis von etwa 1:2 (Fig. 14 unten); Peak "1" repräsentierte eine mehr hydrophile Proteinfraktion. Beide Peakfraktionen wurden gesammelt, rechromatographiert und einer N-terminalen Aminosäureanalyse unterzogen. Die nachfolgende Sequenz wurde aus beiden Fraktionen erhalten (die Cys-Reste in Klammern wurden nicht identifiziert, sondern auf der Basis der konservierten IFN-Sequenzen abgeleitet):

[$^{1}$CYS]-ASP-LEU-PRO-$^{5}$GLN-THR-HIS-SER-LEU-$^{10}$GLY-SER-

ARG-ARG-THR-$^{15}$LEU-MET-LEU-LEU-ALA-$^{20}$GLN-MET-ARG-

$^{23}$ARG-ILE-$^{25}$SER-LEU-PHE-SER-[CYS]-$^{30}$LEU-

Durch Vergleich mit publizierten Sequenzen wurden beide als IFN-α2 identifiziert.

In beiden Peakfraktionen "1" und "2" wurde die Aminosäure an Position 23 eindeutig als Arginin identifiziert; die als LeIFA bezeichnete Variante, die an Position 23 Lysin aufweist (Goeddel et al., Nature, 290, 20-26 (1981)), war demnach in der verwendeten Leukozytenpräparation in nachweisbaren Mengen nicht vorhanden. Die Aminosäure an Position 34 wurde als Histidin identifiziert; das isolierte IFN-α2 war demnach die Variante IFN-α2b.

Die spezifische antivirale Aktivität des natürlichen IFN-α2 bezogen auf die internationale Referenzpräparation für IFN-α2, Gxa01-901-535, basierend auf einer Bestimmung des Proteingehaltes der Probe durch dessen Absorption bei 214 nm (Adolf et al., Virology 175, 410-417 (1990), wurde zu $1{,}5 \times 10^{8}$ IU/mg bestimmt (Mittelwert aus fünf unabhängigen Bioassays).

Bei einem Vergleich der Retentionszeiten des natürlichen IFN-α2 auf der Reverse Phase HPLC mit der des rekombinanten E. coli IFN-α2c wurde offensichtlich, daß das rekombinante Protein signifikant später eluiert wurde (Fig. 14). Die erhöhte Hydrophilizität des natürlichen Proteins ebenso wie dessen Heterogenität muß daher mit posttranslationalen Modifikationen zusammenhängen.

Reverse Phase HPLC des Elutionspeaks "B" ergab ein kompliziertes Muster von fünf unvollständig aufgelösten Peaks. Sequenzanalysen ergaben, daß sämtliche Peaks IFN-α Spezies darstellten, keiner jedoch IFN-α2 repräsentierte.

Das durch HPLCgereinigte IFN-α2 wurde weiterhin durch SDS-PAGE nach Reduktion mit Dithiothreitol analysiert (Fig. 20). Unter den gewählten Bedingungen zeigte das rekombinante IFN-α2c von E. coli ein scheinbares Molekulargewicht von 17.500 D (Molekulargewicht ausgehend von der Aminosäuresequenz: 19.287 D). HPLC-Peakfraktion "1" gab ein einziges relativ breites Band (scheinbares Molekulargewicht 20.000 D) während Peakfraktion "2" in zwei Hauptkomponenten (20.000 bzw. 19.000 D) und in eine Nebenkomponente (21.000 D) aufgespalten wurde. Diese Molekulargewichtsunterschiede im Vergleich zum rekombinanten Protein aus E. coli, die Größenheterogenität wie auch die erhöhte Hydrophilizität deuten darauf hin, daß das natürliche IFN-α2 glycosyliert ist. Da keine Erkennungsstelle für eine N-Glycosylierung in der IFN-α2 Struktur vorhanden ist, muß O-Glycosylierung vorliegen.

Beipiel 9:

Reaktion von natürlichem IFN-α2 mit Endo- und Exoglycosidasen

Die folgenden Versuche wurden jeweils mit beiden Peaks nach Trennung über RP-HPLC (Peaks 1 und 2 aus Fig. 14b) durchgeführt. Beide Proben wurden mit Neuraminidase und anschließend mit O-Glycanase inkubiert. Nach jeder Enzymreaktion wurde ein Aliquot mittels SDS-PAGE untersucht.

Wie in Fig. 16 zu sehen ist, reagierte Peak 1 weder mit Neuraminidase noch mit O-Glycanase. Die scheinbare molekulare Masse blieb mit 20.000 konstant. Die drei Banden des Peaks 2 hingegen reagierten sowohl mit Neuraminidase als auch anschließend mit O-Glycanase. Die Reaktion mit Neuraminidase bewirkte eine Reduktion der scheinbaren molekularen Masse der beiden schwereren Banden (Mr 21.000 und 20.000) auf 19.000. Spuren der Bande mit der scheinbaren molekularen Masse von 20.000 blieben jedoch zurück. Anschließende Inkubation des Proteins mit O-Glycanase führte zu einer weiteren Reduktion der scheinbaren molekularen Masse von 19.000 auf 17.500 (= scheinbare molekulare Masse von E. coli-IFN-α2c). Die Komponente mit Mr 19.000 wurde dabei vollständig abgebaut. Nach wie vor blieben geringe Mengen der Bande mit der scheinbaren molekularen Masse von 20.000 detektierbar. Da die Trennung der beiden Peaks 1 und 2 aus Fig. 14b mittels RP-HPLC nicht vollständig war, ist der nicht spaltbare Anteil der Bande mit Mr 20.000 wahrscheinlich auf eine Verunreinigung des Peaks 2 mit Peak 1 zurückzuführen.

In einem weiteren Versuch wurde Peak 2 mit O-Glycanase inkubiert, ohne zuvor mit Neuraminidase behandelt worden zu sein (O-Glycanase spaltet das Disaccharid Gal(β1-3)GalNAc nur dann vom Protein ab, wenn dieses durch keine weiteren Verbindungen substituiert ist). Das Reaktionsprodukt wurde wieder mittels SDS-PAGE aufgetrennt (Fig. 17). Man erkennt hier deutlich, daß nur die leichteste Komponente des Peaks 2 eine Reduktion ihrer molekularen Masse erfährt (Reduktion von Mr 19.000 auf Mr 17.500). Die scheinbaren molekularen Massen der beiden schwereren Komponenten (Mr 21.000 und 20.000) blieben unverändert.

Beispiel 10:

Reaktion von natürlichem IFN-α2 mit 0,1 M NaOH

Da O-Glycosylierungen schon unter milden alkalischen Bedingungen abbaubar sind, wurde versucht, die O-Glycanase-resistente Komponente (Peak 1 aus Fig. 14b) mittels Inkubation mit 0,1 M NaOH zu deglycosylieren. Die Reaktion erfolgte wie oben beschrieben. Gleichzeitig wurde als Kontrolle E. coli-IFN-α2c und Peak 2 unter denselben Bedingungen inkubiert. Die Reaktionsprodukte wurden mittels SDS-PAGE analysiert. Wie in Fig. 18 ersichtlich ist, wurden die molekularen Massen aller Komponenten von natürlichem IFN-α2 auf die scheinbare molekulare Masse von E. coli-IFN-α2 reduziert. Die Unschärfe der Proteinbanden ist auf die unter den geschilderten Bedingungen geringfügigen Zerstörungen des Proteins zurückzuführen. Auch die Banden im höhermolekularen Bereich (Mr >30.000) traten als Folge der alkalischen Behandlung auf.

Beispiel 11:

Identifizierung der Glycopeptide mittels Peptide Mapping

Die beiden Peaks von natürlichem IFN-α2 (Fig. 14b) sowie E. coli-IFN-α2c wurden mit Trypsin gespalten, reduziert und über RP-HPLC aufgetrennt. In Fig. 19 sind Ausschnitte der Chromatogramme zu sehen. Zwei Peaks aus dem Peptide Map von E. coli-IFN-α2c fallen dabei wegen ihrer Hydrophobizität (und daher relativ späteren Elution) gegenüber den analogen Peaks aus dem natürlichen IFN-α2 auf: Peak I und Peak II ( im Peptide Map des E. coli-IFN-α2c) wurden deutlich später eluiert als ihre korrespondierenden Peaks I/I und I/II vom Peak 1 aus Fig. 14b bzw. Peaks 2/Ia, 2/Ib, 2/IIa und 2/IIb vom Peak 2 aus Fig. 14b.

N-terminale Sequenzierung der erwähnten Peaks von natürlichem IFN-α2 sowie der beiden E. coli-Peaks ergab für die Peaks I, I/I, 2/Ia, 2/Ib (aus Fig. 19) die Sequenz des Peptides von Aminosäure (AS) 84-112 und für die Peaks II, I/II, 2/IIa, 2/IIb die Sequenz von AS 71-112 (Die Aminosäuresequenz von IFN-α2c ist in Fig. 15 dargestellt). Die unterschiedlichen Retentionszeiten mußten also auf eine Glycosylierung der Peptide aus natürlichem IFN-α2 zurückzuführen sein.

Beispiel 12:

Plasmadesorptions Massenspektrometrie der Glycopeptide von natürlichem IFN-α2

Die Peaks I/II, 2/Ia, 2/IIa und 2/IIb wurden weiterhin mit Hilfe von PD-MS charakterisiert. Die Ergebnisse der Messungen sind in Tabelle IV zusammengefaßt. Die Differenz der aus der Aminosäure-Sequenz errechneten molekularen Massen und den tatsächlich erhaltenen molekularen Massen der einzelnen Peptide lassen sich mit unterschiedlichen Glycanstrukturen erklären: Die molekulare Masse des Peptides I/II, das von der O-Glycanase-resistenten Form des IFN-α2 erhalten wurde, entspricht der molekularen Masse des Peptides (AS 71-112), das mit einem Tetrasaccharid, bestehend aus zwei N-Acetylhexosamineinheiten und zwei Hexoseeinheiten, substituiert ist. In Analogie zu bereits beschriebenen Strukturen solcher O-Glycane dürfte es sich hier um ein Oligosaccharid mit folgender Struktur handeln : Gal1-3(Gal1-4GlcNAc1-6)GalNAc-.

Peptid 2/Ia wies eine molekulare Masse von 3.975 amu auf, was mit der Substitution des Peptides mit dem Trisaccharid NeuAc-Gal-GalNAc erklärbar ist. Dieselbe Glycanstruktur läßt sich aus der molekularen Masse des Peptides 2/IIa (5.448 amu) ableiten. Für Peptid 2/IIb wurde ein Wert von 5.132 amu gemessen, was einer Glycosylierung mit dem Disaccharid Gal-GalNAc entspricht.

Prinzipiell wiesen alle analysierten Peaks eine um ca. 23 amu erhöhte molekulare Masse auf. Das ist durch Anlagerung von $Na^+$-Ionen an das Peptid erklärbar. Diese Verunreinigungen hätten durch intensives Waschen der Targets vor der Messung vermieden werden können, wurden aber im speziellen Fall in Kauf genommen um Verluste der Glycopeptide gering zu halten. Aus den Ergebnissen des Glycosidase-Abbaues (s. oben) und den massenspektrometrischen Messungen können die in Tabelle IV angeführten Glycanstrukturen abgeleitet werden. Die kleinen Peaks, die im Bereich der Glycopeptide im Peptide Map zu sehen sind, können von weiteren Glycosylierungsvarianten stammen.

Tabelle IV

| Peak[1] | Peptid (Aminosäurenummer) | Molekulare Masse (amu)[2] gem. | ber. (aus AS-Sequenz) | Diff. | Vorgeschlagene Glycanstruktur Struktur | Masse[3] |
|---|---|---|---|---|---|---|
| 1/II | 71-112 | 5.485 | 4.736 | 749 | -GalNAc-Gal<br>GlcNAc-Gal | 752 |
| 2/Ia | 84-112 | 3.975 | 3.304 | 671 | -GalNAc-Gal-NeuAc | 678 |
| 2/IIa | 71-112 | 5.448 | 4.736 | 712 | -GalNAc-Gal-NeuAc | 678 |
| 2/IIb | 71-112 | 5.132 | 4.736 | 396 | -GalNAc-Gal | 387 |

Tabelle IV : Molekulare Massen einiger Glycopeptide von natürlichem IFN-α2 mit den entsprechenden vorgeschlagenen Glycanstrukturen. (1) Peaknummern entsprechend Fig. 19; (2) amu, atomare Masseneinheit; (3) berechnete Masse inklusive eines $Na^+$-Ions

Beipiel 13:

Identifizierung der O-glycosylierten Aminosäure mittels Gasphasensequenzierung

Da die durch Spaltung mit Trypsin erhaltenen Glycopeptide zu lang waren, um ihre gesamte Sequenz zu bestimmen, wurden diese Peptide mittels Staphylococcus aureus Protease V8 weiter gespalten und über RP-HPLC aufgetrennt. Mit dem entsprechenden Peptiden aus E. coli-IFN-α2c wurde ebenso verfahren. Nach Vergleich der Peptide Maps wurden alle Peptide mit unterschiedlicher Retentionszeit isoliert und sequenziert. Alle Glykopeptide aus natürlichen IFN-α2 enthielten die Aminosäuren 97-112. Während im E. coli-IFN-α2c-Peptid $^{106}$THR nachgewiesen werden konnte, war es in den Peptiden aus natürlichem IFN-α2 nicht nachweisbar. Damit konnte $^{106}$THR als Glycosylierungsstelle identifiziert werden.

## Patentansprüche

1. Rekombinantes Interferon alpha, dadurch gekennzeichnet, daß es O-glycosyliert ist und im wesentlichen die biologischen und/oder immunologischen Eigenschaften eines IFN-α2 aufweist, vorzugsweise daß es das O-glycosylierte humane IFN-α2a, IFN-α2b oder IFN-α2c ist.

2. Interferon alpha gemäß Anpruch 1, dadurch gekennzeichnet, daß das Threonin-106 (THR-106) O-glycosyliert ist.

3. Interferon alpha gemäß einem der Ansprüche 1 oder 2, dadurch gekennzeichnet, daß das Oligosaccarid bevorzugt das neutrale Disaccharid Gal-GalNAc, dessen mono- oder disialylierte Variante oder das neutrale Tetrasaccharid Gal-(Gal-GlcNAc-)GalNAc ist.

4. Verfahren zur Herstellung eines rekombinanten Interferon alpha gemäß Anspruch 1, dadurch gekennzeichnet, daß
   a) in ein für die Transfektion von Zellen multizellulärer Organismen geeignetes Expressionsplasmid eine für IFN-α kodierende DNA eingefügt wird,
   b) daß mit dem so erhaltenen Expressionsplasmid Zellen multizellulärer Organismen, vorzugsweise Wirbeltierzellen transfiziert werden,
   c) daß die transfizierten Organismen in einem geeigneten Medium kultiviert werden,
   d) daß der Zellüberstand geerntet,
   e) daß das O-glycosylierte IFN-α isoliert und in an sich bekannter Weise gereinigt wird.

5. Verfahren gemäß Anpruch 4, dadurch gekennzeichnet, daß das unter a) eingesetzte Expressionsplasmid das pAD-CMV13, 15 oder 19, gemäß Figuren 3 und 4, vorzugsweise das pAD-CMV19, ist, und daß die unter a) einzufügende DNA für ein Protein kodiert, das im wesentlichen die biologischen und/oder immunologischen Eigenschaften eines IFN-α2 aufweist, vorzugsweise für ein humanes IFN-α2a, IFN-α2b oder IFN-α2c, insbesondere für humanes IFN-α2c kodiert.

6. Verfahren gemäß einem der Ansprüche 4 oder 5, dadurch. gekennzeichnet, daß das Expressionsplasmid pAD19B-IFN gemäß Figur 5 eingesetzt wird.

7. Verfahren gemäß einem der Ansprüche 4, 5 oder 6, dadurch gekennzeichnet, daß als Zellen multizellulärer Organismen CHO-Zellen verwendet werden.

8. Expressionsplasmid zur Transfektion multizellulärer Organismen, dadurch gekennzeichnet, daß es pAD-CMV13, pAD-CMV15 oder pAD-CMV19 gemäß Figuren 3 und 4 ist.

9. O-glycosyliertes rekombinantes Interferon alpha, herstellbar nach einem der Ansprüche 4 bis 7.

10. Rekombinantes Interferon alpha gemäß einem der Ansprüche 1 bis 3 oder 9 zur Verwendung als Arzneimittel.

11. Mittel zur Behandlung viraler oder tumoraler Erkrankungen, ein rekombinantes Interferon alpha gemäß einem der Ansprüche 1 bis 3 oder 9 enthaltend.

12. Mittel gemäß Anspruch 11, dadurch gekennzeichnet, daß es aus einer Mischung aus mindestens zwei der rekombinanten O-glycosylierten Proteine IFN-α2a, IFN-α2b oder IFN-α2c besteht.

## Claims

1. Recombinant interferon alpha, characterised in that it is O-glycosylated and essentially has the biological and/or immunological properties of an IFN-α2, preferably that it is the O-glycosylated human IFN-α2a, IFN-α2b or IFN-α2c.

2. Interferon alpha according to claim 1, characterised in that the threonine-106 (THR-106) is 0-glycosylated.

3. Interferon alpha according to claims 1 or 2, characterised in that the oligosaccharide is preferably the neutral disaccharide Gal-GalNAc, the mono- or disialylated variant thereof or the neutral tetrasaccharide Gal-(Gal-GlcNAc-)GalNAc.

**4.** Process for preparing a recombinant interferon alpha according to claim 1, characterised in that
a) a DNA coding for IFN-α is introduced into an expression plasmid which is suitable for the transfection of cells of multicellular organisms,
b) in that cells of multicellular organisms, preferably vertebrate cells, are transfected with the expression plasmid thus obtained,
c) in that the transfected organisms are cultivated in a suitable medium,
d) in that the cell supernatant is harvested,
e) in that the O-glycosylated IFN-α is isolated and purified in a manner known per se.

**5.** Process according to claim 4, characterised in that the expression plasmid used in a) is pAD-CMV13, 15 or 19, according to Figures 3 and 4, preferably pAD-CMV19, and in that the DNA to be inserted under a) codes for a protein which essentially has the biological and/or immunological properties of an IFN-α2, preferably codes for a human IFN-α2a, IFN-α2b or IFN-α2c, more particularly for human IFN-α2c.

**6.** Process according to one of claims 4 or 5, characterised in that the expression plasmid used is pAD19B-IFN according to Figure 5.

**7.** Process according to one of claims 4, 5 or 6, characterised in that CHO cells are used as the cells of multicellular organisms.

**8.** Expression plasmid for transfection of multicellular organisms, characterised in that it is pAD-CMV13, pAD-CMV15 or pAD-CMV19 according to Figures 3 and 4.

**9.** O-glycosylated recombinant interferon alpha, capable of being prepared according to one of claims 4 to 7.

**10.** Recombinant interferon alpha according to one of claims 1 to 3 or 9 for use as a pharmaceutical composition.

**11.** Agent for treating viral or tumoural diseases, containing a recombinant interferon alpha according to one of claims 1 to 3 or 9.

**12.** Agent according to claim 11, characterised in that it consists of a mixture of at least two of the recombinant O-glycosylated proteins IFN-α2a, IFN-α2b or IFN-α2c.

## Revendications

**1.** Interféron alpha recombinant, caractérisé en ce qu'il est O-glycosylé et présente sensiblement les propriétés biologiques et/ou immunologiques d'un IFN α2, de préférence en ce qu'il s'agit d'IFN α2a, d'IFN α2b ou d'IFN α2c O-glycosylé humain.

**2.** Interféron alpha selon la revendication 1, caractérisé en ce que la thréonine 106 (THR-106) est O-glycosylée.

**3.** Interféron alpha selon l'une des revendications 1 ou 2, caractérisé en ce que l'oligosaccharide est de préférence le disaccharide neutre Gal-GalNac, son variant mono- ou disialylé ou le tétrasaccharide neutre Gal-(Gal-GlcNac-)GalNac.

**4.** Procédé de préparation d'un interféron alpha recombinant selon la revendication 1, caractérisé en ce que
a) un ADN codant IFN α est inséré dans un plasmide d'expression convenant pour la transfection de cellules d'organismes multicellulaires,
b) des cellules d'organismes multicellulaires, de préférence des cellules de vertébrés, sont transfectées avec le plasmide d'expression ainsi obtenu,
c) les organismes transfectés sont cultivés dans un milieu approprié,
d) le surnageant des cellules est récolté,
e) l'IFN α O-glycosylé est isolé et purifié de manière connue en soi.

5. Procédé selon la revendication 4, caractérisé en ce que le plasmide d'expression utilisé en a) est le plasmide pAD-CMV13, 15 ou 19, selon les figures 3 et 4, de préférence le plasmide pAD-CMV19, et en ce que l'ADN inséré en a) code une protéine qui présente sensiblement les propriétés biologiques et/ou immunologiques d'un IFN α2, de préférence code un IFN α2a, un IFN α2b ou un IFN α2c humain, en particulier l'IFN α2c humain.

6. Procédé selon l'une des revendications 4 ou 5, caractérisé en ce que l'on utilise le plasmide d'expression pAD19B-IFN selon la figure 5.

7. Procédé selon l'une des revendications 4, 5 ou 6, caractérisé en ce que l'on utilise comme cellules d'organismes multicellulaires des cellules CHO.

8. Plasmide d'expression pour la transfection d'organismes multicellulaires, caractérisé en ce qu'il s'agit du plasmide pAD-CMV13, pAD-CMV15 ou pAD-CMV19 selon les figures 3 et 4.

9. Interféron alpha recombinant O-glycosylé qui peut être préparé selon l'une des revendications 4 à 7.

10. Interféron alpha recombinant selon l'une des revendications 1 à 3 ou 9 destiné à être utilisé comme médicament.

11. Agent pour le traitement des maladies tumorales ou virales, contenant un interféron alpha recombinant selon l'une des revendications 1 à 3 ou 9.

12. Agent selon la revendication 11, caractérisé en ce qu'il consiste en un mélange d'au moins deux des protéines O-glycosylées recombinantes IFN α2a, IFN α2b ou IFN α2c.

FIG.1

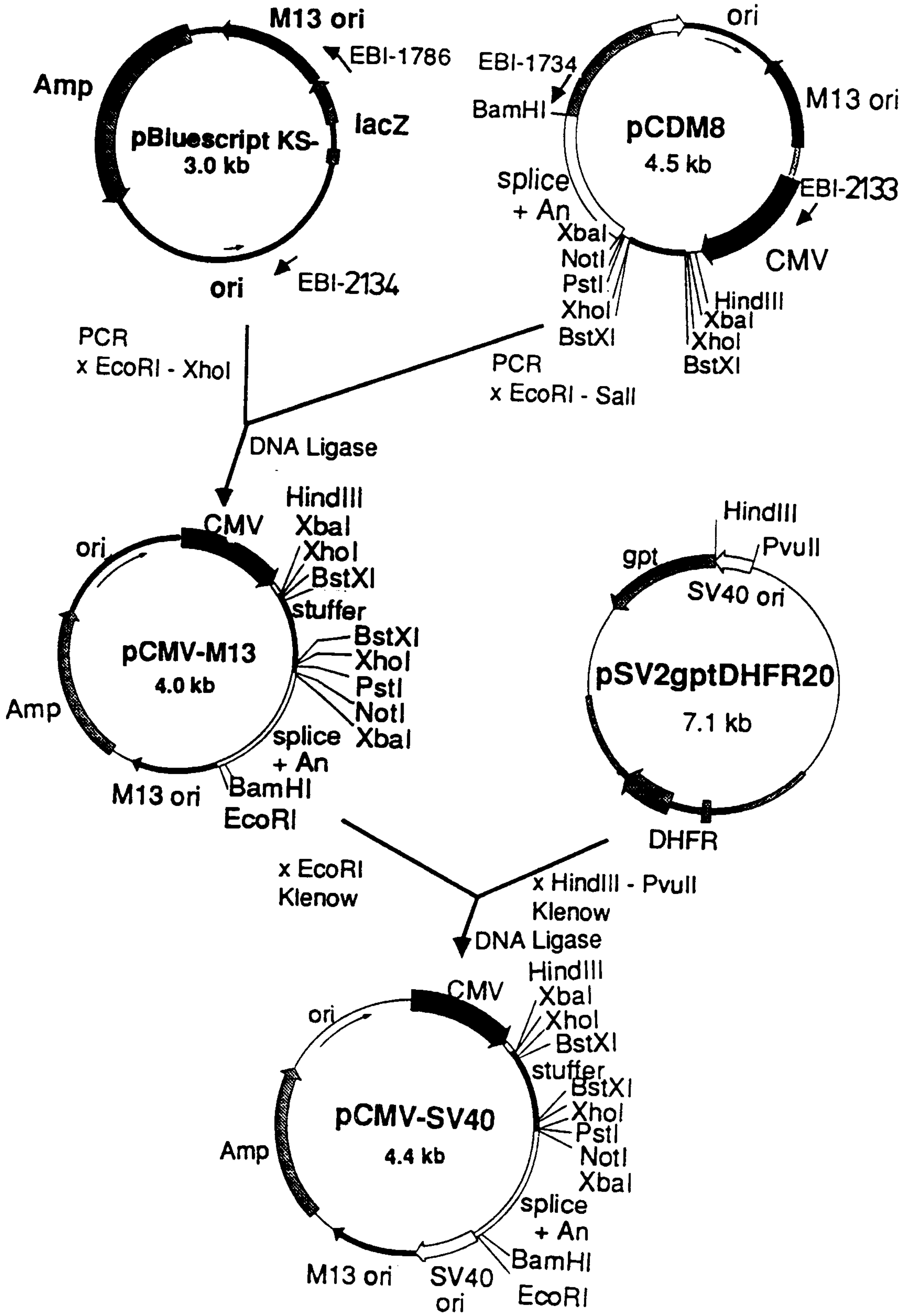
M13 ori
EBI-1786
Amp
pBluescript KS-
3.0 kb
lacZ
ori
EBI-2134
ori
EBI-1734
BamHI
pCDM8
4.5 kb
M13 ori
splice
+ An
EBI-2133
CMV
XbaI
NotI
PstI
XhoI
BstXI
HindIII
XbaI
XhoI
BstXI
PCR
x EcoRI - XhoI
PCR
x EcoRI - SalI
DNA Ligase
CMV
HindIII
XbaI
XhoI
BstXI
stuffer
ori
pCMV-M13
4.0 kb
Amp
BstXI
XhoI
PstI
NotI
XbaI
splice
+ An
M13 ori
BamHI
EcoRI
HindIII
PvuII
gpt
SV40 ori
pSV2gptDHFR20
7.1 kb
DHFR
x EcoRI
Klenow
x HindIII - PvuII
Klenow
DNA Ligase
CMV
HindIII
XbaI
XhoI
BstXI
stuffer
ori
pCMV-SV40
4.4 kb
Amp
BstXI
XhoI
PstI
NotI
XbaI
splice
+ An
BamHI
EcoRI
M13 ori
SV40
ori

FIG. 2

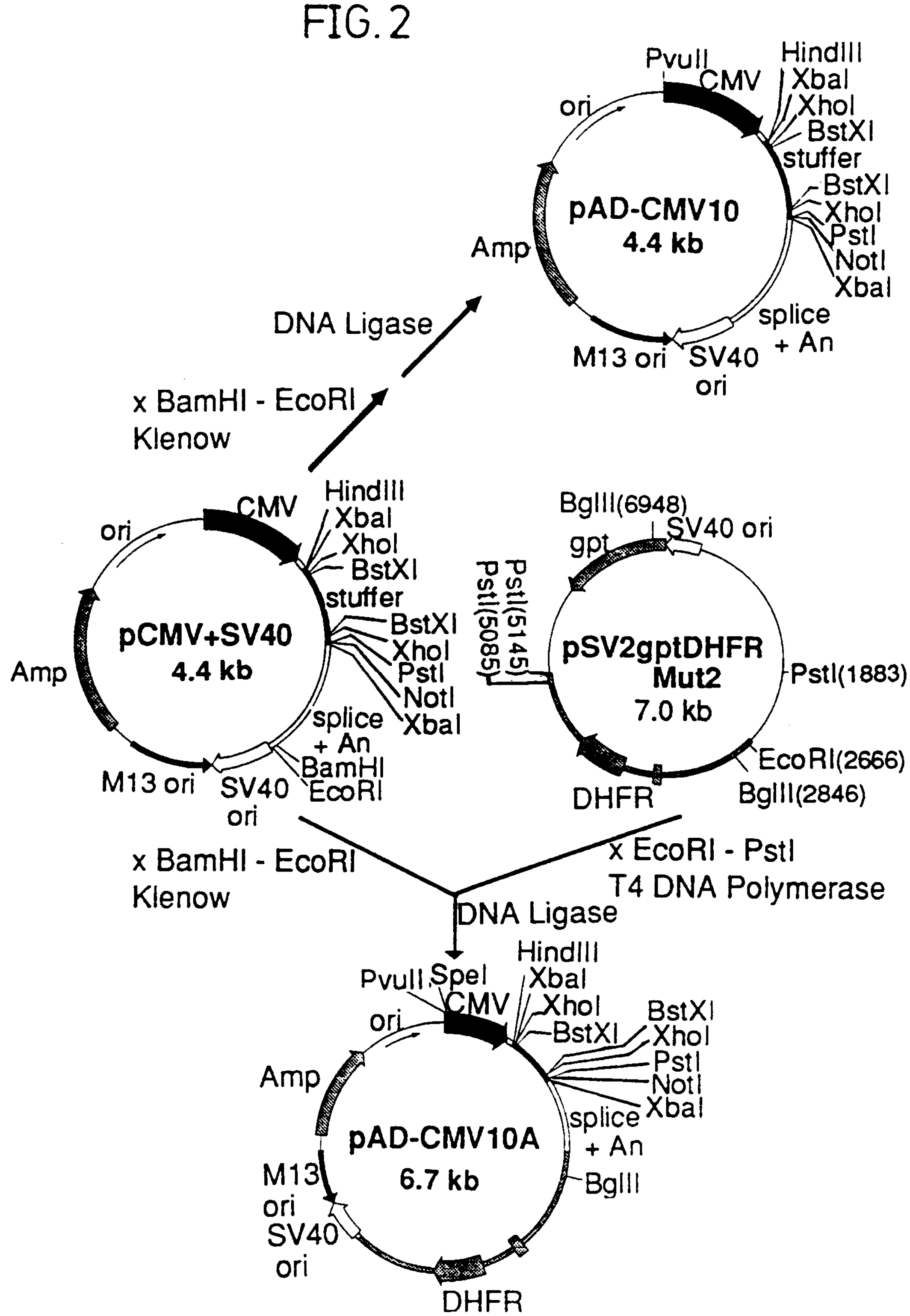
pAD-CMV10
4.4 kb
PvuII
CMV
HindIII
XbaI
XhoI
BstXI
stuffer
BstXI
XhoI
PstI
NotI
XbaI
splice + An
SV40 ori
M13 ori
Amp
ori
DNA Ligase
x BamHI - EcoRI
Klenow
pCMV+SV40
4.4 kb
CMV
HindIII
XbaI
XhoI
BstXI
stuffer
BstXI
XhoI
PstI
NotI
XbaI
splice + An
BamHI
EcoRI
SV40 ori
M13 ori
Amp
ori
pSV2gptDHFR Mut2
7.0 kb
BglII(6948)
SV40 ori
gpt
PstI(5145)
PstI(5085)
PstI(1883)
EcoRI(2666)
BglII(2846)
DHFR
x BamHI - EcoRI
Klenow
x EcoRI - PstI
T4 DNA Polymerase
DNA Ligase
pAD-CMV10A
6.7 kb
PvuII
SpeI
CMV
HindIII
XbaI
XhoI
BstXI
BstXI
XhoI
PstI
NotI
XbaI
splice + An
BglII
DHFR
SV40 ori
M13 ori
Amp
ori

FIG. 3

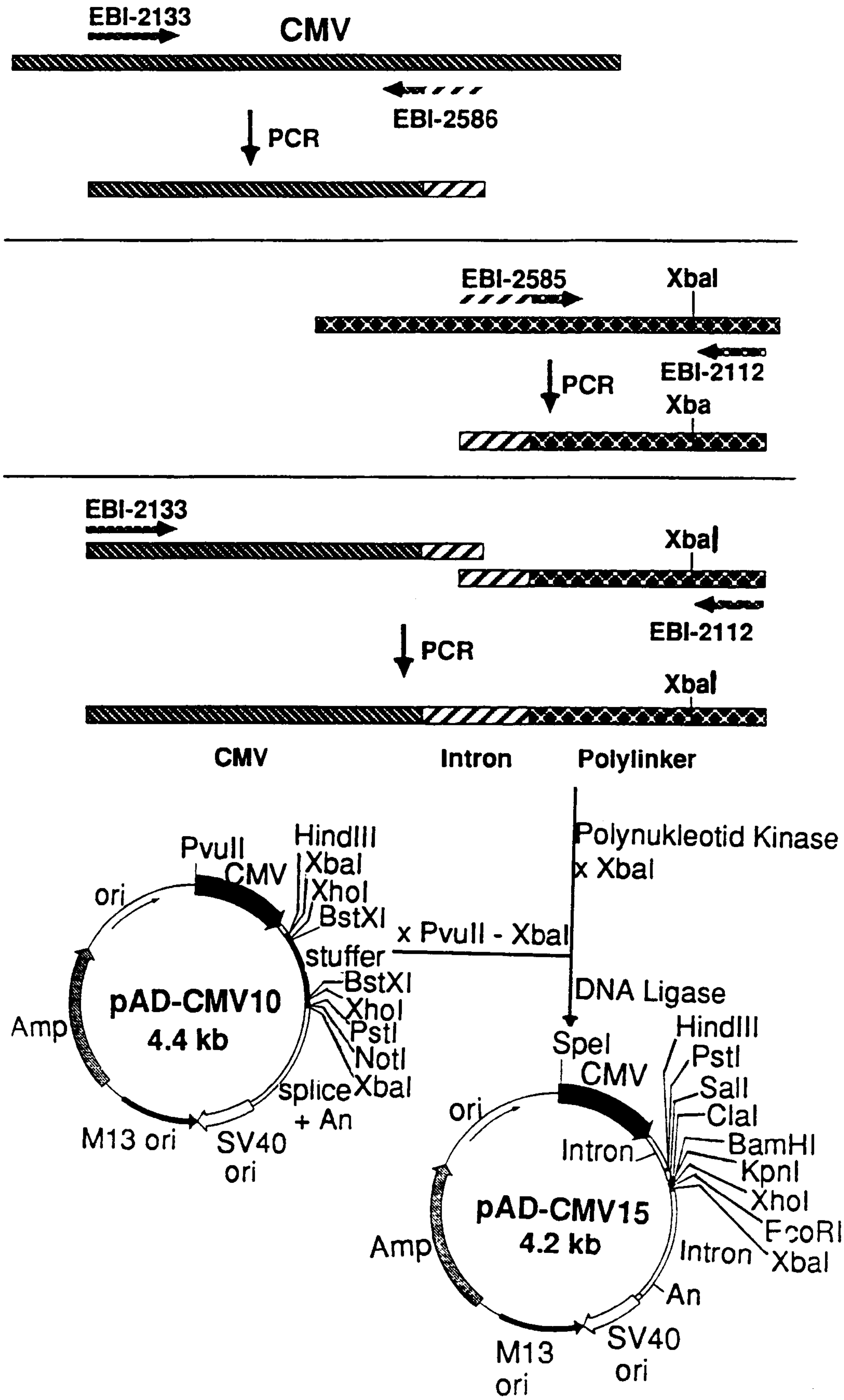
EBI-2133
CMV
EBI-2586
PCR
EBI-2585
XbaI
EBI-2112
PCR
Xba
EBI-2133
XbaI
EBI-2112
PCR
XbaI
CMV
Intron
Polylinker
Polynukleotid Kinase
x XbaI
x PvuII - XbaI
DNA Ligase
PvuII
HindIII
CMV
XbaI
XhoI
BstXI
ori
stuffer
BstXI
XhoI
PstI
NotI
XbaI
splice
+ An
Amp
pAD-CMV10
4.4 kb
M13 ori
SV40
ori
SpeI
CMV
HindIII
PstI
SalI
ClaI
BamHI
KpnI
XhoI
EcoRI
XbaI
ori
Intron
Intron
An
Amp
pAD-CMV15
4.2 kb
M13
ori
SV40
ori

FIG. 4

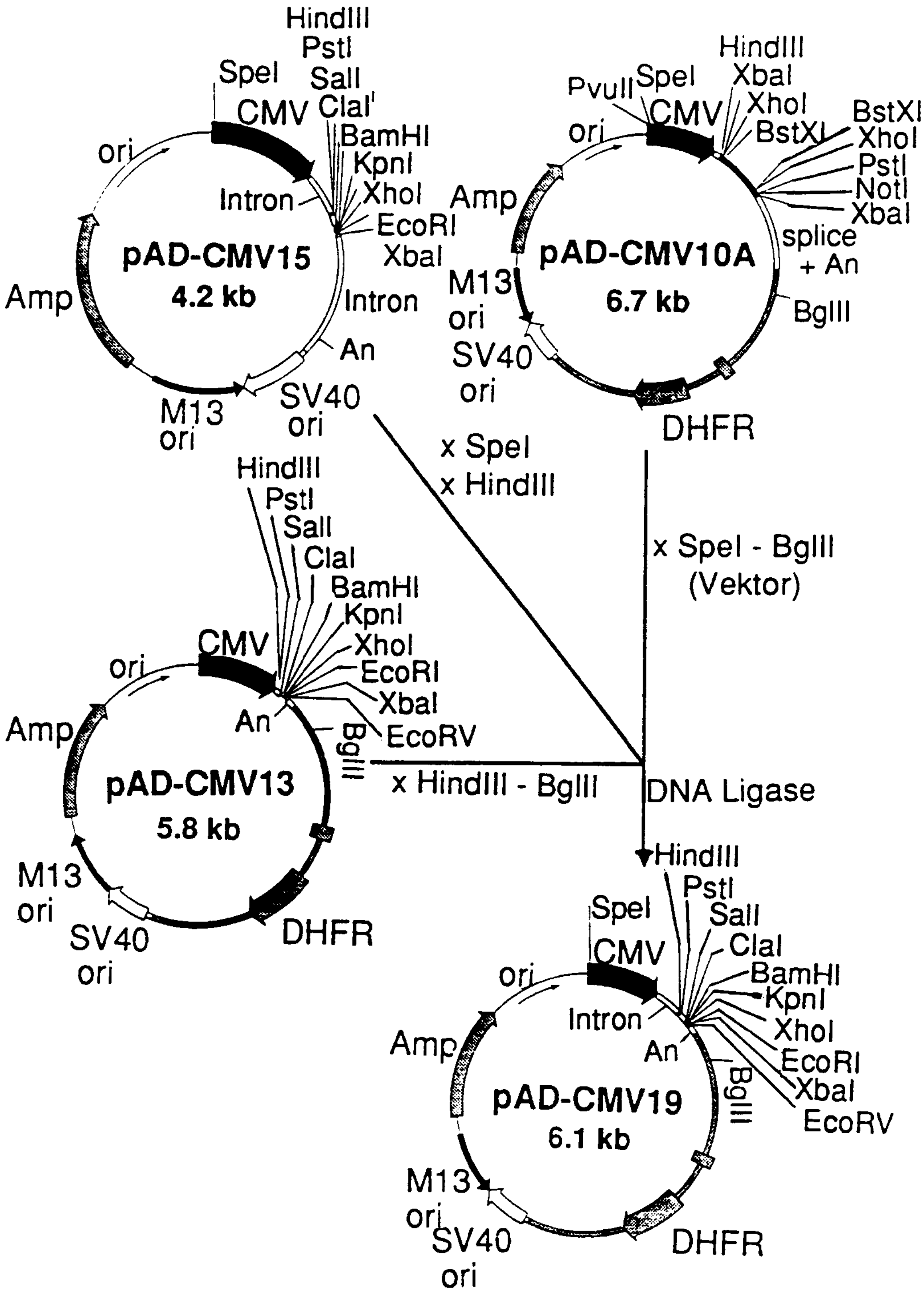
HindIII
PstI
SalI
ClaI'
SpeI
CMV
BamHI
KpnI
XhoI
EcoRI
XbaI
ori
Intron
pAD-CMV15
4.2 kb
Amp
Intron
An
M13
ori
SV40
ori
PvuII
SpeI
CMV
HindIII
XbaI
XhoI
BstXI
BstXI
XhoI
PstI
NotI
XbaI
ori
Amp
pAD-CMV10A
6.7 kb
splice
+ An
M13
ori
BglII
SV40
ori
DHFR
x SpeI
x HindIII
x SpeI - BglII
(Vektor)
HindIII
PstI
SalI
ClaI
BamHI
KpnI
XhoI
EcoRI
XbaI
EcoRV
CMV
ori
Amp
An
BglII
pAD-CMV13
5.8 kb
x HindIII - BglII
DNA Ligase
M13
ori
SV40
ori
DHFR
HindIII
PstI
SalI
ClaI
BamHI
KpnI
XhoI
EcoRI
XbaI
EcoRV
SpeI
CMV
ori
Intron
An
Amp
BglII
pAD-CMV19
6.1 kb
M13
ori
SV40
ori
DHFR

FIG.5

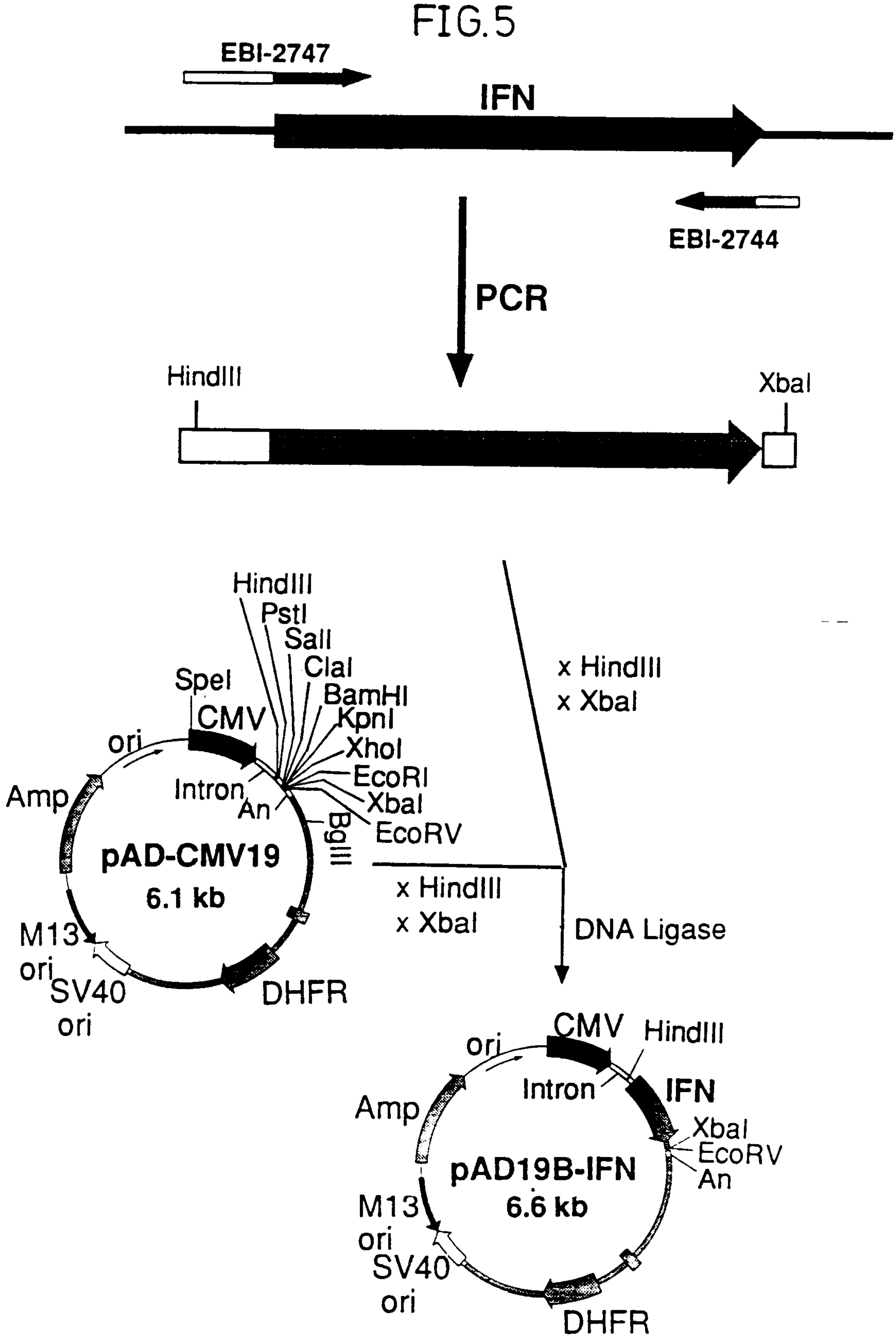
EBI-2747
IFN
EBI-2744
PCR
HindIII
XbaI
HindIII
PstI
SalI
ClaI
BamHI
KpnI
XhoI
EcoRI
XbaI
EcoRV
BglII
SpeI
CMV
ori
Intron
An
Amp
pAD-CMV19
6.1 kb
M13 ori
SV40 ori
DHFR
x HindIII
x XbaI
x HindIII
x XbaI
DNA Ligase
CMV
HindIII
ori
Intron
IFN
Amp
XbaI
EcoRV
An
pAD19B-IFN
6.6 kb
M13 ori
SV40 ori
DHFR

FIG.6A

HindIII/XbaI - Insert von pAD19B-IFN

```
        AAGCTTAC ATT TGC TTC TGA CAC AAC TGT GTT CAC TAG CAA CCT CAA ACA
                  9           18          27          36          45

        -23
        Met Ala Leu Thr Phe Ala Leu Leu Val Ala Leu Leu Val Leu Ser Cys
GAC ACC ATG GCC TTG ACC TTT GCT TTA TTG GTG GCC CTC CTG GTG CTC AGC TGC
    54          63          72          81          90          99

                        -1   1
Lys Ser Ser Cys Ser Val Gly Cys Asp Leu Pro Gln Thr His Ser Leu Gly Ser
AAG TCA AGC TGC TCT GTG GGC TGT GAT CTG CCT CAA ACC CAC AGC CTG GGT AGC
    108         117         126         135         144         153

Arg Arg Thr Leu Met Leu Leu Ala Gln Met Arg Arg Ile Ser Leu Phe Ser Cys
AGG AGG ACC TTG ATG CTC CTG GCA CAG ATG AGG AGA ATC TCT CTT TTC TCC TGC
    162         171         180         189         198         207

Leu Lys Asp Arg Arg Asp Phe Gly Phe Pro Gln Glu Glu Phe Gly Asn Gln Phe
TTG AAG GAC AGA CGT GAC TTT GGA TTT CCC CAG GAG GAG TTT GGC AAC CAG TTC
    216         225         234         243         252         261

Gln Lys Ala Glu Thr Ile Pro Val Leu His Glu Met Ile Gln Gln Ile Phe Asn
CAA AAG GCT GAA ACC ATC CCT GTC CTC CAT GAG ATG ATC CAG CAG ATC TTC AAT
    270         279         288         297         306         315

Leu Phe Ser Thr Lys Asp Ser Ser Ala Ala Trp Asp Glu Thr Leu Leu Asp Lys
CTC TTC AGC ACA AAG GAC TCA TCT GCT GCT TGG GAT GAG ACC CTC CTA GAC AAA
    324         333         342         351         360         369
```

```
Phe Tyr Thr Glu Leu Tyr Gln Gln Leu Asn Asp Leu Glu Ala Cys Val Ile Gln
TTC TAC ACT GAA CTC TAC CAG CAG CTG AAT GAC CTG GAA GCC TGT GTG ATA CAG
  378         387         396         405         414         423

Gly Val Gly Val Thr Glu Thr Pro Leu Met Lys Glu Asp Ser Ile Leu Ala Val
GGG GTG GGG GTG ACA GAG ACT CCC CTG ATG AAG GAG GAC TCC ATT CTG GCT GTG
  432         441         450         459         468         477

Arg Lys Tyr Phe Gln Arg Ile Thr Leu Tyr Leu Lys Glu Lys Lys Tyr Ser Pro
AGG AAA TAC TTC CAA AGA ATC ACT CTC TAT CTG AAA GAG AAG AAA TAC AGC CCT
  486         495         504         513         522         531

Cys Ala Trp Glu Val Val Arg Ala Glu Ile Met Arg Ser Phe Ser Leu Ser Thr
TGT GCC TGG GAG GTT GTC AGA GCA GAA ATC ATG AGA TCT TTT TCT TTG TCA ACA
  540         549         558         567         576         585

Asn Leu Gln Glu Ser Leu Arg Ser Lys Glu TER
AAC TTG CAA GAA AGT TTA AGA AGT AAG GAA TGA AAACTGGTTCTCTAGA
  594         603         612         621        630      639
```

FIG.6B

FIG.7A

pAD-CMV19

```
TCGACATTGA TTATTGACTA GTTATTAATA GTAATCAATT ACGGGGTCAT TAGTTCATAG   60
CCCATATATG GAGTTCCGCG TTACATAACT TACGGTAAAT GGCCCGCCTG GCTGACCGCC  120
CAACGACCCC CGCCCATTGA CGTCAATAAT GACGTATGTT CCCATAGTAA CGCCAATAGG  180
GACTTTCCAT TGACGTCAAT GGGTGGAGTA TTTACGGTAA ACTGCCCACT TGGCAGTACA  240
TCAAGTGTAT CATATGCCAA GTACGCCCCC TATTGACGTC AATGACGGTA AATGGCCCGC  300
CTGGCATTAT GCCCAGTACA TGACCTTATG GGACTTTCCT ACTTGGCAGT ACATCTACGT  360
ATTAGTCATC GCTATTACCA TGGTGATGCG GTTTTGGCAG TACATCAATG GGCGTGGATA  420
GCGGTTTGAC TCACGGGGAT TTCCAAGTCT CCACCCCATT GACGTCAATG GGAGTTTGTT  480
TTGGCACCAA AATCAACGGG ACTTTCCAAA ATGTCGTAAC AACTCCGCCC CATTGACGCA  540
AATGGGCGGT AGGCGTGTAC GGTGGGAGGT CTATATAAGC AGAGCTCGTT TAGTGAACCG  600
TCAGATCGCC TGGAGACGCC ATCCACGCTG TTTTGACCTC CATAGAAGAC ACCGGGACCG  660
ATCCAGCCTC CGCGGCCGGG AACGGTGCAT TGGAACGCGG ATTCCCCGTG CCAAGAGTCA  720
GGTAAGTACC GCCTATAGAG AAGACTCTTG GGTTTCTGAT AGGCACTGAC TCTCTCTGCC  780
TATTGGTCTA TTTTCCCACC CTTAGGCTGC TGGTGCTTAA CTGGCTTATC GAAATTAATA  840
CGACTCACTA TAGGGAGACC CAAGCTTCTG CAGGTCGACA TCGATGGATC CGGTACCTCG  900
AGCGCGAATT CTCTAGAGAT ATCTTGTTTA TTGCAGCTTA TAATGGTTAC AAATAAAGCA  960
ATAGCATCAC AAATTTCACA AATAAAGCAT TTTTTTCACT GCATTCTAGT TGTGGTTTGT 1020
CCAAACTCAT CAATGTATCT TATCATGTCT GGATCAATTC TGAAAAACTA GCCTTAAAGA 1080
CAGACAGCTT TGTTCTAGTC AGCCAGGCAA GCATATGTAA ATAAAGTTCC TCAGGGAACT 1140
GAGGTTAAAA GATGTATCCT GGACCTGCCA GACCTGGCCA TTCACGTAAA CAGAAGATTC 1200
CGCCTCAAGT TCCGGTTAAC AACAGGAGGC AACGAGATCT CAAATCTATT ACTTCTAATC 1260
GGGTAATTAA AACCTTTCAA CTAAAACACG GACCCACGGA TGTCACCCAC TTTTCCTTCC 1320
CCGGCTCCGC CCTTCTCAGT ACTCCCCACC ATTAGGCTCG CTACTCCACC TCCACTTCCG 1380
GGCGCGACAC CCACGTGCCC TCTCCCACCC GACGCTAACC CCGCCCCTGC CCGTCTGACC 1440
```

FIG.7B

```
CCGCCCACCA CCTGGCCCCG CCCCGTTGAG GACAGAAGAA ACCCCGGGCA GCCGCAGCCA   1500
AGGCGGACGG GTAGACGCTG GGGGCGCTGA GGAGTCGTCC TCTACCTTCT CTGCTGGCTC   1560
GGTGGGGGAC GCGGTGGATC TCAGGCTTCC GGAAGACTGG AAGAACCGGC TCAGAACCGC   1620
TTGTCTCCGC GGGGCTTGGG CGGCGGAAGA ATGGCCGCTA GACGCGGACT TGGTGCGAGG   1680
CATCGCAGGA TGCAGAAGAG CAAGCCCGCC GGGAGCGCGC GGCTGTACTA CCCCGCGCCT   1740
GGAGCGGCCA CGCCGGACTG GGCGGGGCCG GCCTGGTGGA GGCGGAGTCT GACCTCGTGG   1800
AGGCGGGGCC TCTGATGTTC AAATAGGATG CTAGGCTTGT TGAGGCGTGG CCTCCGATTC   1860
ACAAGTGGGA AGCAGCGCCG GGCGACTGCA ATTTCGCGCC AAACTTGGGG GAAGCACAGC   1920
GTACAGGCTG CCTAGGTGAT CGCTGCTGCT GTCATGGTTC GACCGCTGAA CTGCATCGTC   1980
GCCGTGTCCC AGAATATGGG CATCGGCAAG AACGGAGACC TTCCCTGGCC AATGCTCAGG   2040
TACTGGCTGG ATTGGGTTAG GGAAACCGAG GCGGTTCGCT GAATCGGGTC GAGCACTTGG   2100
CGGAGACGCG CGGGCCAACT ACTTAGGGAC AGTCATGAGG GGTAGGCCCG CCGGCTGCTG   2160
CCCTTGCCCA TGCCCGCGGT GATCCCCATG CTGTGCCAGC CTTTGCCCAG AGGCGCTCTA   2220
GCTGGGAGCA AAGTCCGGTC ACTGGGCAGC ACCACCCCCC GGACTTGCAT GGGTAGCCGC   2280
TGAGATGGAG CCTGAGCACA CGTGACAGGG TCCCTGTTAA CGCAGTGTTT CTCTAACTTT   2340
CAGGAACGAG TTCAAGTACT TCCAAAGAAT GACCACCACC TCCTCAGTGG AAGGTAAACA   2400
GAACCTGGTG ATTATGGGCC GGAAAACCTG GTTCTCCATT CCTGAGAAGA ATCGACCTTT   2460
AAAGGACAGA ATTAATATAG TTCTCAGTAG AGAGCTCAAG GAACCACCAC AAGGAGCTCA   2520
TTTTCTTGCC AAAAGTCTGG ACCATGCCTT AAAACTTATT GAACAACCAG AGTTAGCAGA   2580
TAAAGTGGAC ATGGTTTGGA TAGTTGGAGG CAGTTCCGTT TACAAGGAAG CCATGAATCA   2640
GCCAGGCCAT CTCAGACTCT TTGTGACAAG GATCATGCAG GAATTTGAAA GTGACACGTT   2700
CTTCCCAGAA ATTGATTTGG AGAAATATAA ACTTCTCCCA GAGTACCCAG GGGTCCTTTC   2760
TGAAGTCCAG GAGGAAAAAG GCATCAAGTA TAAATTTGAA GTCTATGAGA AGAAAGGCTA   2820
ACAGAAAGAT ACTTGCTGAT TGACTTCAAG TTCTACTGCT TTCCTCCTAA AATTATGCAT   2880
TTTTACAAGA CCATGGGACT TGTGTTGGCT TTAGATCCTG TGCATCCTGG GCAACTGTTG   2940
TACTCTAAGC CACTCCCCAA AGTCATGCCC CAGCCCCTGT ATAATTCTAA ACAATTAGAA   3000
```

FIG.7C

```
TTATTTTCAT TTTCATTAGT CTAACCAGGT TATATTAAAT ATACTTTAAG AAACACCATT 3060
TGCCATAAAG TTCTCAATGC CCCTCCCATG CAGCCTCAAG TGGCTCCCCA GCAGATGCAT 3120
AGGGTAGTGT GTGTACAAGA GACCCCAAAG ACATAGAGCC CCTGAGAGCA TGAGCTGATA 3180
TGGGGGCTCA TAGAGATAGG AGCTAGATGA ATAAGTACAA AGGGCAGAAA TGGGTTTTAA 3240
CCAGCAGAGC TAGAACTCAG ACTTTAAAGA AAATTAGATC AAAGTAGAGA CTGAATTATT 3300
CTGCACATCA GACTCTGAGC AGAGTTCTGT TCACTCAGAC AGAAAATGGG TAAATTGAGA 3360
GCTGGCTCCA TTGTGCTCCT TAGAGATGGG AGCAGGTGGA GGATTATATA AGGTCTGGAA 3420
CATTTAACTT CTCCGTTTCT CATCTTCAGT GAGATTCCAA GGGATACTAC AATTCTGTGG 3480
AATGTGTGTC AGTTAGGGTG TGGAAAGTCC CCAGGCTCCC CAGCAGGCAG AAGTATGCAA 3540
AGCATGCATC TCAATTAGTC AGCAACCAGG TGTGGAAAGT CCCCAGGCTC CCCAGCAGGC 3600
AGAAGTATGC AAAGCATGCA TCTCAATTAG TCAGCAACCA TAGTCCCGCC CCTAACTCCG 3660
CCCATCCCGC CCCTAACTCC GCCCAGTTCC GCCCATTCTC CGCCCCATGG CTGACTAATT 3720
TTTTTTATTT ATGCAGAGGC CGAGGCGCCT CTGAGCTATT CCAGAAGTAG TGAGGAGGCT 3780
TTTTTGGAGG CCTAGGCTTT TGCAAAAAAG CTAATTCAGC CTGAATGGCG AATGGGAAAT 3840
TGTAAACGTT AATATTTTGT TAAAATTCGC GTTAAATTTT TGTTAAATCA GCTCATTTTT 3900
TAACCAATAG GCCGAAATCG GCAAAATCCC TTATAAATCA AAAGAATAGA CCGAGATAGG 3960
GTTGAGTGTT GTTCCAGTTT GGAACAAGAG TCCACTATTA AAGAACGTGG ACTCCAACGT 4020
CAAAGGGCGA AAAACCGTCT ATCAGGGCGA TGGCCCACTA CGTGAACCAT CACCCTAATC 4080
AAGTTTTTGG GGTCGAGGTG CCGTAAAGCA CTAAATCGGA ACCCTAAAGG GAGCCCCCGA 4140
TTTAGAGCTT GACGGGGAAA GCCGGCGAAC GTGGCGAGAA AGGAAGGGAA GAAAGCGAAA 4200
GGAGCGGGCG CTAGGGCGCT GGCAAGTGTA GCGGTCACGC TGCGCGTAAC CACCACACCC 4260
GCCGCGCTTA ATGCGCCGCT ACAGGGCGCG TCAGGTGGCA CTTTTCGGGG AAATGTGCGC 4320
GGAACCCCTA TTTGTTTATT TTTCTAAATA CATTCAAATA TGTATCCGCT CATGAGACAA 4380
TAACCCTGAT AAATGCTTCA ATAATATTGA AAAAGGAAGA GTATGAGTAT TCAACATTTC 4440
CGTGTCGCCC TTATTCCCTT TTTTGCGGCA TTTTGCCTTC CTGTTTTTGC TCACCCAGAA 4500
ACGCTGGTGA AAGTAAAAGA TGCTGAAGAT CAGTTGGGTG CACGAGTGGG TTACATCGAA 4560
```

FIG.7D

```
CTGGATCTCA ACAGCGGTAA GATCCTTGAG AGTTTTCGCC CCGAAGAACG TTTTCCAATG 4620
ATGAGCACTT TTAAAGTTCT GCTATGTGGC GCGGTATTAT CCCGTATTGA CGCCGGGCAA 4680
GAGCAACTCG GTCGCCGCAT ACACTATTCT CAGAATGACT TGGTTGAGTA CTCACCAGTC 4740
ACAGAAAAGC ATCTTACGGA TGGCATGACA GTAAGAGAAT TATGCAGTGC TGCCATAACC 4800
ATGAGTGATA ACACTGCGGC CAACTTACTT CTGACAACGA TCGGAGGACC GAAGGAGCTA 4860
ACCGCTTTTT TGCACAACAT GGGGGATCAT GTAACTCGCC TTGATCGTTG GGAACCGGAG 4920
CTGAATGAAG CCATACCAAA CGACGAGCGT GACACCACGA TGCCTGTAGC AATGGCAACA 4980
ACGTTGCGCA AACTATTAAC TGGCGAACTA CTTACTCTAG CTTCCCGGCA ACAATTAATA 5040
GACTGGATGG AGGCGGATAA AGTTGCAGGA CCACTTCTGC GCTCGGCCCT TCCGGCTGGC 5100
TGGTTTATTG CTGATAAATC TGGAGCCGGT GAGCGTGGGT CTCGCGGTAT CATTGCAGCA 5160
CTGGGGCCAG ATGGTAAGCC CTCCCGTATC GTAGTTATCT ACACGACGGG GAGTCAGGCA 5220
ACTATGGATG AACGAAATAG ACAGATCGCT GAGATAGGTG CCTCACTGAT TAAGCATTGG 5280
TAACTGTCAG ACCAAGTTTA CTCATATATA CTTTAGATTG ATTTAAAACT TCATTTTTAA 5340
TTTAAAAGGA TCTAGGTGAA GATCCTTTTT GATAATCTCA TGACCAAAAT CCCTTAACGT 5400
GAGTTTTCGT TCCACTGAGC GTCAGACCCC GTAGAAAAGA TCAAAGGATC TTCTTGAGAT 5460
CCTTTTTTTC TGCGCGTAAT CTGCTGCTTG CAAACAAAAA AACCACCGCT ACCAGCGGTG 5520
GTTTGTTTGC CGGATCAAGA GCTACCAACT CTTTTTCCGA AGGTAACTGG CTTCAGCAGA 5580
GCGCAGATAC CAAATACTGT CCTTCTAGTG TAGCCGTAGT TAGGCCACCA CTTCAAGAAC 5640
TCTGTAGCAC CGCCTACATA CCTCGCTCTG CTAATCCTGT TACCAGTGGC TGCTGCCAGT 5700
GGCGATAAGT CGTGTCTTAC CGGGTTGGAC TCAAGACGAT AGTTACCGGA TAAGGCGCAG 5760
CGGTCGGGCT GAACGGGGGG TTCGTGCACA CAGCCCAGCT TGGAGCGAAC GACCTACACC 5820
GAACTGAGAT ACCTACAGCG TGAGCATTGA GAAAGCGCCA CGCTTCCCGA AGGGAGAAAG 5880
GCGGACAGGT ATCCGGTAAG CGGCAGGGTC GGAACAGGAG AGCGCACGAG GGAGCTTCCA 5940
GGGGGAAACG CCTGGTATCT TTATAGTCCT GTCGGGTTTC GCCACCTCTG ACTTGAGCGT 6000
CGATTTTTGT GATGCTCGTC AGGGGGGCGG AGCCTATGGA AAAACGCCAG CAACGCAGCT 6060
GC
```

FIG.8

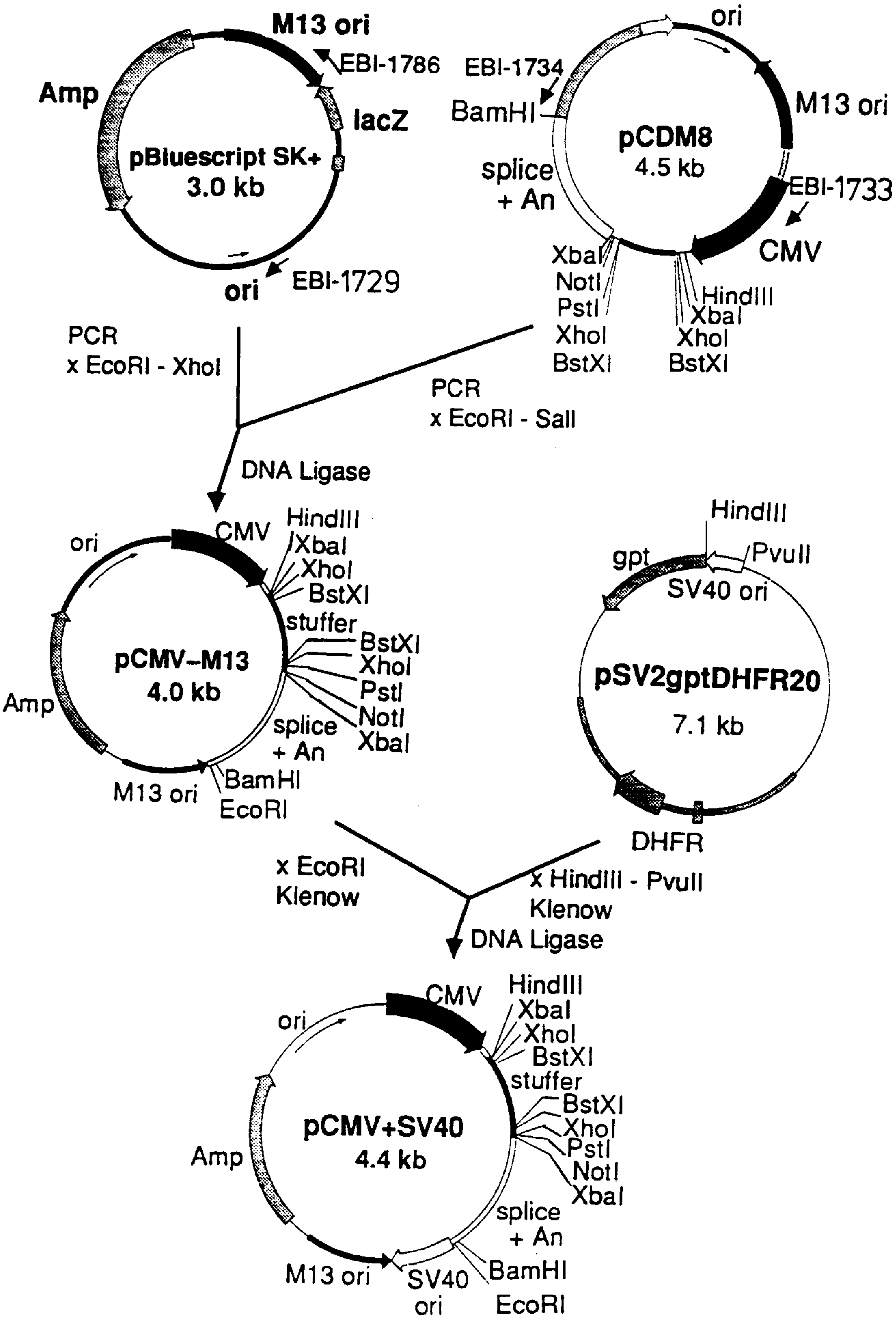
M13 ori
EBI-1786
Amp
lacZ
pBluescript SK+
3.0 kb
ori
EBI-1729
ori
EBI-1734
BamHI
M13 ori
pCDM8
4.5 kb
splice
+ An
EBI-1733
CMV
XbaI
NotI
PstI
XhoI
BstXI
HindIII
XbaI
XhoI
BstXI
PCR
x EcoRI - XhoI
PCR
x EcoRI - SalI
DNA Ligase
ori
CMV
HindIII
XbaI
XhoI
BstXI
stuffer
BstXI
XhoI
PstI
NotI
XbaI
pCMV-M13
4.0 kb
Amp
splice
+ An
M13 ori
BamHI
EcoRI
HindIII
gpt
PvuII
SV40 ori
pSV2gptDHFR20
7.1 kb
DHFR
x EcoRI
Klenow
x HindIII - PvuII
Klenow
DNA Ligase
ori
CMV
HindIII
XbaI
XhoI
BstXI
stuffer
BstXI
XhoI
PstI
NotI
XbaI
pCMV+SV40
4.4 kb
Amp
splice
+ An
M13 ori
SV40
ori
BamHI
EcoRI

FIG.9

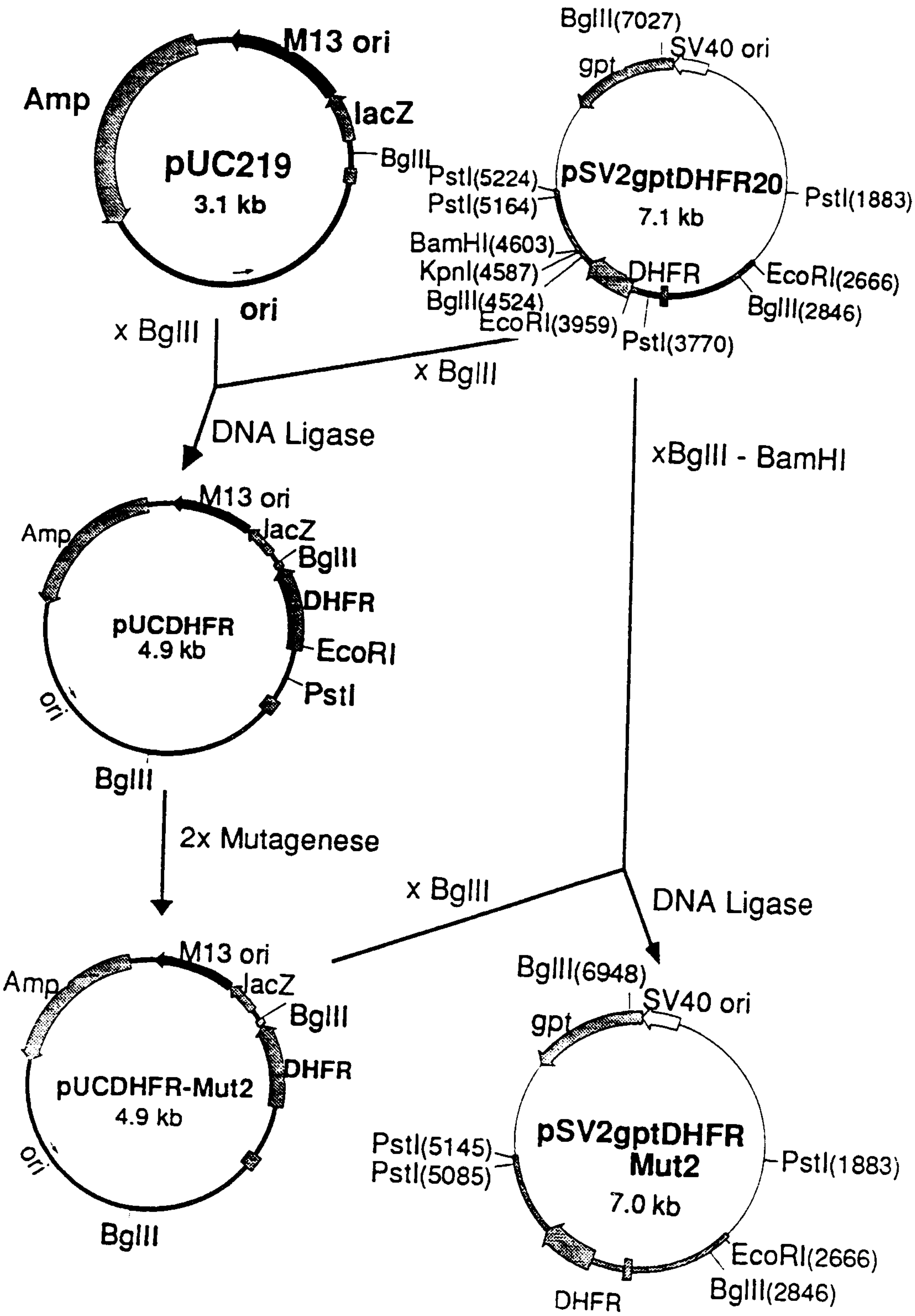
M13 ori
Amp
lacZ
pUC219
3.1 kb
BglII
ori
BglII(7027)
SV40 ori
gpt
PstI(5224)
PstI(5164)
pSV2gptDHFR20
7.1 kb
PstI(1883)
BamHI(4603)
KpnI(4587)
BglII(4524)
EcoRI(3959)
DHFR
PstI(3770)
EcoRI(2666)
BglII(2846)
x BglII
x BglII
DNA Ligase
xBglII - BamHI
M13 ori
Amp
lacZ
BglII
DHFR
pUCDHFR
4.9 kb
EcoRI
PstI
ori
BglII
2x Mutagenese
x BglII
DNA Ligase
M13 ori
Amp
lacZ
BglII
DHFR
pUCDHFR-Mut2
4.9 kb
ori
BglII
BglII(6948)
SV40 ori
gpt
pSV2gptDHFR
Mut2
7.0 kb
PstI(5145)
PstI(5085)
PstI(1883)
EcoRI(2666)
BglII(2846)
DHFR

FIG.10

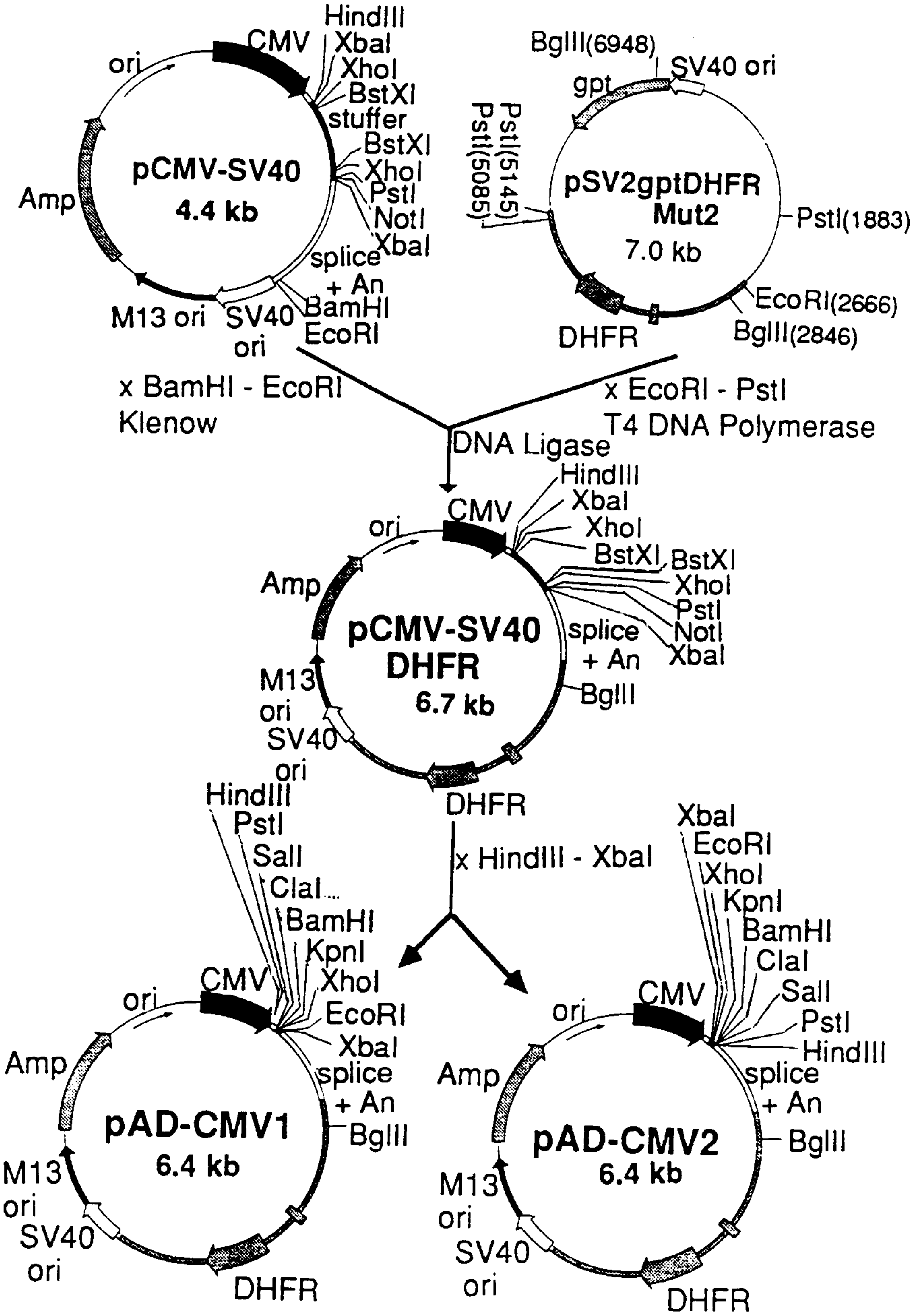
pCMV-SV40
4.4 kb
ori
CMV
HindIII
XbaI
XhoI
BstXI
stuffer
BstXI
XhoI
PstI
NotI
XbaI
splice
+ An
BamHI
EcoRI
Amp
M13 ori
SV40 ori
pSV2gptDHFR
Mut2
7.0 kb
BglII(6948)
SV40 ori
gpt
PstI(5145)
PstI(5085)
PstI(1883)
EcoRI(2666)
BglII(2846)
DHFR
x BamHI - EcoRI
Klenow
x EcoRI - PstI
T4 DNA Polymerase
DNA Ligase
pCMV-SV40
DHFR
6.7 kb
ori
CMV
HindIII
XbaI
XhoI
BstXI
BstXI
XhoI
PstI
NotI
XbaI
splice
+ An
BglII
Amp
M13 ori
SV40 ori
DHFR
x HindIII - XbaI
pAD-CMV1
6.4 kb
ori
CMV
HindIII
PstI
SalI
ClaI...
BamHI
KpnI
XhoI
EcoRI
XbaI
splice
+ An
BglII
Amp
M13 ori
SV40 ori
DHFR
pAD-CMV2
6.4 kb
ori
CMV
XbaI
EcoRI
XhoI
KpnI
BamHI
ClaI
SalI
PstI
HindIII
splice
+ An
BglII
Amp
M13 ori
SV40 ori
DHFR

# FIG.11A

**pAD-CMV1 : 6414 bp**

```
TCGACATTGA TTATTGACTA GTTATTAATA GTAATCAATT ACGGGGTCAT TAGTTCATAG     60
CCCATATATG GAGTTCCGCG TTACATAACT TACGGTAAAT GGCCCGCCTG GCTGACCGCC    120
CAACGACCCC CGCCCATTGA CGTCAATAAT GACGTATGTT CCCATAGTAA CGCCAATAGG    180
GACTTTCCAT TGACGTCAAT GGGTGGAGTA TTTACGGTAA ACTGCCCACT TGGCAGTACA    240
TCAAGTGTAT CATATGCCAA GTACGCCCCC TATTGACGTC AATGACGGTA AATGGCCCGC    300
CTGGCATTAT GCCCAGTACA TGACCTTATG GGACTTTCCT ACTTGGCAGT ACATCTACGT    360
ATTAGTCATC GCTATTACCA TGGTGATGCG GTTTTGGCAG TACATCAATG GGCGTGGATA    420
GCGGTTTGAC TCACGGGGAT TTCCAAGTCT CCACCCCATT GACGTCAATG GGAGTTTGTT    480
TTGGCACCAA AATCAACGGG ACTTTCCAAA ATGTCGTAAC AACTCCGCCC CATTGACGCA    540
AATGGGCGGT AGGCGTGTAC GGTGGGAGGT CTATATAAGC AGAGCTCTCT GGCTAACTAG    600
AGAACCCACT GCTTAACTGG CTTATCGAAA TTAATACGAC TCACTATAGG GAGACCCAAG    660
CTTCTGCAGG TCGACATCGA TGGATCCGGT ACCTCGAGCG CGAATTCTCT AGAGGATCTT    720
TGTGAAGGAA CCTTACTTCT GTGGTGTGAC ATAATTGGAC AAACTACCTA CAGAGATTTA    780
AAGCTCTAAG GTAAATATAA AATTTTTAAG TGTATAATGT GTTAAACTAC TGATTCTAAT    840
TGTTTGTGTA TTTTAGATTC CAACCTATGG AACTGATGAA TGGGAGCAGT GGTGGAATGC    900
CTTTAATGAG GAAAACCTGT TTTGCTCAGA AGAAATGCCA TCTAGTGATG ATGAGGCTAC    960
TGCTGACTCT CAACATTCTA CTCCTCCAAA AAAGAAGAGA AAGGTAGAAG ACCCCAAGGA   1020
CTTTCCTTCA GAATTGCTAA GTTTTTTGAG TCATGCTGTG TTTAGTAATA GAACTCTTGC   1080
TTGCTTTGCT ATTTACACCA CAAAGGAAAA AGCTGCACTG CTATACAAGA AAATTATGGA   1140
AAAATATTTG ATGTATAGTG CCTTGACTAG AGATCATAAT CAGCCATACC ACATTTGTAG   1200
AGGTTTTACT TGCTTTAAAA AACCTCCCAC ACCTCCCCCT GAACCTGAAA CATAAAATGA   1260
ATGCAATTGT TGTTGTTAAC TTGTTTATTG CAGCTTATAA TGGTTACAAA TAAAGCAATA   1320
GCATCACAAA TTTCACAAAT AAAGCATTTT TTTCACTGCA TTCTAGTTGT GGTTTGTCCA   1380
AACTCATCAA TGTATCTTAT CATGTCTGGA TCAATTCTGA GAAACTAGCC TTAAAGACAG   1440
ACAGCTTTGT TCTAGTCAGC CAGGCAAGCA TATGTAAATA AAGTTCCTCA GGGAACTGAG   1500
```

FIG.11B

```
GTTAAAAGAT GTATCCTGGA CCTGCCAGAC CTGGCCATTC ACGTAAACAG AAGATTCCGC   1560
CTCAAGTTCC GGTTAACAAC AGGAGGCAAC GAGATCTCAA ATCTATTACT TCTAATCGGG   1620
TAATTAAAAC CTTTCAACTA AAACACGGAC CCACGGATGT CACCCACTTT TCCTTCCCCG   1680
GCTCCGCCCT TCTCAGTACT CCCCACCATT AGGCTCGCTA CTCCACCTCC ACTTCCGGGC   1740
GCGACACCCA CGTGCCCTCT CCCACCCGAC GCTAACCCCG CCCCTGCCCG TCTGACCCCG   1800
CCCACCACCT GGCCCCGCCC CGTTGAGGAC AGAAGAAACC CCGGGCAGCC GCAGCCAAGG   1860
CGGACGGGTA GACGCTGGGG GCGCTGAGGA GTCGTCCTCT ACCTTCTCTG CTGGCTCGGT   1920
GGGGGACGCG GTGGATCTCA GGCTTCCGGA AGACTGGAAG AACCGGCTCA GAACCGCTTG   1980
TCTCCGCGGG GCTTGGGCGG CGGAAGAATG GCCGCTAGAC GCGGACTTGG TGCGAGGCAT   2040
CGCAGGATGC AGAAGAGCAA GCCCGCCGGG AGCGCGCGGC TGTACTACCC CGCGCCTGGA   2100
GCGGCCACGC CGGACTGGGC GGGGCCGGCC TGGTGGAGGC GGAGTCTGAC CTCGTGGAGG   2160
CGGGGCCTCT GATGTTCAAA TAGGATGCTA GGCTTGTTGA GGCGTGGCCT CCGATTCACA   2220
AGTGGGAAGC AGCGCCGGGC GACTGCAATT TCGCGCCAAA CTTGGGGGAA GCACAGCGTA   2280
CAGGCTGCCT AGGTGATCGC TGCTGCTGTC ATGGTTCGAC CGCTGAACTG CATCGTCGCC   2340
GTGTCCCAGA ATATGGGCAT CGGCAAGAAC GGAGACCTTC CCTGGCCAAT GCTCAGGTAC   2400
TGGCTGGATT GGGTTAGGGA AACCGAGGCG GTTCGCTGAA TCGGGTCGAG CACTTGGCGG   2460
AGACGCGCGG GCCAACTACT TAGGGACAGT CATGAGGGGT AGGCCCGCCG GCTGCTGCCC   2520
TTGCCCATGC CCGCGGTGAT CCCCATGCTG TGCCAGCCTT TGCCCAGAGG CGCTCTAGCT   2580
GGGAGCAAAG TCCGGTCACT GGGCAGCACC ACCCCCCGGA CTTGCATGGG TAGCCGCTGA   2640
GATGGAGCCT GAGCACACGT GACAGGGTCC CTGTTAACGC AGTGTTTCTC TAACTTTCAG   2700
GAACGAGTTC AAGTACTTCC AAAGAATGAC CACCACCTCC TCAGTGGAAG GTAAACAGAA   2760
CCTGGTGATT ATGGGCCGGA AAACCTGGTT CTCCATTCCT GAGAAGAATC GACCTTTAAA   2820
GGACAGAATT AATATAGTTC TCAGTAGAGA GCTCAAGGAA CCACCACAAG GAGCTCATTT   2880
TCTTGCCAAA AGTCTGGACC ATGCCTTAAA ACTTATTGAA CAACCAGAGT TAGCAGATAA   2940
AGTGGACATG GTTTGGATAG TTGGAGGCAG TTCCGTTTAC AAGGAAGCCA TGAATCAGCC   3000
AGGCCATCTC AGACTCTTTG TGACAAGGAT CATGCAGGAA TTTGAAAGTG ACACGTTCTT   3060
```

FIG. 11C

```
CCCAGAAATT GATTTGGAGA AATATAAACT TCTCCCAGAG TACCCAGGGG TCCTTTCTGA   3120
AGTCCAGGAG GAAAAAGGCA TCAAGTATAA ATTTGAAGTC TATGAGAAGA AAGGCTAACA   3180
GAAAGATACT TGCTGATTGA CTTCAAGTTC TACTGCTTTC CTCCTAAAAT TATGCATTTT   3240
TACAAGACCA TGGGACTTGT GTTGGCTTTA GATCCTGTGC ATCCTGGGCA ACTGTTGTAC   3300
TCTAAGCCAC TCCCCAAAGT CATGCCCCAG CCCCTGTATA ATTCTAAACA ATTAGAATTA   3360
TTTTCATTTT CATTAGTCTA ACCAGGTTAT ATTAAATATA CTTTAAGAAA CACCATTTGC   3420
CATAAAGTTC TCAATGCCCC TCCCATGCAG CCTCAAGTGG CTCCCCAGCA GATGCATAGG   3480
GTAGTGTGTG TACAAGAGAC CCCAAAGACA TAGAGCCCCT GAGAGCATGA GCTGATATGG   3540
GGGCTCATAG AGATAGGAGC TAGATGAATA AGTACAAAGG GCAGAAATGG GTTTTAACCA   3600
GCAGAGCTAG AACTCAGACT TTAAAGAAAA TTAGATCAAA GTAGAGACTG AATTATTCTG   3660
CACATCAGAC TCTGAGCAGA GTTCTGTTCA CTCAGACAGA AAATGGGTAA ATTGAGAGCT   3720
GGCTCCATTG TGCTCCTTAG AGATGGGAGC AGGTGGAGGA TTATATAAGG TCTGGAACAT   3780
TTAACTTCTC CGTTTCTCAT CTTCAGTGAG ATTCCAAGGG ATACTACAAT TCTGTGGAAT   3840
GTGTGTCAGT TAGGGTGTGG AAAGTCCCCA GGCTCCCCAG CAGGCAGAAG TATGCAAAGC   3900
ATGCATCTCA ATTAGTCAGC AACCAGGTGT GGAAAGTCCC CAGGCTCCCC AGCAGGCAGA   3960
AGTATGCAAA GCATGCATCT CAATTAGTCA GCAACCATAG TCCCGCCCCT AACTCCGCCC   4020
ATCCCGCCCC TAACTCCGCC CAGTTCCGCC CATTCTCCGC CCCATGGCTG ACTAATTTTT   4080
TTTATTTATG CAGAGGCCGA GGCGCCTCTG AGCTATTCCA GAAGTAGTGA GGAGGCTTTT   4140
TTGGAGGCCT AGGCTTTTGC AAAAAGCTA ATTCAGCCTG AATGGCGAAT GGGACGCGCC   4200
CTGTAGCGGC GCATTAAGCG CGGCGGGTGT GGTGGTTACG CGCAGCGTGA CCGCTACACT   4260
TGCCAGCGCC CTAGCGCCCG CTCCTTTCGC TTTCTTCCCT TCCTTTCTCG CCACGTTCGC   4320
CGGCTTTCCC CGTCAAGCTC TAAATCGGGG GCTCCCTTTA GGGTTCCGAT TTAGTGCTTT   4380
ACGGCACCTC GACCCCAAAA ACTTGATTAG GGTGATGGTT CACGTAGTGG GCCATCGCCC   4440
TGATAGACGG TTTTTCGCCC TTTGACGTTG GAGTCCACGT TCTTTAATAG TGGACTCTTG   4500
TTCCAAACTG GAACAACACT CAACCCTATC TCGGTCTATT CTTTTGATTT ATAAGGGATT   4560
TTGCCGATTT CGGCCTATTG GTTAAAAAAT GAGCTGATTT AACAAAAATT TAACGCGAAT   4620
```

FIG.11D

```
TTTAACAAAA TATTAACGTT TACAATTTCA GGTGGCACTT TTCGGGGAAA TGTGCGCGGA   4680
ACCCCTATTT GTTTATTTTT CTAAATACAT TCAAATATGT ATCCGCTCAT GAGACAATAA   4740
CCCTGATAAA TGCTTCAATA ATATTGAAAA AGGAAGAGTA TGAGTATTCA ACATTTCCGT   4800
GTCGCCCTTA TTCCCTTTTT TGCGGCATTT TGCCTTCCTG TTTTTGCTCA CCCAGAAACG   4860
CTGGTGAAAG TAAAAGATGC TGAAGATCAG TTGGGTGCAC GAGTGGGTTA CATCGAACTG   4920
GATCTCAACA GCGGTAAGAT CCTTGAGAGT TTTCGCCCCG AAGAACGTTT TCCAATGATG   4980
AGCACTTTTA AAGTTCTGCT ATGTGGCGCG GTATTATCCC GTATTGACGC CGGGCAAGAG   5040
CAACTCGGTC GCCGCATACA CTATTCTCAG AATGACTTGG TTGAGTACTC ACCAGTCACA   5100
GAAAAGCATC TTACGGATGG CATGACAGTA AGAGAATTAT GCAGTGCTGC CATAACCATG   5160
AGTGATAACA CTGCGGCCAA CTTACTTCTG ACAACGATCG GAGGACCGAA GGAGCTAACC   5220
GCTTTTTTGC ACAACATGGG GGATCATGTA ACTCGCCTTG ATCGTTGGGA ACCGGAGCTG   5280
AATGAAGCCA TACCAAACGA CGAGCGTGAC ACCACGATGC CTGTAGCAAT GGCAACAACG   5340
TTGCGCAAAC TATTAACTGG CGAACTACTT ACTCTAGCTT CCCGGCAACA ATTAATAGAC   5400
TGGATGGAGG CGGATAAAGT TGCAGGACCA CTTCTGCGCT CGGCCCTTCC GGCTGGCTGG   5460
TTTATTGCTG ATAAATCTGG AGCCGGTGAG CGTGGGTCTC GCGGTATCAT TGCAGCACTG   5520
GGGCCAGATG GTAAGCCCTC CCGTATCGTA GTTATCTACA CGACGGGGAG TCAGGCAACT   5580
ATGGATGAAC GAAATAGACA GATCGCTGAG ATAGGTGCCT CACTGATTAA GCATTGGTAA   5640
CTGTCAGACC AAGTTTACTC ATATATACTT TAGATTGATT TAAAACTTCA TTTTTAATTT   5700
AAAAGGATCT AGGTGAAGAT CCTTTTTGAT AATCTCATGA CCAAAATCCC TTAACGTGAG   5760
TTTTCGTTCC ACTGAGCGTC AGACCCCGTA GAAAAGATCA AAGGATCTTC TTGAGATCCT   5820
TTTTTTCTGC GCGTAATCTG CTGCTTGCAA ACAAAAAAAC CACCGCTACC AGCGGTGGTT   5880
TGTTTGCCGG ATCAAGAGCT ACCAACTCTT TTTCCGAAGG TAACTGGCTT CAGCAGAGCG   5940
CAGATACCAA ATACTGTCCT TCTAGTGTAG CCGTAGTTAG GCCACCACTT CAAGAACTCT   6000
GTAGCACCGC CTACATACCT CGCTCTGCTA ATCCTGTTAC CAGTGGCTGC TGCCAGTGGC   6060
GATAAGTCGT GTCTTACCGG GTTGGACTCA AGACGATAGT TACCGGATAA GGCGCAGCGG   6120
TCGGGCTGAA CGGGGGGTTC GTGCACACAG CCCAGCTTGG AGCGAACGAC CTACACCGAA   6180
```

FIG. 11E

```
CTGAGATACC TACAGCGTGA GCATTGAGAA AGCGCCACGC TTCCCGAAGG GAGAAAGGCG   6240
GACAGGTATC CGGTAAGCGG CAGGGTCGGA ACAGGAGAGC GCACGAGGGA GCTTCCAGGG   6300
GGAAACGCCT GGTATCTTTA TAGTCCTGTC GGGTTTCGCC ACCTCTGACT TGAGCGTCGA   6360
TTTTTGTGAT GCTCGTCAGG GGGGCGGAGC CTATGGAAAA ACGCCAGCAA CGCC
```

FIG.12

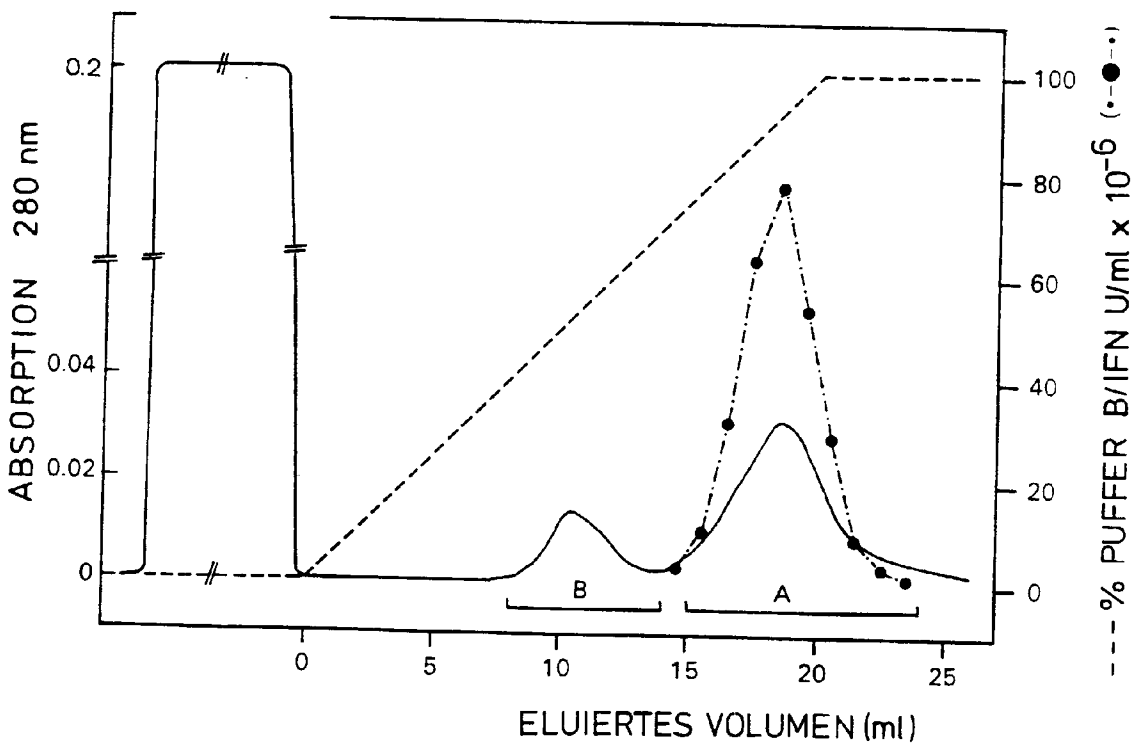
ABSORPTION 280 nm
0.2
0.04
0.02
0
0
5
10
15
20
25
B
A
ELUIERTES VOLUMEN (ml)
100
80
60
40
20
0
--- % PUFFER B/IFN U/ml x $10^{-6}$ (•-•-•) ●

FIG.13

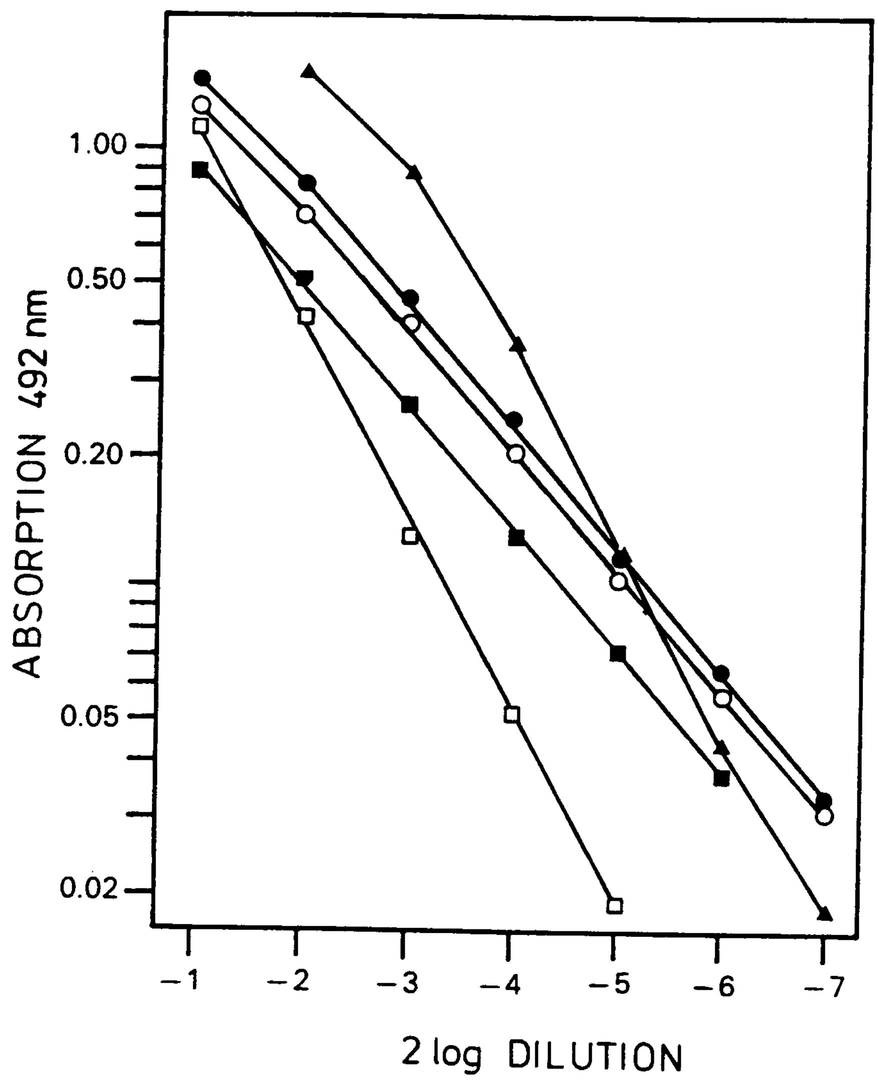

FIG. 14

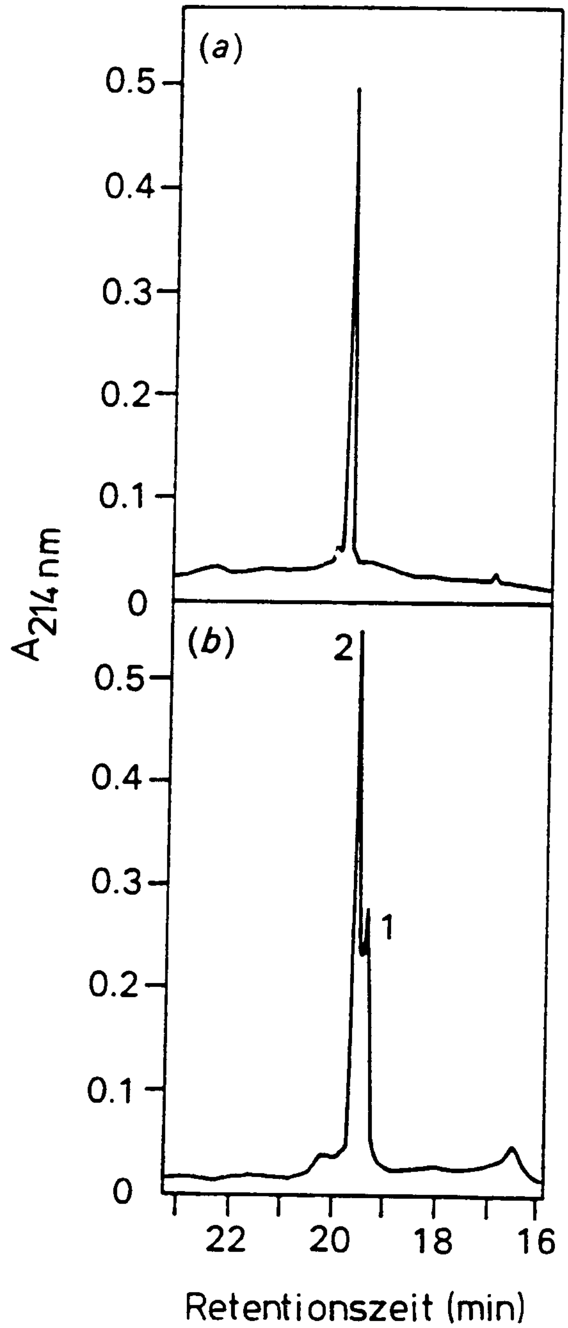

FIG.15

1 5 10 15
CYS-ASP-LEU-PRO-GLN-THR-HIS-SER-LEU-GLY-SER-ARG-ARG-THR-LEU-

20 25 30
MET-LEU-LEU-ALA-GLN-MET-ARG-ARG-ILE-SER-LEU-PHE-SER-CYS-LEU-

35 40 45
LYS-ASP-ARG-ARG-ASP-PHE-GLY-PHE-PRO-GLN-GLU-GLU-PHE-GLY-ASN-

50 55 60
GLN-PHE-GLN-LYS-ALA-GLU-THR-ILE-PRO-VAL-LEU-HIS-GLU-MET-ILE-

65 70 75
GLN-GLN-ILE-PHE-ASN-LEU-PHE-SER-THR-LYS-ASP-SER-SER-ALA-ALA-

80 85 90
TRP-ASP-GLU-THR-LEU-LEU-ASP-LYS-PHE-TYR-THR-GLU-LEU-TYR-GLN-

95 100 105
GLN-LEU-ASN-ASP-LEU-GLU-ALA-CYS-VAL-ILE-GLN-GLY-VAL-GLY-VAL-

110 115 120
THR-GLU-THR-PRO-LEU-MET-LYS-GLU-ASP-SER-ILE-LEU-ALA-VAL-ARG

125 130 135
LYS-TYR-PHE-GLN-ARG-ILE-THR-LEU-TYR-LEU-LYS-GLU-LYS-LYS-TYR-

140 145 150
SER-PRO-CYS-ALA-TRP-GLU-VAL-VAL-ARG-ALA-GLU-ILE-MET-ARG-SER-

155 160 165
PHE-SER-LEU-SER-THR-ASN-LEU-GLN-GLU-SER-LEU-ARG-SER-LYS-GLU

α2c

FIG. 16

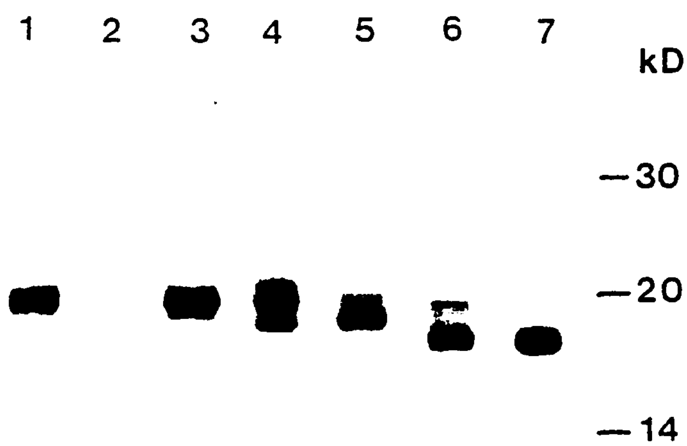

FIG. 17

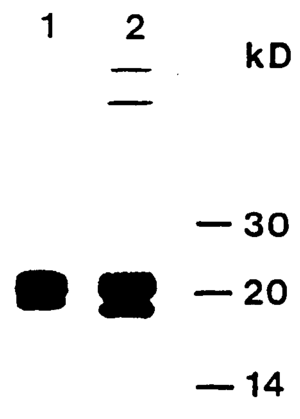

FIG.18

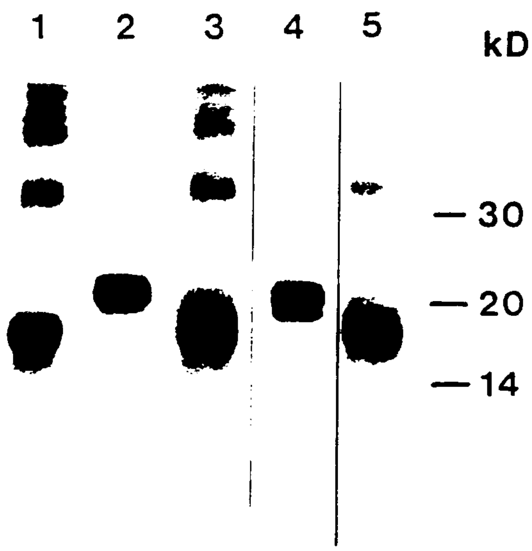

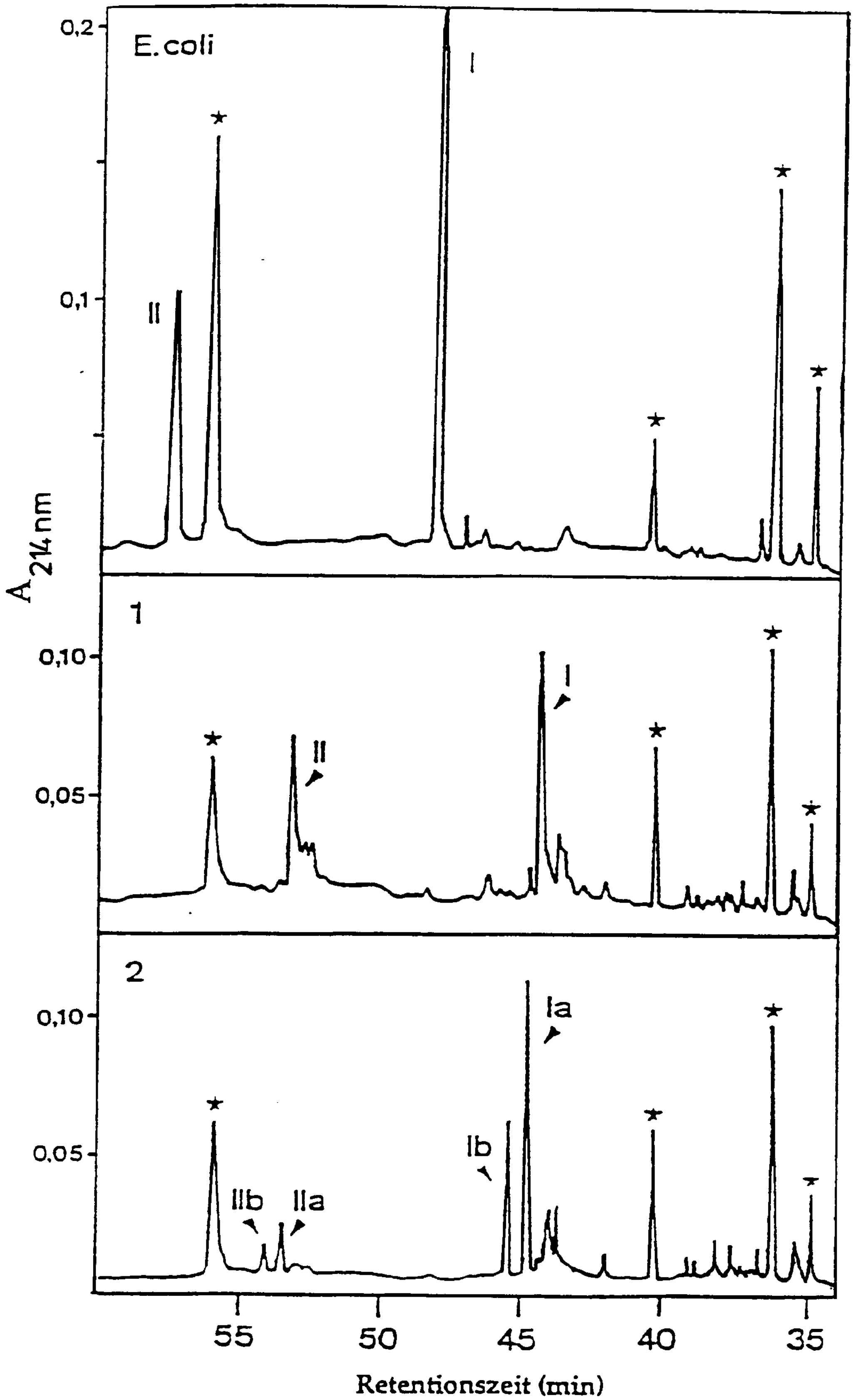
0,2
0,1
E.coli
I
II
★
A214nm
1
0,10
0,05
2
Ia
Ib
IIb
IIa
55
50
45
40
35
Retentionszeit (min)

FIG.19

FIG. 20

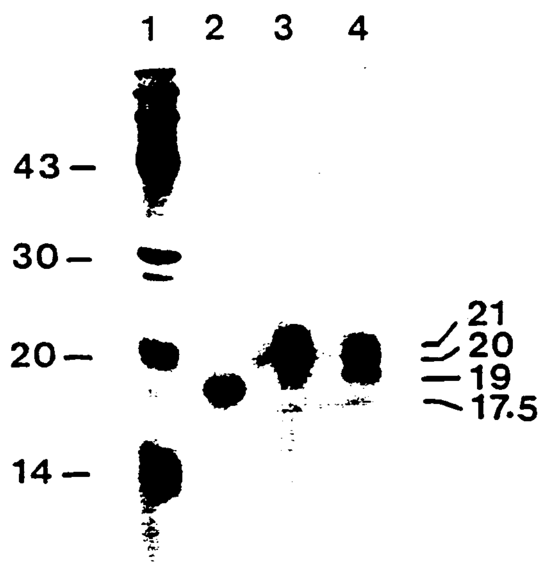

FIG. 21

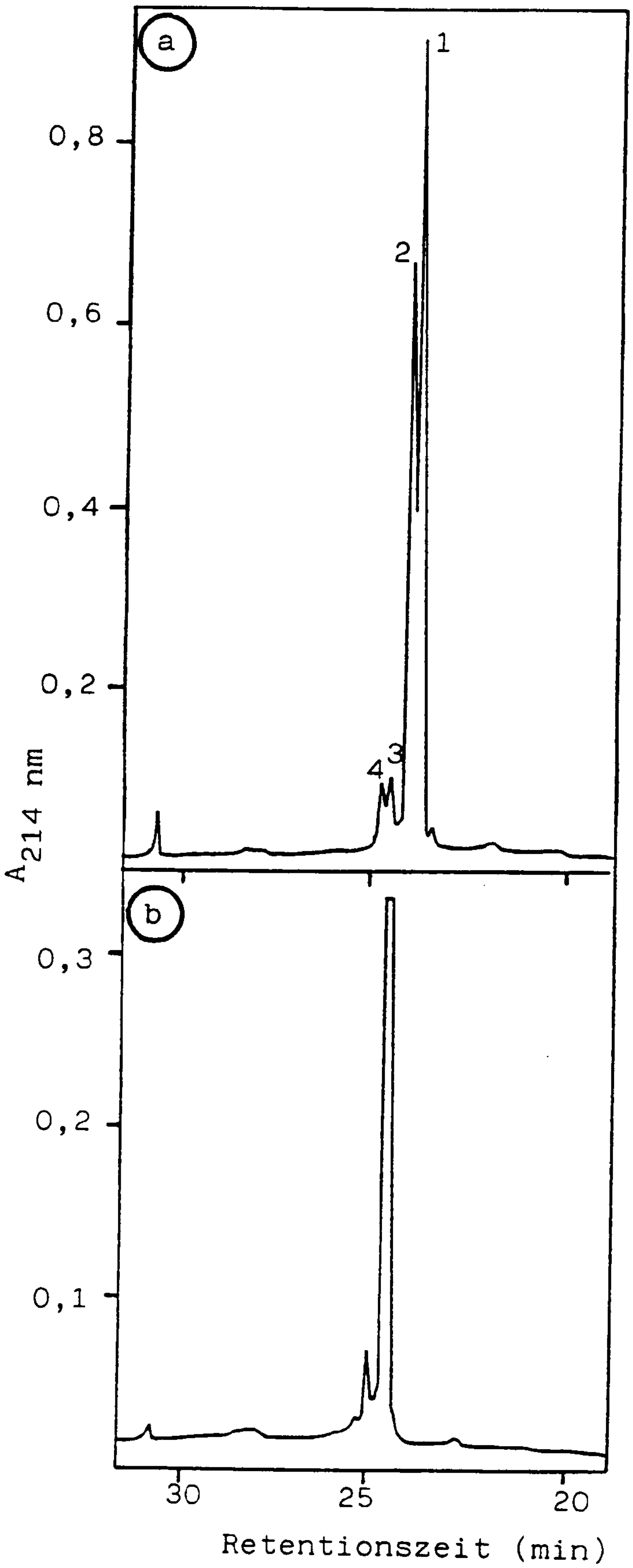

FIG. 22

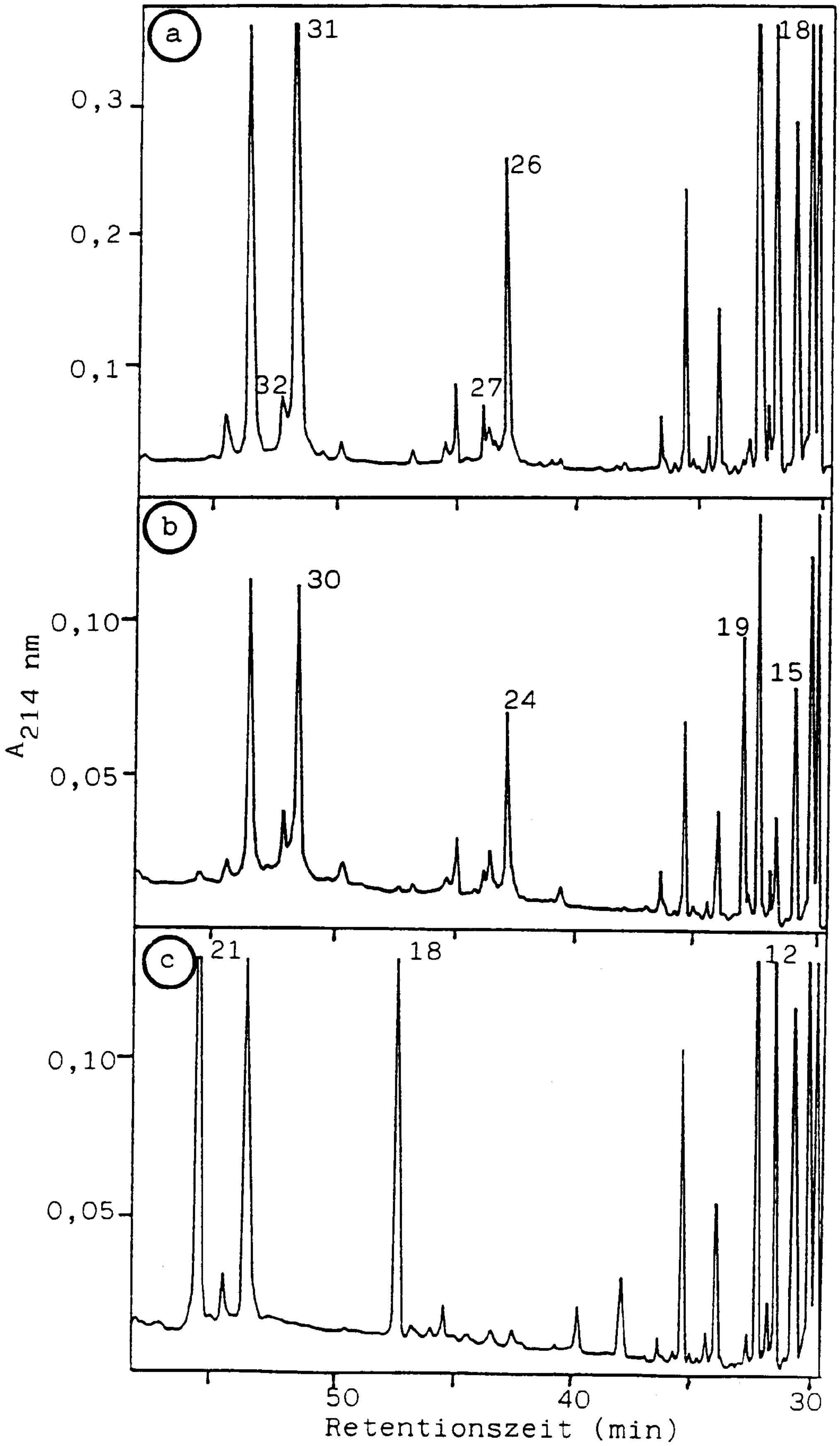
a
b
c
31
32
26
27
18
30
24
19
15
21
18
12
0,3
0,2
0,1
0,10
0,05
0,10
0,05
$A_{214}$ nm
50
40
30
Retentionszeit (min)

FIG. 23

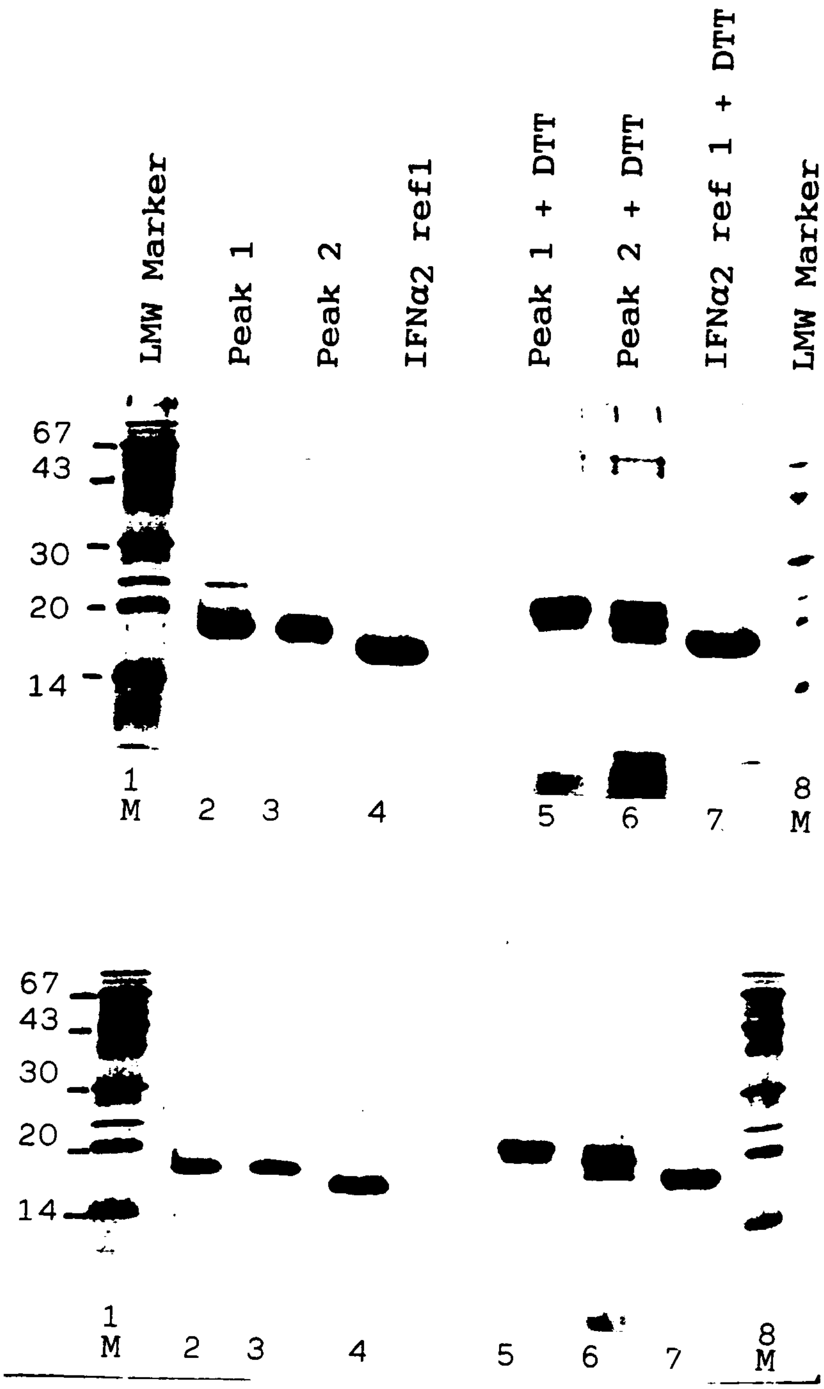
LMW Marker
Peak 1
Peak 2
IFNα2 ref1
Peak 1 + DTT
Peak 2 + DTT
IFNα2 ref 1 + DTT
LMW Marker
67
43
30
20
14
1 M
2
3
4
5
6
7
8 M
67
43
30
20
14
1 M
2
3
4
5
6
7
8 M

FIG.24

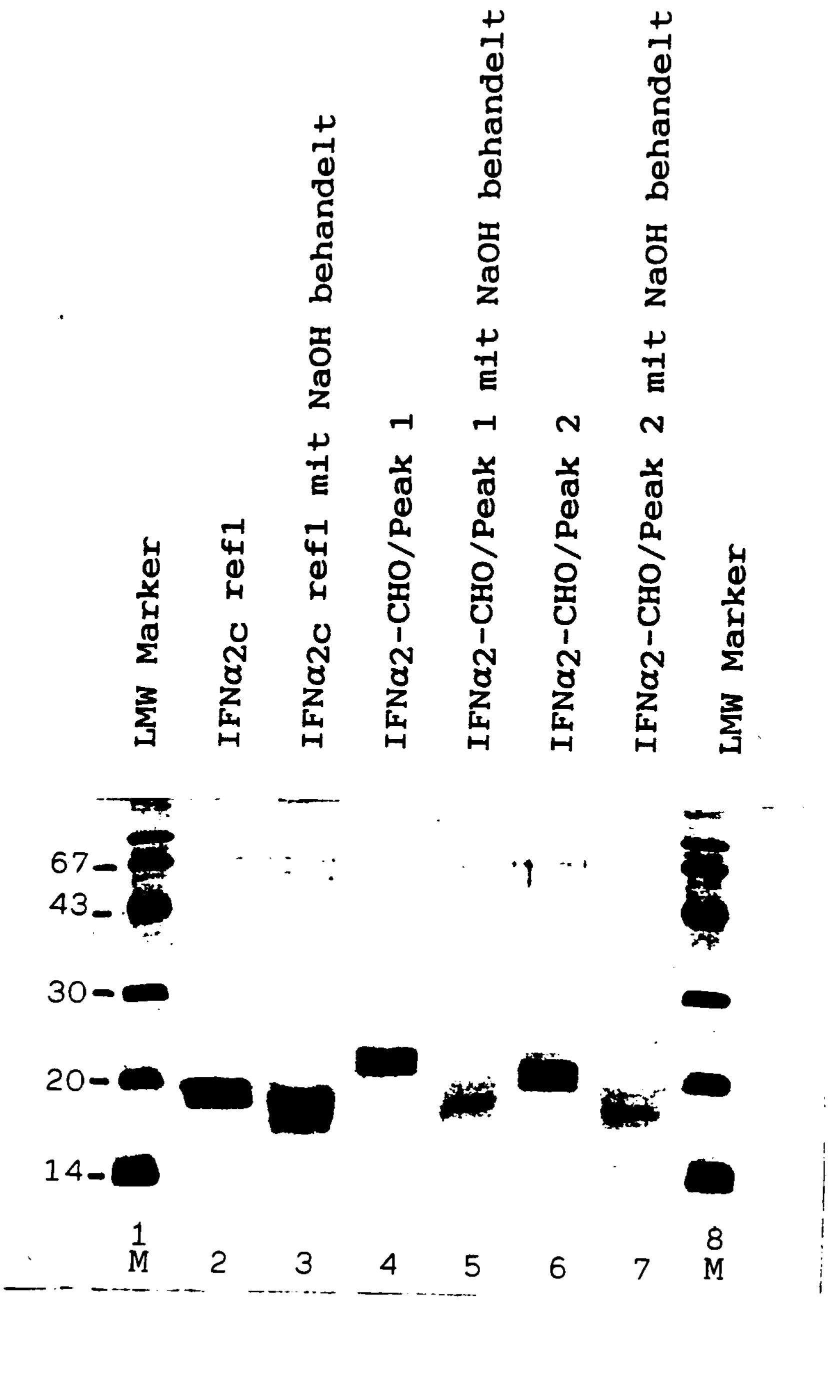
LMW Marker
IFNα2c refl
IFNα2c refl mit NaOH behandelt
IFNα2-CHO/Peak 1
IFNα2-CHO/Peak 1 mit NaOH behandelt
IFNα2-CHO/Peak 2
IFNα2-CHO/Peak 2 mit NaOH behandelt
LMW Marker
67
43
30
20
14
1 M
2
3
4
5
6
7
8 M